(19) Europäisches Patentamt
European Patent Office
Office européen des brevets

(11) Veröffentlichungsnummer: **0 489 360 B1**

# (12) EUROPÄISCHE PATENTSCHRIFT

(45) Veröffentlichungstag der Patentschrift: **20.09.95**

(21) Anmeldenummer: **91120427.9**

(22) Anmeldetag: **28.11.91**

(51) Int. Cl.[6]: **C09B 62/507**, C07C 317/14, C07C 317/18, C07C 317/36, C07C 317/44, C07C 323/09, C07C 323/12, C07C 323/52

## (54) Wasserlösliche Azoverbindungen, Verfahren zu ihrer Herstellung und ihre Verwendung als Farbstoffe.

(30) Priorität: **01.12.90 DE 4038343**
**18.09.91 DE 4131050**

(43) Veröffentlichungstag der Anmeldung:
**10.06.92 Patentblatt 92/24**

(45) Bekanntmachung des Hinweises auf die Patenterteilung:
**20.09.95 Patentblatt 95/38**

(84) Benannte Vertragsstaaten:
**CH DE ES FR GB IT LI**

(56) Entgegenhaltungen:
**EP-A- 0 392 351**
**BE-A- 654 544**

(73) Patentinhaber: **HOECHST AKTIENGESELLSCHAFT**

**D-65926 Frankfurt am Main (DE)**

(72) Erfinder: **Dannheim, Jörg, Dr.**
**Strubbergstrasse 32**
**W-6000 Frankfurt/aM (DE)**

**EP 0 489 360 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung liegt auf dem Gebiet der faserreaktiven Farbstoffe.

In der europäischen Patentanmeldungs-Veröffentlichung Nr. 0 392 351 werden Isobutenyl-sulfonyl-anilin-Verbindungen beschrieben, deren im Isobutenyl-Rest endständige Methylgruppe durch eine Hydroxygruppe substituiert ist. In der allgemeinen Beschreibung wird erwähnt, daß anstelle dieses Hydroxysubstituenten auch (beispielsweise) ein Chloratom, eine niedere Alkanoyloxy- oder Sulfatogruppe stehen kann. Diese Anilinverbindungen sollen unter anderem als Diazokomponenten zur Synthese von Farbstoffen geeignet sein; Farbstoffe selbst mit diesen Vorstufen werden jedoch nicht beschrieben.

Es wurden neue wasserlösliche Azoverbindungen entsprechend der allgemeinen Formel (1)

D - N = N - K (1)

gefunden, die wertvolle faserreaktive Farbstoffeigenschaften besitzen.

In dieser Formel (1) bedeuten:

D ist ein Rest der allgemeinen Formel (2)

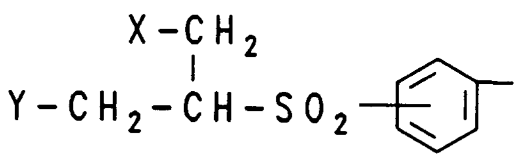

(2)

in welcher

Y ein Substituent ist, der durch Alkali unter Bildung der Vinylgruppe eliminierbar ist, wobei die Sulfonylgruppe bevorzugt in meta- oder para-Stellung zur freien Bindung an den Benzolkern gebunden ist, und

X Chlor oder Brom, bevorzugt Chlor, ist;

K ist ein Rest einer einfachen ankuppelbaren, bevorzugt wasserlöslichen, Kupplungskomponente, die noch eine Azogruppe enthalten kann, oder der Rest einer doppelankuppelbaren, bevorzugt wasserlöslichen, Kupplungskomponente, jeweils aus der Reihe der Aminobenzole, der Phenole, insbesondere deren Sulfonsäuren und Carbonsäuren, der Naphthole, insbesondere deren Sulfonsäuren und Carbonsäuren, der Aminonaphthole, insbesondere deren Sulfonsäuren, der Acylaminonaphthole, insbesondere deren Sulfonsäuren, mit dem Acylrest einer Alkan- oder Alkencarbonsäure mit jeweils 1 bis 4 bzw. 2 bis 4 C-Atomen im Alkyl- bzw. Alkenylrest oder einer aromatischen Carbonsäure, wie der Benzoesäure, oder einer aromatischen Sulfonsäure, wie der Benzol- oder Toluolsulfonsäure, oder einer N-substituierten Carbaminsäure, wie der N-Phenylureido-Rest, oder aus der Reihe der Dihydroxynaphthalinsulfonsäuren, der Phenylazo- und Naphthylazo-aminonaphtholsulfonsäuren, der 5-Pyrazolone und 5-Aminopyrazole, der Acetoacetylarylide, der 2-Hydroxy-6-pyridone und der Hydroxychinoline, wobei K neben den in Farbstoffen üblichen Substituenten auch eine fasserreaktive Gruppe der allgemeinen Formel $-SO_2-Y^1$, in welcher $Y^1$ die Vinylgruppe oder eine Gruppe der Formel $-CH_2-CH_2-Y$ mit Y einer der obigen Bedeutungen ist, enthalten kann.

Alkalisch eliminierbare Substituenten Y sind beispielsweise Halogenatome, wie das Bromatom und Chloratom, die Cyanogruppe, Trialkylammoniumgruppen mit Alkylgruppen von jeweils 1 bis 4 C-Atomen, wie die Trimethylammonium- und Triethylammoniumgruppe, und Estergruppen organischer Carbon- und Sulfonsäuren, wie ein Alkanoyloxyrest von 2 bis 5 C-Atomen, beispielsweise die Acetyloxygruppe, oder ein Sulfobenzoyloxy-, Benzoyloxy-, Phenylsulfonyloxy oder Toluylsulfonyloxy-Rest und die Phosphato-, Sulfato- und Thiosulfatogruppen. Bevorzugt ist Y Chlor.

Sulfogruppen sind Gruppen entsprechend der allgemeinen Formel $-SO_3M$ , Carboxygruppen sind Gruppen entsprechend der allgemeinen Formel -COOM , Sulfatogruppen sind Gruppen entsprechend der allgemeinen Formel $-OSO_3M$ , Thiosulfatogruppen sind Gruppen entsprechend der allgemeinen Formel $-S-SO_3M$ und Phosphatogruppen sind Gruppen entsprechend der allgemeinen Formel $-OPO_3M_2$ , in welchen

M ein Wasserstoffatom oder ein salzbildendes Metallatom, wie insbesondere ein Alkalimetallatom, wie beispielsweise Natrium, Kalium oder Lithium, ist.

Von den erfindungsgemäßen Verbindungen der allgemeinen Formel (1) können beispielsweise solche Verbindungen hervorgehoben werden, in welchen K einen Rest der nachstehenden Formel (3a), (3b), (3c), (3d), (3e), (3f), (3g), (3h), (3i), (3k), (3m), (3n), (3p), (3q), (3r), (3s), (3t), (3v) und (3w) bedeutet:

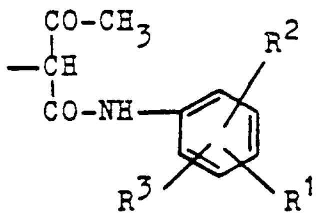 (3a)

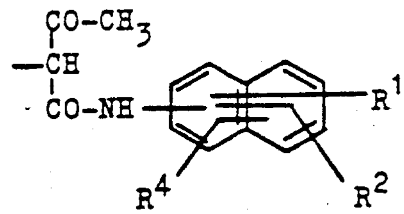 (3b)

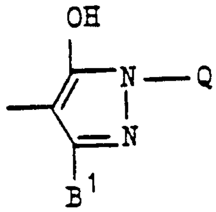 (3c)

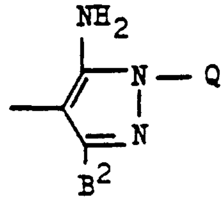 (3d)

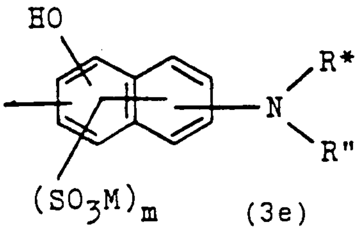
(3e)

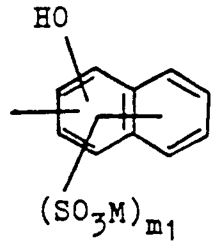
(3f)

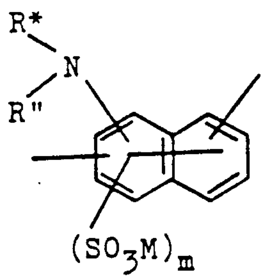
(3g)

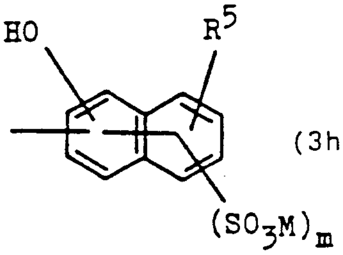
(3h)

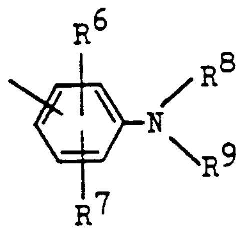
(3i)

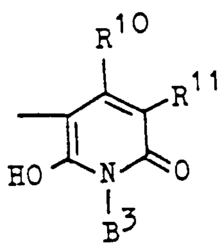
(3k)

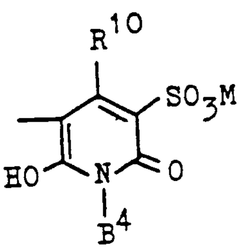
(3m)

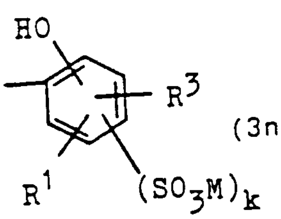
(3n)

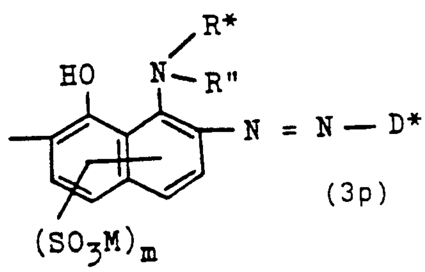
(3p)

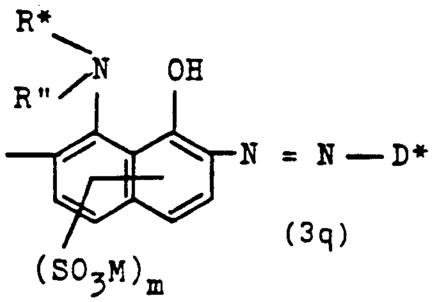

(3q)

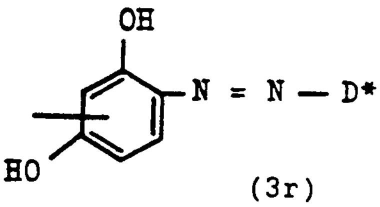

(3r)

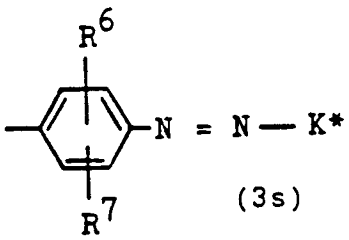

(3s)

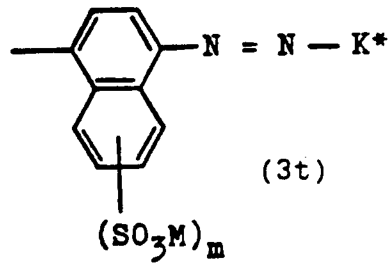

(3t)

In diesen Formeln bedeuten:

- $R^1$ ist Wasserstoff, Carboxy, Sulfo oder eine Gruppe der allgemeinen Formel $-G^*-SO_2-Y^1$ , in welcher $Y^1$ eine der obengenannten Bedeutungen hat und G* eine direkte Bindung, die Methylen- oder Ethylengruppe oder eine Propylenoxygruppe der Formel $-O-(CH_2)_3-$ oder $-O-CH_2-CH(CH_3)-$ ist;
- $R^2$ ist Wasserstoff, Alkyl von 1 bis 4 C-Atomen, Alkoxy von 1 bis 4 C-Atomen, Chlor, Brom, Carboxy oder Sulfo, bevorzugt Wasserstoff, Alkyl von 1 bis 4 C-Atomen, Alkoxy von 1 bis 4 C-Atomen, Carboxy oder Sulfo;
- $R^3$ ist Wasserstoff, Alkyl von 1 bis 4 C-Atomen, Alkoxy von 1 bis 4 C-Atomen, Chlor oder Brom, bevorzugt Wasserstoff oder Alkoxy von 1 bis 4 C-Atomen;
- $R^4$ ist Wasserstoff, Sulfo oder Carboxy, bevorzugt Wasserstoff;
- $B^1$ ist Alkyl von 1 bis 4 C-Atomen, wie insbesondere Methyl, Carboxy, Carbalkoxy 2 bis 5 C-Atomen, wie insbesondere Carbomethoxy, Carbamoyl, Phenyl oder durch 1 oder 2 Substituenten aus der Gruppe Sulfo, Carboxy, Methyl, Ethyl, Methoxy, Ethoxy und Chlor substituiertes Phenyl, und ist bevorzugt Methyl, Carboxy oder Carbalkoxy von 2 bis 5 C-Atomen;
- $B^2$ ist Alkyl von 1 bis 4 C-Atomen, wie insbesondere Methyl, Carboxy, Carbalkoxy von 2 bis 5 C-Atomen, Carbamoyl, Phenyl oder durch 1 oder 2 Substituenten aus der Gruppe Alkyl von 1 bis 4 C-Atomen, Alkoxy von 1 bis 4 C-Atomen, Chlor, Brom und Sulfo substituiertes Phenyl, und ist bevorzugt Methyl oder Carboxy;
- Q ist Phenyl, das substituiert sein kann, wie beispielsweise durch 1, 2 oder 3, bevorzugt 1 oder 2, Substituenten aus der Gruppe Chlor, Brom, Methyl, Ethyl, Methoxy, Ethoxy, Carboxy, Sulfo und Alkanoylamino von 2 bis 5 C-Atomen und/oder durch eine Gruppe der allgemeinen Formel $-SO_2-Y^1$ mit $Y^1$ der obengenannten Bedeutung, oder ist Naphthyl, das durch 1, 2 oder 3 Sulfo und gegebenenfalls durch 1 Alkyl von 1 bis 4 C-Atomen, 1 Alkoxy von 1 bis 4 C-Atomen, 1 Chlor oder 1 Alkanoylamino von 2 bis 5 C-Atomen und/oder durch eine Gruppe der allgemeinen Formel $-SO_2-Y^1$ mit $Y^1$ der obengenannten Bedeutung substituiert sein kann, und ist bevorzugt durch Sulfo und/oder eine Gruppe $-SO_2-Y^1$ substituiertes Phenyl;
- R* ist Wasserstoff oder Alkyl von 1 bis 4 C-Atomen, das durch Phenyl oder durch Sulfo und/oder $-SO_2-Y^1$ mit $Y^1$ der obigen Bedeutung substituiertes Phenyl substituiert sein kann, oder ist Alkanoyl von 2 bis 5 C-Atomen, wie Acetyl, oder gegebenenfalls substituiertes Phenylsulfonyl oder Benzoyl, wie Sulfobenzoyl, und ist bevorzugt Wasserstoff, Acetyl, Sulfobenzoyl oder Benzoyl;
- R" ist Wasserstoff oder Alkyl von 1 bis 4 C-Atomen, wie Ethyl und insbesondere Methyl, das durch Phenyl, Sulfophenyl oder eine Gruppe der Formel $-SO_2-Y^1$ mit $Y^1$ der obigen Bedeutung substituiert sein kann, oder ist Phenyl oder durch 1 oder 2 Substituenten aus

der Gruppe Alkyl von 1 bis 4 C-Atomen, Alkoxy von 1 bis 4 C-Atomen, Chlor, Brom, Sulfo und $-SO_2-Y^1$ mit $Y^1$ der obigen Bedeutung substituiertes Phenyl, und ist bevorzugt Alkyl von 1 bis 4 C-Atomen und insbesondere Wasserstoff;

$R^5$ ist Phenylureido, dessen Phenylrest durch eine Gruppe der Formel $-SO_2-Y^1$ mit $Y^1$ der obigen Bedeutung substituiert sein kann, oder ist Alkanoylamino von 2 bis 5 C-Atomen, das im Alkylrest durch eine Gruppe der Formel $-SO_2-Y^1$ mit $Y^1$ der obigen Bedeutung substituiert sein kann, oder ist Alkenoylamino von 3 bis 5 C-Atomen, wie Acryloylamino, oder ist Benzoylamino, das durch Substituenten aus der Gruppe Chlor, Methyl, Methoxy, Nitro, Sulfo, Carboxy und $-SO_2-Y^1$ mit $Y^1$ der obigen Bedeutung substituiert sein kann, und ist bevorzugt Acetylamino oder Benzoylamino;

$R^6$ ist Wasserstoff, Alkyl von 1 bis 4 C-Atomen, Alkoxy von 1 bis 4 C-Atomen, Sulfo, Carboxy, Carbalkoxy von 2 bis 5 C-Atomen, Halogen, wie Brom oder Chlor, oder Alkoxy von 1 bis 4 C-Atomen, das durch Hydroxy, Acetyloxy, Carboxy, Carbamoyl, Cyano, oder Halogen, wie Chlor, substituiert ist, und ist bevorzugt Wasserstoff, Sulfo, Methyl, Ethyl, Methoxy oder Ethoxy;

$R^7$ ist Wasserstoff, Alkyl von 1 bis 4 C-Atomen, Alkoxy von 1 bis 4 C-Atomen, Halogen, wie Brom oder Chlor, Cyano, Trifluormethyl, Alkoxy von 1 bis 4 C-Atomen, das durch Hydroxy, Acetyloxy, Carboxy, Carbamoyl, Cyano oder Halogen, wie Chlor, oder durch eine Gruppe der Formel $-SO_2-Y^1$ mit $Y^1$ der obigen Bedeutung substituiert ist, oder ist Alkanoylamino von 2 bis 5 C-Atomen, wie Acetylamino und Propionylamino, das durch Chlor, Brom, Alkoxy von 1 bis 4 C-Atomen, Phenoxy, Phenyl, Hydroxy, Carboxy oder Sulfo oder eine Gruppe der Formel $-SO_2-Y^1$ mit $Y^1$ der obigen Bedeutung substituiert sein kann, oder ist Alkenoylamino von 3 bis 5 C-Atomen, das durch Chlor, Brom, Carboxy oder Sulfo substituiert sein kann, oder ist Benzoylamino, das im Benzolkern substituiert sein kann, beispielsweise durch Substituenten aus der Gruppe Chlor, Methyl, Sulfo und eine Gruppe der Formel $-SO_2-Y^1$ mit $Y^1$ der obigen Bedeutung, oder ist Alkylsulfonyl von 1 bis 4 C-Atomen oder Phenylsulfonyl, das im Benzolkern substituiert sein kann, beispielsweise durch Substituenten aus der Gruppe Chlor, Methyl, Sulfo und eine Gruppe der Formel $-SO_2-Y^1$ mit $Y^1$ der obigen Bedeutung, oder ist Alkylsulfonylamino von 1 bis 4 C-Atomen, das durch Hydroxy, Sulfato, Chlor, Brom, Alkoxy von 1 bis 4 C-Atomen oder eine Gruppe der Formel $-SO_2-Y^1$ mit $Y^1$ der obigen Bedeutung substituiert sein kann, oder ist Phenylsulfonylamino, das im Benzolkern substituiert sein kann, beispielsweise durch Substituenten aus der Gruppe Chlor, Methyl, Sulfo und eine Gruppe der Formel $-SO_2-Y^1$ mit $Y^1$ der obigen Bedeutung, oder ist Carbamoyl, das am Stickstoffatom durch 1 oder 2 Substituenten mono- oder disubstituiert sein kann, wobei die Substituenten der Gruppe Alkyl von 1 bis 4 C-Atomen, durch Substituenten substituiertes Alkyl von 1 bis 4 C-Atomen (wie beispielsweise durch Hydroxy, Sulfo, Carboxy, Sulfato, Phenyl oder eine Gruppe der Formel $-SO_2-Y^1$ mit $Y^1$ der obigen Bedeutung), Cycloalkyl mit 5 bis 8 C-Atomen, Phenyl und durch Substituenten, beispielsweise aus der Gruppe Chlor, Sulfo, Methyl, Methoxy, Carboxy und eine Gruppe der Formel $-SO_2-Y^1$ mit $Y^1$ der obigen Bedeutung, substituiertes Phenyl angehören, oder ist Sulfamoyl, das am Stickstoffatom durch 1 oder 2 Substituenten mono- oder disubstituiert sein kann, wobei die Substituenten der Gruppe Alkyl von 1 bis 4 C-Atomen, durch Substituenten substituiertes Alkyl von 1 bis 4 C-Atomen (wie beispielsweise durch Hydroxy, Sulfo, Carboxy, Sulfato, Phenyl oder eine Gruppe der Formel $-SO_2-Y^1$ mit $Y^1$ der obigen Bedeutung), Cycloalkyl mit 5 bis 8 C-Atomen, Phenyl und durch Substituenten (beispielsweise aus der Gruppe Chlor, Sulfo, Methyl, Methoxy, Carboxy und eine Gruppe der Formel $-SO_2-Y^1$ mit $Y^1$ der obigen Bedeutung) substituiertes Phenyl angehören, oder ist Ureido oder Ureido, das am endständigen Stickstoffatom durch 1 oder 2 Substituenten mono- oder disubstituiert ist, wobei die Substituenten der Gruppe Alkyl von 1 bis 4 C-Atomen, durch beispielsweise Hydroxy, Sulfo, Carboxy, Sulfato, Phenyl oder eine Gruppe der Formel $-SO_2-Y^1$ mit $Y^1$ der obigen Bedeutungen substituiertes Alkyl von 1 bis 4 C-Atomen, Cycloalkyl mit 5 bis 8 C-Atomen, Phenyl und durch Substituenten (beispielsweise aus der Gruppe Chlor, Sulfo, Methyl, Methoxy, Carboxy und eine Gruppe der Formel $-SO_2-Y^1$ mit $Y^1$ der obigen Bedeutung) substituiertes Phenyl angehören, und ist bevorzugt Wasserstoff, Methyl, Ethyl, Methoxy, Ethoxy, Chlor, Amino, substituiertes Amino, Alkanoylamino von 2 bis 5 C-Atomen, Benzoylamino oder Phenylsulfonylamino und insbesondere Ureido, Acetylamino oder Benzoylamino;

$R^8$ ist Wasserstoff oder Alkyl von 1 bis 4 C-Atomen, wie Methyl oder Ethyl, das durch beispielsweise Hydroxy, Sulfo, Carboxy, Sulfato, eine Gruppe $-SO_2-Y^1$ mit $Y^1$ der obigen Bedeutung, Phenyl oder Sulfophenyl substituiert sein kann, oder ist Alkenyl von 2 bis 4 C-Atomen, das durch Carboxy, Sulfo, Chlor oder Brom substituiert sein kann, oder ist Cycloalkyl von 5 bis 8 C-Atomen, und ist bevorzugt Wasserstoff, Methyl, Ethyl oder durch Hydroxy, Sulfo, Sulfato oder Carboxy substituiertes Alkyl von 2 bis 4 C-Atomen, wie $\beta$-Sulfatoethyl oder $\beta$-Sulfoethyl;

$R^9$ ist Wasserstoff oder Alkyl von 1 bis 4 C-Atomen, das substituiert sein kann, beispielsweise durch Hydroxy, Sulfo, Carboxy, Sulfato, Phenyl oder $-SO_2-Y^1$ mit $Y^1$ obiger Bedeutung, oder ist Alkenyl von 2 bis 5 C-Atomen, das durch Carboxy, Sulfo oder $-SO_2-Y^1$ mit $Y^1$ obiger Bedeutung oder durch Chlor oder Brom substituiert sein kann, oder $R^9$ ist Cycloalkyl von 5 bis 8 C-Atomen oder Phenyl, das substituiert sein kann, beispielsweise durch Substituenten aus der Gruppe Chlor, Sulfo, Methyl, Methoxy, Carboxy und $-SO_2-Y^1$ mit $Y^1$ obiger Bedeutung, oder ist Naphthyl, das durch 1, 2 oder 3 Sulfo oder durch 1 oder 2 Sulfo und 1 oder 2 Gruppen der Formel $-SO_2-Y^1$ mit $Y^1$ der obigen Bedeutung oder nur durch eine solche Gruppe $-SO_2-Y^1$ substituiert ist, und ist bevorzugt Wasserstoff, Methyl, Ethyl oder Alkyl von 2 bis 4 C-Atomen, das durch Hydroxy, Sulfo, Sulfato oder Carboxy substituiert ist, wie $\beta$-Sulfatoethyl oder $\beta$-Sulfoethyl, oder

$R^8$ und $R^9$ stellen zusammen mit dem Stickstoffatom und gegebenenfalls einem weiteren Heteroatom oder einer Heterogruppe, wie N, O, S und NH, einen 5- bis 8-gliedrigen, bevorzugt gesättigten, heterocyclischen Rest dar, wie beispielsweise den N-Piperidino-, N-Morpholino- oder N-Piperazino-Rest;

$R^{10}$ ist Wasserstoff oder Alkyl von 1 bis 4 C-Atomen oder durch Alkoxy von 1 bis 4 C-Atomen oder Cyano substituiertes Alkyl von 1 bis 4 C-Atomen, bevorzugt Wasserstoff oder insbesondere Methyl;

$R^{11}$ ist Wasserstoff, Carboxy, Sulfo, Sulfoalkyl mit einem Alkylenrest von 1 bis 4 C-Atomen, wie Sulfomethyl, Cyano oder Carbamoyl, bevorzugt Wasserstoff, Cyano, Sulfomethyl, Carbamoyl oder Carboxy;

$B^3$ ist Wasserstoff oder Alkyl von 1 bis 6 C-Atomen, bevorzugt von 1 bis 4 C-Atomen, das durch Phenyl, Sulfo, Sulfophenyl oder $-SO_2-Y^1$ mit $Y^1$ obiger Bedeutung substituiert sein kann, und ist bevorzugt Wasserstoff, Methyl, Ethyl oder durch Sulfo, Phenyl oder Sulfophenyl substituiertes Alkyl von 2 bis 4 C-Atomen, wie $\beta$-Sulfoethyl;

$B^4$ ist Wasserstoff, Alkyl von 1 bis 4 C-Atomen oder durch Alkoxy von 1 bis 4 C-Atomen, Sulfo, Carboxy, Sulfato, Phenyl, Sulfophenyl, Acetylamino, Benzoylamino, Cyano oder eine Gruppe der Formel $-SO_2-Y^1$ mit $Y^1$ obiger Bedeutung substituiertes Alkyl von 1 bis 4 C-Atomen oder ist Alkenyl von 2 bis 4 C-Atomen, Cyclohexyl, Phenyl oder durch Substituenten aus der Gruppe Carboxy, Sulfo, Benzoylamino, Acetylamino` $-SO_2-Y^1$ mit $Y^1$ obiger Bedeutung und Chlor substituiertes Phenyl, und ist bevorzugt Wasserstoff, Methyl, Ethyl oder durch Sulfo, Phenyl oder Sulfophenyl substituiertes Alkyl von 2 bis 4 C-Atomen;

k ist die Zahl Null oder 1 (wobei im Falle k = Null diese Gruppe für Wasserstoff steht);

m ist die Zahl 1 oder 2;

$m_1$ ist die Zahl 1, 2 oder 3;

D* ist eine Gruppe der allgemeinen Formel (2), (2a) oder (2b) oder ist Phenyl, das durch 1, 2 oder 3, bevorzugt 1 oder 2, Substituenten aus der Gruppe Alkyl von 1 bis 4 C-Atomen, Alkoxy von 1 bis 4 C-Atomen, Chlor, Brom, Hydroxy, Carboxy, Sulfo, Carbamoyl, Sulfamoyl und Alkanoylamino von 2 bis 5 C-Atomen, bevorzugt hiervon Methyl, Methoxy, Ethoxy, Chlor, Sulfo, Carboxy und Hydroxy, und/oder durch eine Gruppe der Formel $-SO_2-Y^1$ mit $Y^1$ der obengenannten Bedeutung substituiert sein kann, wobei bevorzugt einer dieser Substituenten eine Sulfo- oder Carboxygruppe ist und die Gruppe $-SO_2-Y^1$ bevorzugt in meta- oder para-Stellung zur Azogruppe steht, oder D* ist Naphthyl, das durch 1, 2 oder 3 Sulfogruppen oder durch 1 oder 2 Sulfogruppen und 1 oder 2 Gruppen der allgemeinen Formel $-SO_2-Y^1$ mit $Y^1$ der obengenannten Bedeutung oder nur durch eine solche Gruppe $-SO_2-Y^1$ substituiert ist,

wobei D und D* zueinander gleiche oder voneinander verschiedene Bedeutungen besitzen können;

K* ist ein Rest aus einer der oben genannten und definierten allgemeinen Formeln (3a) bis (3m), wobei K und K* zueinander gleiche oder voneinander verschiedene Bedeutungen

besitzen können;

M hat eine der oben genannten Bedeutungen.

Die einzelnen Formelglieder, auch die gegebenenfalls in ein und derselben Formel zweifach auftretenden Formelglieder, können zueinander gleiche oder voneinander verschiedene Bedeutungen besitzen.

Die in den obigen Formeln (3e), (3f), (3g), (3h), (3i) und (3n) befindlichen freien Bindungen, welche zur Azogruppe führen, bzw. die Azogruppe in Formel (3p) und (3q) befinden sich in ortho-Stellung zur Hydroxy- bzw. Aminogruppe gebunden. Bevorzugt steht diese Hydroxygruppe in α-Stellung an den Naphthalinrest gebunden.

Alkylgruppen von 1 bis 4 C-Atomen sind bevorzugt die Ethyl- und insbesondere die Methylgruppe; Alkoxygruppen von 1 bis 4 C-Atomen sind bevorzugt die Ethoxy- und insbesondere die Methoxygruppe; Alkanoylaminogruppen von 2 bis 5 C-Atomen sind bevorzugt die Propionylaminogruppe und insbesondere die Acetylaminogruppe und Carbalkoxygruppen von 2 bis 5 C-Atomen sind bevorzugt die Carbomethoxy- und Carbethoxygruppe.

Von den erfindungsgemäßen Verbindungen der allgemeinen Formel (1) sind insbesondere diejenigen mit K gleich einem Rest der Formel (3a) bis (3q) bevorzugt und insbesondere solche bevorzugt, in welchen K einen Rest der allgemeinen Formel (3c), (3f), (3h), (3i), (3p) oder (3q) bedeutet, in welchen wiederum die einzelnen Formelglieder die folgenden bevorzugten Bedeutungen besitzen:

$B^1$ ist Carboxy oder Methyl;

Q ist Phenyl, das durch 1 oder 2 Substituenten substituiert sein kann, die aus der folgenden Menge an Substituenten ausgewählt sind: 2 Methyl, 2 Methoxy, 1 Chlor oder Brom, 2 Sulfo, 1 Carboxy und 1 Vinylsulfonyl oder β-Sulfatoethylsulfonyl;

$R^5$ ist Acetylamino, Propionylamino oder Benzoylamino, das durch 1 oder 2 Substituenten aus der Gruppe Chlor, Methyl, Methoxy, Nitro, Sulfo und β-Sulfatoethylsulfonyl substituiert sein kann;

$R^6$ hat eine der obengenannten, insbesondere bevorzugten Bedeutungen;

$R^7$ ist Ureido, Acetylamino oder Benzoylamino;

$R^8$ und $R^9$ sind beide Wasserstoff;

R* ist Wasserstoff, Acetyl oder Benzoyl;

R'' ist Wasserstoff;

m ist in Formeln (3p) und (3q) die Zahl 2, und die eine Gruppe $-SO_3M$ steht in meta-Stellung zur Hydroxygruppe und die andere Gruppe $-SO_3M$ in meta- oder para-Stellung zur Aminogruppe.

Die vorliegende Erfindung betrifft weiterhin Verfahren zur Herstellung der erfindungsgemäßen Azoverbindungen der allgemeinen Formel (1), beispielsweise durch Kupplungsreaktion der Diazoniumverbindung einer Aminoverbindung der allgemeinen Formel (4)

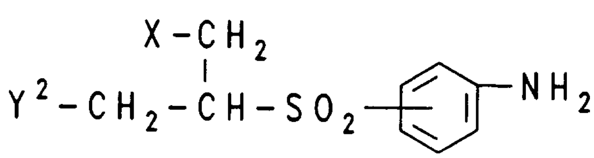

(4)

in welchen X die obengenannte Bedeutung hat und $Y^2$ die Hydroxygruppe ist oder eine der Bedeutungen von Y besitzt, wobei $Y^2$ bevorzugt Chlor, Brom, Acetyloxy oder Sulfato und insbesondere bevorzugt Chlor, Brom oder Acetyloxy ist, mit einer Kupplungskomponente der allgemeinen Formel H-K mit K der obengenannten Bedeutung; sofern K, wie oben angegeben, eine bivalente Kupplungskomponente ist, kann eine Disazoverbindung, sofern diese erwünscht ist, durch Umsetzung dieser bivalenten Kupplungskomponente mit der zweifach äquimolaren Menge der Diazokomponente hergestellt werden. Im Falle der Verwendung einer Verbindung der Formel (4) mit $Y^2$ gleich einer Hydroxygruppe wird die Hydroxygruppe in der gebildeten Azoverbindung in eine Gruppe Y der erfindungsgemäßen Azoverbindung (1), wie später noch angegeben, übergeführt.

Die Diazotierungs- und Kupplungsreaktionen erfolgen in üblicher und altbekannter Weise, so die Diazotierung des Amins (4) in der Regel bei einer Temperatur zwischen -5 °C und + 15 °C und einem pH-Wert unterhalb von 2 mittels einer starken Säure und Alkalinitrit in bevorzugt wäßrigem Medium und die Kupplungsreaktion in der Regel bei einer Temperatur zwischen 0 und 35 °C und bei einem pH-Wert zwischen 1,5 und 4,5 im Falle einer aminogruppenhaltigen Kupplungskomponente und bei einem pH-Wert zwischen 3 und 7,5 im Falle einer hydroxygruppenhaltigen Kupplungskomponente, bevorzugt in wäßrigem

Medium. Ist die Kupplungskomponente eine bivalente, doppelankuppelbare Verbindung, enthält sie beispielsweise eine kupplungsfähige Aminogruppe und gleichzeitig eine kupplungsfähige Hydroxygruppe, so kann zur Herstellung einer Disazoverbindung die Kupplung zunächst mit dem ersten Mol der Diazoniumverbindung des Amins im sauren pH-Bereich zur Monoazoverbindung erfolgen und die zweite Kupplungsreaktion mit dem zweiten Mol der Diazoniumverbindung des Amins anschließend im schwach sauren bis schwach alkalischen Bereich. Diese Verfahrensweise gilt beispielsweise für die Verbindungen entsprechend den allgemeinen Formeln (3p) und (3q), so durch Kupplung der Aminonaphtholsulfonsäure zunächst mit dem ersten Mol der Diazoniumverbindung des Amins der allgemeinen Formel (4) oder eines anderen aromatischen Amins entsprechend der allgemeinen Formel $D^*-NH_2$ mit D* der obengenannten anderen Bedeutung als D im sauren Medium und sodann durch Kupplung der gebildeten Monoazoverbindung mit dem Zweiten Mol einer Diazoniumverbindung eines Amins $D^*-NH_2$ mit D* der obengenannten Bedeutung im schwach sauren, neutralen oder schwach alkalischen Bereich, wobei D* Zwingend eine der für D angegebenen Bedeutungen besitzt, sofern die erste Kupplungsreaktion nicht mit einer Diazoniumverbindung eines Amins (4) durchgeführt wurde; insbesondere führt man die erste Kupplungsreaktion zunächst bei einem pH-Wert Zwischen etwa 0,5 und 2,5 und die zweite Kupplungsreaktion bei einem pH-Wert zwischen 4 und 6,5 durch. Sofern die Diazoniumverbindungen in beiden Kupplungsreaktionen identisch sind, also solche der Aminoverbindung (4), kann die erste und zweite Kupplungsreaktion in ein und demselben Ansatz, zunächst im sauren Bereich und sodann im schwach sauren bis schwach alkalischen Bereich, durchgeführt werden. Zur Herstellung einer Disazoverbindung entsprechend der allgemeinen Formel (3r) erfolgt die Umsetzung der Kupplungskomponente Resorcin mit der bzw. den Diazoniumverbindung(en) vorteilhaft zunächst bei einem pH-Wert zwischen 0,8 und 2 und sodann bei einem pH-Wert zwischen 6 und 7,5.

Disazoverbindungen entsprechend der allgemeinen Formel (1), deren Rest K dem Rest einer Azoverbindung entspricht, welcher aus einer kupplungsfähigen Diazokomponente und einer Kupplungskomponente zusammengesetzt ist, wie beispielsweise ein Rest entsprechend der allgemeinen Formel (3s) oder (3t), lassen sich auch erfindungsgemäß in der Weise herstellen, daß man zunächst die Diazoniumverbindung eines Amins (4) mit der aminogruppenhaltigen und somit diazotierbaren Kupplungskomponente, wie beispielsweise in den Formeln (3s) und (3t) die durch die Substituenten $R^6$ und $R^7$ substituierten Anilin- bzw. Sulfo-aminonaphthalin-Komponenten, kuppelt und in der so gebildeten Amino-Azoverbindung die Aminogruppe diazotiert und mit einer Kupplungskomponente, wie beispielsweise der Kupplungskomponente H-K* , zur Disazoverbindung kuppelt.

Alle diese Umsetzungsmöglichkeiten zur Synthese von Disazoverbindungen sind analog den in der Literatur bekannten oder dem Fachmann geläufigen Methoden zur Synthese von Disazoverbindungen.

Kupplungskomponenten, die zur Herstellung der erfindungsgemäßen Farbstoffe verwendet werden können und beispielsweise den allgemeinen Formeln (3a) bis (3n) entsprechen, sind beispielsweise: 1,3-Diamino-benzol-5-sulfonsäure, Phenol, Kresol, Resorcin, 2-Ethoxy-phenol, 4-Methylphenol, 3-Sulfophenol, Salicylsäure, 3-Sulfo-1-naphthol, 4-Sulfo-1-naphthol, 5-Sulfo-1-naphthol, 3,6-Disulfo-8-naphthol, 4,6-Disulfo-8-naphthol,1-Naphthol-3,8-disulfonsäure, 1-Amino-8-naphthol-4-sulfonsäure, 1-Amino-8-naphthol-5-sulfonsäure, 1-Amino-8-naphthol-2,4-disulfonsäure, 2-Amino-5-naphthol-7-sulfonsäure, 2-Amino-5-naphthol-1,7-disulfonsäure, 1-Amino-5-naphthol-7-sulfonsäure, 2-Amino-8-naphthol-6-sulfonsäure, 2-Amino-8-naphthol-3,6-disulfonsäure, 2-Amino-8-naphthol-4,6-disulfonsäure, 1-Amino-8-naphthol-3,6- oder -4,6-disulfonsäure, 1-Acryloyl-amino-8-naphthol-3,6- oder -4,6-disulfonsäure, 1-Propionyl-amino-8-naphthol-3,6- oder 4,6-disulfonsäure, 1-Acetylamino-8-naphthol-4-sulfonsäure, 1-Acetylamino-8-naphthol-3,6- oder -4,6-disulfonsäure, 1-Benzoylamino-8-naphthol-3,6- oder -4,6-disulfonsäure, 2-Naphthol-5,7-disulfonsäure, 2-Naphthol-3,6- und -6,8-disulfonsäure, 1,8-Dihydroxynaphthalin-3,6-disulfonsaure, 1,8-Dihydroxynaphthalin-6-sulfonsäure, 1-Naphthol-3,6,8-trisulfonsäure,2-Acetylamino-5-naphthol-7-sulfonsäure, 2-Benzoylamino-8-naphthol-6-sulfonsäure, 2-(p'-Tosylamino)-5-naphthol-7-sulfonsäure, 2-Acetylamino-8-naphthol-3,6-disulfonsäure, 2-Acetylamino-5-naphthol-1,7-disulfonsäure, 3-Benzoylamino-8-naphthol-6-sulfonsäure, 2-Phenylsulfonylamino-5-naphthol-7-sulfonsäure, 2-(N-Methyl-N-acetyl)-amino-8-naphthol-6-sulfonsäure, N-Ethyl-N-benzyl-anilin-3-sulfonsäure, N,N-Bis-($\beta$-hydroxyethyl)-anilin, N,N-Bis-($\beta$-sulfatoethyl)-anilin, N,N-Bis-($\beta$-hydroxyethyl)-2-methoxy-5-chlor-anilin, N-($\beta$-Sulfatoethyl)-2-5-dimethoxyanilin, N-($\beta$-Sulfatoethyl)-2-chloranilin, Acetoacetyl-2-naphthylamid-5-sulfonsäure, N-Acetoacetylanilin-3- oder -4-sulfonsäure, N-Acetoacetyl-2-methoxy-5-sulfo-anilin, N-Acetoacetyl-4-methoxy-3-sulfoanilin, N-Acetoacetyl-2-methoxy-5-methyl-4-sulfo-anilin, N-Acetoacetyl-2,5-dimethoxy-4-sulfo-anilin, N-Acetoacetyl-2-methoxy-5-methyl-4-($\beta$-sulfatoethylsulfonyl)-anilin, N-Acetoacetyl-2,5-dimethoxy-4-($\beta$-sulfatoethylsulfonyl)-anilin, N-Acetoacetyl-2-methoxy-5-($\beta$-sulfatoethylsulfonyl)-anilin, N-Acetoacetyl-4-($\beta$-sulfatoethylsulfonyl)-anilin, N-Acetoacetyl-3-(($\beta$-sulfatoethylsulfonyl)-anilin, 1-(4'-$\beta$-sulfatoethylsulfonyl-phenyl)-3-methyl-pyrazolon(5), 1-(4'-$\beta$-sulfatoethylsulfonyl-phenyl)-3-carboxy-pyrazolon(5), 1-(4'-Sulfophenyl)-3-methyl-pyrazolon(5), 1-(4'-Sulfophenyl)-3-carboxy-pyrazolon(5), 1-(2'-Chlor-5'sulfo-phenyl)-3-methyl- oder -3-carboxy-pyrazolon(5), 1-(3'-Sulfophenyl)-3-carboxypyrazolon(5),1-(2'-Methoxy-4'-sulfophenyl)-3-carboxy-pyrazolon(5), 1-(3'-Sulfophenyl)-3-methyl-5-amino-pyrazol, 1-(4'-Sulfo-phenyl)-3-methyl-5-amino-pyrazol, 1-(2'-Methoxy-5'-sulfo-phenyl)-3-methyl-5-aminopyrazol, 1-(2'-Methoxy-5'-methyl-4'-sulfo-phenyl)-3-methyl-5-aminopyrazol, 1-(2'-Chlor-5'-sulf-phenyl)-3-methyl-5-aminopyrazol, 1-(3'-Amino-4'-sulfo-phenyl)-3-carbethoxy-pyrazolon(5), 1-(4'-β-sulfatoethylsulfonyl-phenyl)-3-carbethoxy-pyrazolon(5), 1-(3'-Amino-6'-methyl-phenyl)-3-carboxy-pyrazolon-(5), 2-N-Methylamino-8-naphthol-6-sulfonsäure, 3-Carboxy-pyrazolon(5),1-Phenyl-3-carboxy-pyrazolon(5), 1-(4'-Nitrophenyl)-3-carboxy-pyrazolon(5), 1-(3'-Acetylaminophenyl)-3-carboxy-pyrazolon(5), 1-(3'-Carboxyphenyl)-3-methyl-pyrazolon(5), 2-Hydroxy-3-carboxy-naphthalin, 2-Hydroxy-6-carboxy-naphthalin, 8-Hydroxy-chinolin-5-sulfonsäure, 1,4-Dimethyl-2-hydroxy-6-pyridon-5-sulfonsäure, N-Sulfomethyl-anilin, 3-Acetylamino-5-naphthol-7-sulfonsäure, 2-Methylamino-8-naphthol-6-sulfonsäure, 1-(β-Hydroxyethyl)-4-methyl-6-hydroxy-2-pyridon, 1-(β-Hydroxyethyl)-3-cyano-4-methyl-6-hydroxy-2-pyridon, 1-(β-Hydroxyethyl)-3-carbamoyl-4-methyl-6-hydroxy-2-pyridon, 1-(β-Hydroxyethyl)-4-methyl-6-hydroxy-2-pyridon-3-sulfonsäure, 1-(β-Sulfatoethyl)-4-methyl-6-hydroxy-2-pyridon, 1-(β-Sulfatoethyl)-3-cyano-4-methyl-6-hydroxy-2-pyridon, 1-(β-Sulfatoethyl)-3-carbamoyl-4-methyl-6-hydroxy-2-pyridon, 1-(β-Sulfatoethyl)-4-methyl-6-hydroxy-2-pyridon-3-sulfonsäure, 1-(β-Sulfatoethyl)-4-methyl-6-hydroxy-2-pyridon, 1-(β-Sulfatoethyl)-3-carbamoyl-4-methyl-6-hydroxy-2-pyridon, 1-(β-Sulfatoethyl)-4-methyl-6-hydroxy-2-pyridon-3-sulfonsäure, 1-Carboxymethyl-4-methyl-6-hydroxy-2-pyridon, 1-Carboxymethyl-3-cyano-4-methyl-6-hydroxy-2-pyridon, 1-Carboxymethyl-3-carbamoyl-4-methyl-6-hydroxy-2-pyridon, 1-Carboxymethyl-4-methyl-6-hydroxy-2-pyridon-3-sulfonsäure, 1-(β-Carboxyethyl)-4-methyl-6-hydroxy-2-pyridon, 1-(β-Carboxyethyl)-3-cyano-4-methyl-6-hydroxy-2-pyridon, 1-(β-Carboxyethyl)-3-carbamoyl-4-methyl-6-hydroxy-2-pyridon, 1-(β-Carboxyethyl)-4-methyl-6-hydroxy-2-pyridon-2-sulfonsäure, 1-(β-Acetylaminoethyl)-4-methyl-6-hydroxy-2-pyridon, 1-(β-Acetylaminoethyl)-3-cyano-4-methyl-6-hydroxy-2-pyridon, 1-(β-Acetylaminoethyl)-3-carbamoyl-4-methyl-6-hydroxy-2-pyridon, 1-(β-Acetylaminoethyl)-4-methyl-6-hydroxy-2-pyridon-3-sulfonsäure, 1-(β-Acetylaminopropyl)-4-methyl-6-hydroxy-2-pyridon, 1-(β-Acetylaminopropyl)-3-cyano-4-methyl-6-hydroxy-2-pyridon, 1-(β-Acetylamino-propyl)-3-carbamoyl-4-methyl-6-hydroxy-2-pyridon, 1-(β-Acetylamino-propyl)-4-methyl-6-hydroxy-2-pyridon-3-sulfonsäure, 4-Hydroxy-chinolin-(2), 1-Amino-8-hydroxy-2-(phenylazo)-naphthalin-3,6-disulfonsäure, 1-Amino-8-hydroxy-2-(4'-sulfophenylazo)-naphthalin-3,6-disulfonsäure, 1-Amino-8-hydroxy-2-(2',5'-disulfophenylazo)-naphthalin-3,6-disulfonsäure, 1-(β-Aminoethyl)-3-cyano-4-methyl-6-hydroxy-2-pyridon, 1-(γ-Aminopropyl)-3-sulfomethyl-4-methyl-6-hydroxy-2-pyridon, 1,3-Diaminobenzol, 1-Amino-3-(N,N-di-β-hydroxyethylamino)-benzol, 1-Amino-3-(N,N-di-β-sulfatoethylamino)-benzol, 1-Amino-(3-N,N-di-β-hydroxyethylamino)-4-methoxybenzol, 1-Amino-3-(N,N-di-β-sulfatoethylamino)-4-methoxy-benzol, 1-Amino-3-(sulfo-benzylamino)-benzol, 1-Amino-3-(sulfobenzylamino)-4-chlorobenzol, 1-Amino-3-(N,N-di-sulfobenzylamino)-benzol, 1-Hydroxy-3- oder -4-methyl-benzol, 1-Hydroxy-benzol-4-sulfosäure, 1-Hydroxynaphthalin, 2-Hydroxynaphthalin, 2-Hydroxynaphthalin-6- oder -7-sulfonsäure, 1-Hydroxynaphthalin-4,7-disulfonsäure, 1-Amino-3-methyl-benzol, 1-Amino-2-methoxy-5-methyl-benzol, 1-Amino-2,5-dimethyl-benzol, 3-Aminophenyl-harnstoff, 1-Amino-3-acetylamino-benzol, 1-Amino-3-(hydroxyacetylamino)-benzol, 1,3-Diaminobenzol-4-sulfonsäure, 1-Amino-naphthalin-6- oder -8-sulfonsäure, 1-Amino-2-methoxy-naphthalin-6-sulfonsäure, 2-Amino-naphthalin-5,7-disulfonsäure, 1-Amino-8-hydroxy-naphthalin-6-sulfonsäure, 2-Hydroxy-3-aminonaphthalin-5,7-disulfonsäure, 1-Amino-8-hydroxy-naphthalin-2,4,6-trisulfonsäure, 1-Hydroxy-8-acetylamino-naphthalin-3-sulfonsäure, 1-Benzoylamino-8-hydroxy-naphthalin-3,6- oder -4,6-disulfonsäure, 2-Benzoylamino-5-hydroxy-naphthalin-7-sulfonsäure, 2-Methyl- und 2-Ethylamino-5-hydroxy-naphthalin-7-sulfonsäure, 2-(N-Acetyl-N-methylamino)-5-hydroxy-naphthalin-7-sulfonsäure, 2-Ethylamino-8-hydroxy-naphthalin-6-sulfonsäure, 2-Acetylamino-8-hydroxy-naphthalin-6-sulfonsäure, 1-(4'-Aminobenzoylamino)-8-hydroxynaphthalin-3,6- und -4,6-disulfonsäure, 1-(4'-Nitrobenzoylamino)-8-hydroxy-naphthalin-3,6- und -4,6-disulfonsäure, 1-(3'-Amino-benzoylamino)-6-hydroxy-naphthalin-3,6- und -4,6-disulfonsäure, 1-(3'-Nitrobenzoylamino)-8-hydroxy-naphthalin-3,6-und -4,6-disulfonsäure, 2-(4'-Amino-3'-sulfophenyl)-amino-5-hydroxy-naphthalin-7-sulfonsäure, 3-Methyl-5-pyrazolon, 1-Phenyl-3-methyl-5-pyrazolon, 1-(3'-Aminophenyl)-3-methyl-5-pyrazolon, 1-(2',5'-Disulfophenyl)-3-methyl-5-pyrazolon, 1-(2'-Methyl-4'-sulfophenyl)-5-pyrazolon-3-carbonsäure, 1-(4',8'-Disulfonaphth-2'-yl)-3-methyl-5-pyrazolon, 1-(5',7'-Disulfonaphth-2'-yl)-3-methyl-5-pyrazolon, 1-(2',5'-Dichlor-4'-sulfophenyl)-3-methyl-5-pyrazolon, 3-Aminocarbonyl-4-methyl-6-hydroxy-2-pyridon, 1 -Ethyl-3-cyano- oder 3-chlor-4-methyl-6-hydroxy-2-pyridon, 1-Ethyl-3-sulfomethyl-4-methyl-6-hydroxy-2-pyridon, 2,4,6-Triamino-3-cyano-pyridin, 2-(3'-Sulfophenyl)-amino-4,6-diamino-3-cyano-pyridin, 2-(2'-Hydroxyethylamino)-3-cyano-4-methyl-6-amino-pyridin, 2,6-Bis-(2'-hydroxyethylamino)-3-cyano-4-methyl-pyridin, 1-Ethyl-3-carbamoyl-4-methyl-6-hydroxy-2-pyridon, 1-Ethyl-3-sulfomethyl-4-methyl-5-carbamoyl-6-hydroxy-2-pyridon,N-Acetoacetylaminobenzol, 5-Acetylamino-2-sulfo-anilin.

Die erfindungsgemäß zur Synthese der erfindungsgemäßen Azoverbindungen (1) einsetzbaren Verbindungen entsprechend den allgemeinen Formel (4) sind bisher noch nicht bekannt. Die Erfindung betrifft somit auch diese Verbindungen, Verfahren zu deren Herstellung und ihre Verwendung zur Synthese von Farbstoffen, insbesondere der erfindungsgemäßen Azoverbindungen (1). Sie lassen sich erfindungsgemäß

herstellen, indem man eine Verbindung der allgemeinen Formel (5)

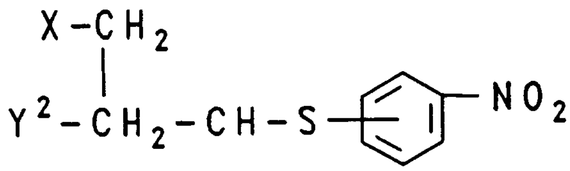

(5)

in welcher $Y^2$ und X eine der obengenannten Bedeutungen haben, wobei $Y^2$ insbesondere bevorzugt Chlor, Brom, Alkanoyloxy von 2 bis 5 C-Atomen, wie Acetyloxy, oder Cyano ist, zur Sulfonylverbindung oxidiert, wobei als Oxidationsmittel beispielsweise Kaliumpermanganat, Natrium- oder Kaliumperhydrogensulfat, organische Persäuren, wie beispielsweise Peroxyessigsäure, Natriumperoxid, Wasserstoffperoxid und Chlor in wäßriger Lösung verwendet werden. Die Oxidationsreaktion kann bei einer Temperatur zwischen 10 und 100 °C, bevorzugt zwischen 50 und 90 °C, erfolgen. Bevorzugt führt man die Oxidation der Verbindungen der allgemeinen Formel (5) zur entsprechenden Sulfonylverbindung der nachstehend angegebenen allgemeinen Formel (6) in wäßrig-organischem oder in rein organischem Medium durch, wobei als organische Lösemittel solche eingesetzt werden, die in Wasser löslich und mit Wasser mischbar und gegenüber den Oxidationsmitteln inert sind. Solche organischen Lösemittel sind beispielsweise Eisessig und Dioxan. Vorzugsweise verwendet man Eisessig, insbesondere in Mischung mit Wasser. Beispielsweise kann man die Oxidationsreaktion mittels Wasserstoffperoxid in wasserfreier Essigsäure oder in wasserhaltiger Essigsäure mit einem Wassergehalt von bis zu 20 Gew.-% bei einer Temperatur zwischen 30 und 100 °C, bevorzugt zwischen 60 und 90 °C, durchführen. Der Gehalt an Wasserstoffperoxid beträgt in der Regel zwischen 10 und 35 Gew.-%, bevorzugt zwischen 20 und 35 Gew.-%.

Die so erhältlichen Verbindungen der allgemeinen Formel (6)

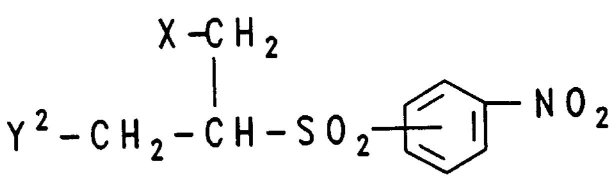

(6)

in welcher $Y^2$ und X die obengenannten Bedeutungen besitzen, können zu einer erfindungsgemäßen Anilinverbindung der allgemeinen Formel (4) reduziert werden, wie beispielsweise durch katalytische Reduktion mittels Wasserstoff in Gegenwart eines üblichen Edelmetallkatalysators oder in saurem, wäßrig-organischem Medium mittels unedler Metalle, wie Eisen oder Zink, wobei als wäßriges saures Medium bevorzugt wäßrige Salzsäure dient. Bevorzugt erfolgt die Reduktion in wäßrigem, saurem Medium oder in wasserfreien organischen Lösemitteln, wie beispielsweise Eisessig, mittels Palladium/Aktivkohle, bei einer Temperatur zwischen 10 und 100 °C, bevorzugt zwischen 20 und 50 °C.

Die Verbindungen der allgemeinen Formel (5) und (6) sind ebenfalls neu. Die Erfindung betrifft somit auch diese Verbindungen, Verfahren zu deren Herstellung und ihre Verwendung als Vorprodukte zur Synthese von Azoverbindungen. Die Verbindungen der allgemeinen Formeln (4), (5) und (6) können unter die allgemeine Formel (7)

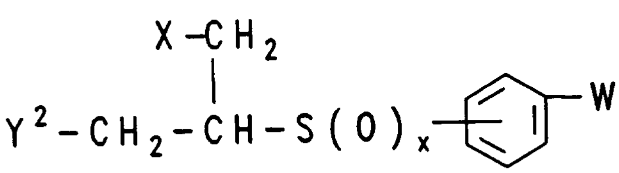

(7)

in welcher $Y^2$ und X die obengenannten Bedeutungen haben, x für die Zahl Null oder 2 steht und W die Nitro- oder Aminogruppe ist, zusammengefaßt werden.

Die Ausgangsverbindungen der allgemeinen Formel (5) lassen sich erfindungsgemäß herstellen, indem man ein Nitrobenzol-sulfenylhalogenid, vorzugsweise als Sulfenylchlorid, an eine Verbindung der allgemei-

nen Formel (8)

$Y^2 - CH_2 - CH = CH_2$ (8)

in welcher $Y^2$ die obengenannten Bedeutungen hat, anlagert. Die Umsetzung erfolgt wie beispielsweise in J.Org.Chem. 49, 1314 (1984) und J.Chem.Soc. 1968, 1339, beschrieben. Insbesondere erfolgt die erfindungsgemäße Umsetzung in inerten organischen Lösemitteln, wie beispielsweise Dichlormethan, Tetrachlorkohlenstoff, Chlorbenzol oder den Dichlorbenzolen, bei einer Temperatur zwischen -50°C und 0°C, bevorzugt zwischen -40°C und -25°C.

Die Nitrophenyl-sulfenylhalogenid-Ausgangsverbindungen selbst kann man analog bekannten Verfahrensweisen (s. bspw. Liebigs Ann. 400, 2 (1913)) herstellen, indem man von einem Nitrobenzol-thiol oder -thiolat oder einem Bis-(nitrophenyl)disulfid ausgeht und dieses in einem inerten Lösemittel, wie beispielsweise Dichlormethan, Chloroform, Tetrachlorkohlenstoff, Chlorbenzol oder einem Dichlorbenzol oder einem isomeren Dichlorbenzolgemisch, bevorzugt in Dichlormethan, Chloroform, Chlorbenzol und Dichlorbenzol, bei einer Temperatur zwischen -80°C und +20°C, bevorzugt zwischen -50°C und -20°C, mit einem Halogenierungsmittel umsetzt. Solche Halogenierungsmittel sind bevorzugt Chlor, Brom, N-Chlor-succinimid und N-Brom-succinimid.

Die Nitrobenzol-isopropyl-sulfon-Verbindungen entsprechend der allgemeinen Formel (6), in welcher $Y^2$ eine der obengenannten Bedeutungen besitzt und bevorzugt ein Halogenatom, wie Brom und insbesondere Chlor, die Cyanogruppe oder eine Alkanoyloxygruppe von 2 bis 5 C-Atomen, wie die Acetyloxygruppe, ist, lassen sich auch in einfacher Weise dadurch herstellen, daß man Thiophenol mit Chlor zum Phenylsulfenylchlorid umsetzt und dieses an eine Olefinverbindung der obengenannten und definierten allgemeinen Formel (8) zur Verbindung der allgemeinen Formel (9)

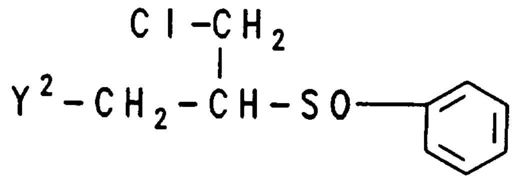

(9)

in welcher $Y^2$ die obengenannte Bedeutung hat, anlagert, diese Verbindung der Formel (9) sodann mittels Wasserstoffperoxid zur Verbindung der allgemeinen Formel (10)

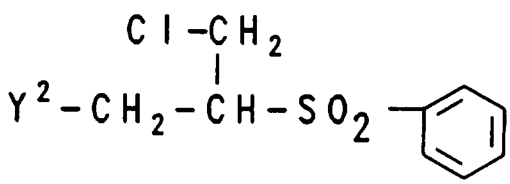

(10)

mit $Y^2$ der obengenannten Bedeutung oxidiert und diese sodann analog bekannten Verfahrensweisen der Nitrierung von Benzolverbindungen zu einer metaNitrobenzol-isopropylsulfonyl-Verbindung entsprechend der allgemeinen Formel (6) nitriert.

Hierbei erfolgt die Umsetzung des Thiophenols mit Chlor bei einer Temperatur zwischen -20°C und +20°C, bevorzugt zwischen -10°C und + 10°C, in einem gegenüber den Reaktanten inerten organischen Lösemittel, bevorzugt einem halogensubstituierten Kohlenwasserstoff, wie beispielsweise Dichlormethan und Chlorbenzol. Ohne das erhaltene Phenylsulfenylchlorid zu isolieren, kann man es im gleichen Ansatz mit dem Olefin der allgemeinen Formel (8) umsetzen, wobei man auch hier die Reaktion in einem gegenüber den Reaktanten inerten Lösemittel, wie beispielsweise den obengenannten, bei einer Temperatur zwischen -50°C und 0°C, bevorzugt zwischen -45°C und -35°C, durchführt. Das Lösemittel wird nach beendeter Reaktion abdestilliert und die erhaltene Verbindung der allgemeinen Formel (9) entweder gereinigt oder direkt dem Oxidationsprozeß unterworfen. Dieser erfolgt ebenfalls in einem gegenüber den Reaktanten inerten, mit Wasser mischbaren organischen Lösemittel, wie beispielsweise einer Alkancarbonsäure, bevorzugt einer solchen mit einem Alkylrest von 1 bis 4 C-Atomen, wie insbesondere Essigsäure, bei einer Temperatur zwischen 50°C und 100°C, bevorzugt zwischen 60 und 90°C, mittels Wasserstoffperoxid, das als 10 bis 40 gew.-%ige, bevorzugt 25 bis 35 gew.-%ige, wäßrige Lösung, in die Reaktion eingesetzt wird;

hierbei wird ein üblicher Oxidationskatalysator, wie beispielsweise ein Alkalimetall-wolframat, mitverwendet. Nach dem Abkühlen des Oxidationsansatzes kann die erhaltene Verbindung der allgemeinen Formel (10), gegebenenfalls nach Zusatz von Wasser, aus dem Ansatz abfiltriert werden. Eine weitere Reinigung ist nicht mehr erforderlich. Diese Sulfonylverbindung der allgemeinen Formel (10) wird sodann mittels einer sog. Nitriersäure, einem Gemisch aus konzentrierter Schwefelsäure und Salpetersäure mit bevorzugt einem Salpetersäuregehalt von etwa 30 Gew.-%, bei einer Temperatur zwischen 30 und 70 °C, bevorzugt zwischen 40 und 50 °C, nitriert.

Sowohl die Verbindungen entsprechend der allgemeinen Formel (1) als auch die der allgemeinen Formeln (4), (5) und (6), in welchen Y bzw. $Y^2$ die Hydroxygruppe bedeuten, können in üblicher und bekannter Verfahrensweise in Verbindungen übergeführt werden, in welchen Y bzw. $Y^2$ eine andere Bedeutung als die Hydroxygruppe besitzt, so beispielsweise in deren Esterderivate, wie beispielsweise von mehrwertigen anorganischen Säuren oder von aliphatischen und aromatischen Carbon- oder Sulfonsäuren, so beispielsweise in Verbindungen, in welchen Y bzw. $Y^2$ für ein Chloratom oder eine Sulfato-, Phosphato-, Acetyloxy- oder Toluylsulfonyloxygruppe steht.

Hierfür geeignete Veresterungs- und Acylierungsmittel sind beispielsweise die entsprechenden anorganischen oder organischen Säuren oder deren Anhydride, Halogenide oder Amide, wie beispielsweise Schwefelsäure, Schwefeltrioxid enthaltende Schwefelsäure, Chlorsulfonsäure, Amidosulfonsäure, Phosphorsäure, Phosphoroxychlorid, Gemische aus Phosphorsäure und Phosphorpentoxid, Acetanhydrid, Toluolsulfochlorid und Thionylchlorid.

Die Abscheidung und Isolierung der Verbindungen (1) aus den wäßrigen Syntheselösungen kann nach allgemein bekannten Methoden für wasserlösliche Verbindungen erfolgen, so beispielsweise durch Ausfällen aus dem Reaktionsmedium mittels eines Elektrolyten, wie beispielsweise Natriumchlorid oder Kaliumchlorid, oder aber durch Eindampfen der Reaktionslösung selbst, beispielsweise durch Sprühtrocknung. Falls die letztgenannte Art der Isolierung gewählt wird, empfiehlt es sich vielfach, vor dem Eindampfen eventuell in den Lösungen vorhandene Sulfatmengen durch Fällung als Calciumsulfat und Abtrennung durch Filtration zu entfernen.

Die erfindinngsgemäßen Verbindungen der allgemeinen Formel (1) - im nachfolgenden als Verbindungen (1) bezeichnet - haben faserreaktive Eigenschaften und besitzen sehr wertvolle Farbstoffeigenschaften. Sie können deshalb zum Färben (einschließlich Bedrucken) von hydroxygruppenhaltigen und/oder carbonamidgruppenhaltigen Materialien verwendet werden. Hierzu können die bei der Synthese der Verbindungen (1) anfallenden Lösungen, gegebenenfalls nach Zusatz einer Puffersubstanz und gegebenenfalls auch nach Konzentrierung, direkt als Flüssigpräparation der färberischen Verwendung zugeführt werden.

Gegenstand der vorliegenden Erfindung ist deshalb auch die Verwendung der Verbindungen (1) zum Färben (einschließlich Bedrucken) von hydroxy- und/oder carbonamidgruppenhaltigen Materialien bzw. Verfahren zu deren Anwendung auf diesen Substraten. Bevorzugt kommen die Materialien in Form von Fasermaterialien zur Anwendung, insbesondere in Form von Textilfasern, wie Garnen, Wickelkörpern und Geweben. Hierbei kann man analog bekannten Verfahrensweisen vorgehen.

Hydroxygruppenhaltige Materialien sind solche natürlichen oder synthetischen Ursprungs, wie beispielsweise Cellulosefasermaterialien oder deren Regeneratprodukte und Polyvinylalkohole. Cellulosefasermaterialien sind vorzugsweise Baumwolle, aber auch andere Pflanzenfasern, wie Leinen, Hanf, Jute und Ramiefasern; regenerierte Cellulosefasern sind beispielsweise Zellwolle und Viskosekunstseide.

Carbonamidgruppenhaltige Materialien sind beispielsweise synthetische und natürliche Polyamide und Polyurethane, insbesondere in Form von Fasern, beispielsweise Wolle und andere Tierhaare, Seide, Leder, Polyamid-6,6, Polyamid-6, Polyamid-11 und Polyamid-4.

Die Verbindungen (1) lassen sich, gemäß der erfindungsgemäßen Anwendung, auf den genannten Substraten, insbesondere auf den genannten Fasermaterialien, nach den für wasserlösliche, faserreaktive Farbstoffe bekannten Anwendungstechniken applizieren und fixieren, so beispielsweise, indem man die Verbindung (1) in gelöster Form auf das Substrat aufbringt oder sie darin einbringt und sie auf diesem oder in diesem durch Hitzeeinwirkung oder durch Einwirkung eines alkalisch wirkenden Mittels oder durch beides fixiert. Solche Färbe- und Fixierweisen sind in der Literatur zahlreich beschrieben (bspw. in der europäischen Patentanmeldungs-Veröffentlichung Nr. 0 181 585 A2).

Insbesondere heben sich die Verbindungen (1) dadurch vorteilhaft hervor, daß sie in den üblichen Färbeverfahren auch bei Verwendung von Elektrolyten und alkalisch wirkenden Verbindungen in deutlich verringerter Menge als üblich in überraschender Weise Färbungen mit höheren Farbausbeuten als den üblichen Salzmengen liefern. Denn in der Regel werden beim heutigen Stand der Technik noch große Mengen an Elektrolytzusätzen in den Färbeflotten benötigt, um intensive Färbungen mit den faserreaktiven Farbstoffen zu erhalten. Aus ökologischen Gründen sind derartig hohe Salzmengen, die in der Regel oberhalb von 50 g pro Liter Färbeflotte liegen, unerwünscht. Die Verbindungen (1) ermöglichen es jedoch,

Färbeflotten zu verwenden, in welchen die Elektrolytzusätze unterhalb von 40 g pro Liter Färbeflotte, bevorzugt nur zwischen 10 und 35 g pro Liter Färbeflotte, liegen.

Die erfindungsgemäß erhältlichen Färbungen besitzen, insbesondere auf Cellulosefasermaterialien, gute Lichtechtheiten sowohl im trockenen Zustand der Färbung als auch im nassen, beispielsweise mit einer Schweißlösung befeuchteten, Zustand sowie gute Naßechtheiten, wie beispielsweise gute Waschechtheiten bei 60 bis 95°C, auch in Gegenwart von Perboraten, gute saure und alkalische Walk-, Überfärbe- und Schweißechtheiten, eine hohe Dampfbeständigkeit, gute Alkali-, Säure-, Wasser- und Seewasserechtheiten, des weiteren eine gute Plissierechtheit, Bügelechtheit und Reibechtheit. Ebenso besitzen sie eine gute Säurelagerbeständigkeit ("acid fading") beim Lagern von feuchten, noch Essigsäure enthaltendem, gefärbtem Material.

Die nachstehenden Beispiele dienen zur Erläuterung der Erfindung. Die Teile sind Gewichtsteile, die Prozentangaben stellen Gewichtsprozente dar, sofern nicht anders vermerkt. Gewichtsteile beziehen sich zu Volumenteilen wie Kilogramm zu Liter.

Die in diesen Beispielen formelmäßig beschriebenen Verbindungen sind in Form der freien Säuren angegeben; im allgemeinen werden sie in Form ihrer Alkalimetallsalze, wie Lithium-, Natrium- oder Kaliumsalze, hergestellt und isoliert und in Form ihrer Salze zum Färben verwendet. Ebenso können die in den nachfolgenden Beispielen, insbesondere Tabellenbeispielen, in Form der freien Säure genannten Ausgangsverbindungen und Komponenten als solche oder in Form ihrer Salze, vorzugsweise Alkalimetallsalze, in die Synthese eingesetzt werden.

Die für die erfindungsgemäßen Verbindungen angegebenen Absorptionsmaxima ($\lambda_{max}$) im sichtbaren Bereich wurden anhand derer Alkalimetallsalze in wäßriger Lösung ermittelt. In den Tabellenbeispielen sind die $\lambda_{max}$-Werte bei der Farbtonangabe in Klammern gesetzt; die Wellenlängenangabe bezieht sich auf nm.

Die Messung der $^1$H-NMR-spektroskopischen Daten erfolgte in Dimethylsulfoxid-$d_6$ als Lösemittel und mit Tetramethylsilan als innerem Standard, sofern nicht anders vermerkt.

Beispiel A

Zur Synthese von 1,3-Dichlor-isopropyl-(4'-nitro-phenyl)-sulfid leitet man unter Kühlung bei -10°C in eine Suspension von 100 Teilen Bis-(4-nitro-phenyl)-disulfid in 1000 Vol.-Teilen Dichlormethan so lange Chlor ein, bis alles Disulfid in Lösung gegangen ist. Sodann kühlt man den Ansatz auf -40°C ab und gibt während etwa 30 Minuten langsam 80 Teile Allylchlorid hinzu. Man läßt den Ansatz danach sich auf 20°C erwärmen, rührt noch etwa 16 Stunden bei dieser Temperatur nach und entfernt sodann flüchtige Bestandteile unter reduziertem Druck.
Der erhaltene Rückstand wird aus Diethylether umkristallisiert. Man erhält etwa 122 bis 123 Teile der Verbindung der Formel

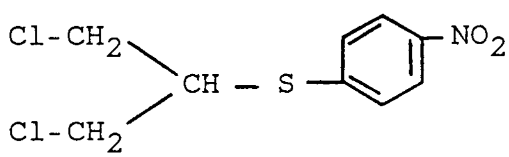

in Form blaßgelber Kristalle mit einem Festpunkt von 62°C.

| C,H,N-Analyse: | | | |
|---|---|---|---|
| ber.: | C 40,6 %, | H 3,4 %, | N 5,2 % ; |
| gef.: | C 40,4 %, | H 3,4 %, | N 5,3 % . |

$^1$H-NMR-Analyse: 4,0 ppm (4H;d), 4,22 ppm (1H;quint.), 7,65 ppm (2H;d), 8,18 ppm (2H;d).

Beispiel B

Zur Herstellung von 1,3-Dichlor-isopropyl-(4'-nitro-phenyl)-sulfon löst man 13,3 Teile 1,3-Dichlor-isopropyl-(4'-nitro-phenyl)-sulfid (Beispiel A; in Form des Rohproduktes oder nach Umkristallisation) in 100 Vol.-Teilen Eisessig, gibt 0,05 Teile Natriumwolframat hinzu, erhitzt den Ansatz auf 80°C und gibt bei dieser Temperatur 11 Vol.-Teile 35 %iges wäßriges Wasserstoffperoxid langsam unter Rühren hinzu. Man rührt

danach noch etwa 15 Minuten nach und kühlt den Ansatz auf 0 °C ab. Die ausgefallenen Kristalle werden abgesaugt und aus Ethanol umkristallisiert. Man erhält etwa 11,5 Teile der Verbindung der Formel

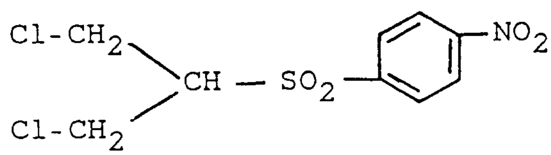

mit einem Festpunkt von 105 °C.

| C,H,N-Analyse: | | | |
|---|---|---|---|
| ber.:<br>gef.: | C 36,25 %,<br>C 36,3 %, | H 3,04 %,<br>H 3,0 %, | N 4,7 % ;<br>N 4,8 % . |

$^1$H-NMR-Analyse (in $CDCl_3$): 4,07 ppm (4H;m), 4,5 ppm (1H;m), 8,2 ppm (4H;d), 8,45 ppm (4H;d).

Beispiel C

Zur Herstellung von 1,3-Dichlor-isopropyl-(4'-amino-phenyl)-sulfon bereitet man zunächst eine Mischung aus 80 Teilen Zinkpulver, 360 Teilen Wasser, 160 Vol.-Teilen Dioxan und 10 Vol.-Teilen einer konzentrierten wäßrigen Salzsäure. In diese Mischung gibt man unter gutem Rühren bei etwa 20 °C gleichzeitig eine Lösung von 48 Teilen 1,3-Dichlor-isopropyl-(4'-nitro-phenyl)-sulfon (Beispiel B) in 320 Vol.-Teilen Dioxan und eine Mischung aus 310 Vol.-Teilen konzentrierter wäßriger Salzsäure, 160 Teilen Wasser und 80 Vol.-Teilen Dioxan langsam hinzu. Man rührt noch eine Zeit weiter, bis das Zink weitgehend aufgebraucht und in Lösung gegangen ist, filtriert den Ansatz vom Niederschlag ab, wäscht diesen mit Dioxan aus und stellt die vereinigten Filtrate mittels Natriumacetat auf einen pH-Wert von 4. Man rührt das Filtrat noch etwa 12 Stunden bei etwa 20 °C nach und saugt den ausgefallenen weißen Niederschlag ab und trocknet ihn. Man erhält etwa 36 Teile einer feinkristallinen Verbindung der Formel

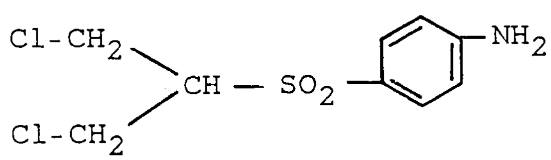

mit einem Festpunkt von 104 °C.

| Elementaranalyse: | | | | |
|---|---|---|---|---|
| ber.:<br>gef.: | C 40,3 %,<br>C 40,8 %, | H 4,1 %,<br>H 4,2 %, | N 5,2 %,<br>N 5,3 %, | Cl 26,4 % ;<br>Cl 25,9 % . |

$^1$H-NMR-Analyse: 3,95 ppm (5H;m), 6,25 ppm (2H;br), 6,65 ppm (2H;d), 7,5 ppm (2H;d).

Beispiel D

Zur Herstellung von 1-Chlor-3-acetyloxy-isopropyl-(4'-nitro-phenyl)-sulfid leitet man in eine Suspension von 92,4 Teilen Bis-(4-nitro-phenyl)-disulfid in 500 Vol.-Teilen Dichlormethan bei -10 °C Chlor ein, bis eine klare Lösung entstanden ist. Anschließend leitet man etwa 15 Minuten trockenen Stickstoff ein und gibt sodann bei einer Temperatur von -40 °C innerhalb von etwa 15 Minuten langsam 108 Vol.-Teile Allylacetat hinzu. Man rührt noch eine Stunde nach, kühlt sodann die Lösung auf 70 °C ab und isoliert den blaßgelben Niederschlag.
Es werden 152 Teile eines feinkristallinen Pulvers der Verbindung der Formel

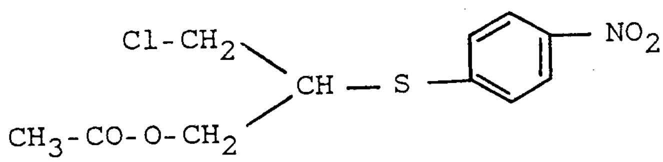

mit einem Festpunkt von 110 °C.

| Elementaranalyse: | | | | |
|---|---|---|---|---|
| ber.: | C 45,6 %, | H 4,17 %, | N 4,8 %, | Cl 12,2 % ; |
| gef.: | C 45,6 %, | H 4,2 %, | N 4,8 %, | Cl 12,4 % . |

$^1$H-NMR-Analyse: 2,05 ppm (3H;s), 3,97 ppm (2H;d), 4,18 ppm (1H;quint.), 4,35 ppm (2H;m), 7,67 ppm (2H;d), 8,18 ppm (2H;d).

Beispiel E

Zur Herstellung von 1-Chlor-3-acetyloxy-isopropyl-(4'-nitro-phenyl)-sulfon versetzt man eine 70 °C heiße Lösung von 145 Teilen 1-Chlor-3-acetyloxy-isopropyl-(4'-nitro-phenyl)-sulfid (Beispiel D) in 450 Vol.-Teilen Eisessig, nach Zugabe von 2 Teilen Natriumwolframat, langsam innerhalb von etwa 30 Minuten mit 120 Vol.-Teilen 35 %igem wäßrigem Wasserstoffperoxid. Man rührt noch etwa 15 Minuten nach, kühlt den Ansatz auf etwa 20 °C, gibt 100 Teile Wasser hinzu, kühlt die Lösung auf 0 °C ab und isoliert den weißen Niederschlag. Man erhält 145 Teile eines feinkristallinen Pulvers der Verbindung 1-Chlor-3-acetyloxy-isopropyl-(4'-nitro-phenyl)-sulfon mit einem Festpunkt von 89 °C.

| Elementaranalyse: | | | | |
|---|---|---|---|---|
| ber.: | C 41,1 %, | H 3,7 %, | N 4,3 %, | Cl 11,0 % ; |
| gef.: | C 41,4 %, | H 3,6 %, | N 4,1 %, | Cl 11,1 % . |

$^1$H-NMR-Analyse: 1,83 ppm (3H;s), 3,9-4,17 ppm (2H;m), 4,36 ppm (1H;quint.), 4,48 ppm (2H;m), 8,22 ppm (2H;d), 8,48 ppm (2H;d) .

Beispiel F

Zur Herstellung von 1-Chlor-3-cyano-isopropyl-(4'-nitro-phenyl)-sulfid gibt man zu einer Lösung von 7,6 Teilen 4-Nitrophenyl-sulfenylchlorid langsam bei -20 °C 4,0 Teile Allylcyanid. Man rührt noch 1 Stunde nach und entfernt sodann flüchtige Bestandteile unter reduziertem Druck. Der zurückbleibende braune Rückstand wird nach einiger Zeit fest. Nach Umkristallisation aus einem Gemisch aus gleichen Teilen Diäthylether und Dichlormethan erhält man 6,7 Teile der Verbindung der Formel

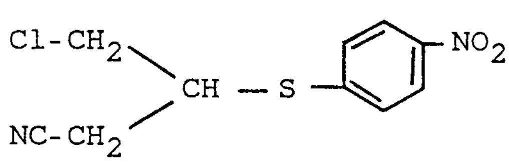

in Form gelblicher Kristalle mit einem Festpunkt von 68 °C.

| Elementaranalyse: | | | | |
|---|---|---|---|---|
| ber.: | C 46,7 %, | H 3,5 %, | N 10,9 %, | Cl 13,8 % ; |
| gef.: | C 45,9 %, | H 3,5 %, | N 10,9 %, | Cl 14,6 % . |

$^1$H-NMR-Analyse: 3,1 ppm (2H;m), 3,9 ppm (2H;m), 4,27 ppm (1H;m), 7,75 ppm (2H;d), 8,2 ppm (2H;d).

Beispiel G

Zur Herstellung von 1-Chlor-3-cyano-isopropyl-(4'-nitro-phenyl)-sulfon versetzt man eine 60 °C warme Lösung von 3,84 Teilen 1-Chlor-3-cyano-isopropyl-(4'-nitro-phenyl)-sulfid (Beispiel F) in 30 Vol.-Teilen Eisessig, nach Zugabe von 0,005 Teilen Natriumwolframat, langsam innerhalb von 30 Minuten mit 7 Vol.-Teilen einer 35 %igen wäßrigen Wasserstoffperoxidlösung. Man rührt noch kurze Zeit nach, kühlt den Ansatz auf -15 °C ab und isoliert nach einiger Zeit das ausgefallene Produkt. Es werden 2,6 Teile der Verbindung der Formel

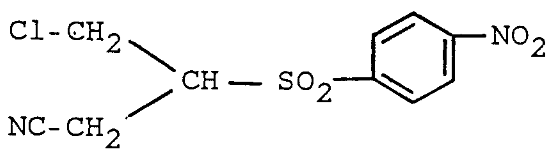

in Form eines weißen Pulvers mit einem Festpunkt von 101 °C erhalten.

| Elementaranalyse: | | | | |
|---|---|---|---|---|
| ber.: | C 41,6 %, | H 3,1 %, | N 9,7 %, | Cl 12,2 % ; |
| gef.: | C 41,4 %, | H 3,0 %, | N 9,7 %, | Cl 12,4 % . |

$^1$H-NMR-Analyse: 3,2 ppm (2H;m), 4,0 ppm (2H;m), 4,5 ppm (1H;quint.), 8,25 ppm (2H;d), 8,5 ppm (2H;d).

Beispiel H

Zur Herstellung von 1,3-Dichlor-isopropyl-(3'-nitro-phenyl)-sulfid leitet man bei -10 °C in eine Lösung aus 55,45 Teilen Bis-(3-nitro-phenyl)-disulfid in 300 Vol.-Teilen Methylenchlorid Chlor ein und rührt den Ansatz bei dieser Temperatur noch einige Zeit nach. Anschließend leitet man während etwa 15 Minuten trockenen Stickstoff durch die Reaktionslösung und gibt anschließend bei -40 °C langsam 37 Vol.-Teile Allylchlorid hinzu. Man rührt noch kurze Zeit nach und entfernt sodann flüchtige Bestandteile unter reduziertem Druck. Man erhält ein braunes Öl, das ohne weitere Reinigung zur Herstellung der entsprechenden Sulfonylverbindung (s. Beispiel J) eingesetzt werden kann.

Eine kleine Menge des erhaltenen rohen Sulfids wurde chromatographisch gereinigt. Es zeigte folgende $^1$H-NMR-Analysen-Werte: 4,1 ppm (4H;m), 4,5 ppm (1H;quint.), 7,98 ppm (1H;t), 8,38 ppm (1H;d), 8,61 ppm (1H;d), 8,6 ppm (1H;d).

Beispiel J

Zur Herstellung von 1,3-Dichlor-isopropyl-(3'-nitro-phenyl)-sulfon gibt man langsam zu einer 70 °C heißen Lösung der Sulfidverbindung von Beispiel H, nach Zugabe von 0,02 Teilen Natriumwolframat, 80 Vol.-Teile einer 35 %igen wäßrigen Wasserstoffperoxidlösung. Man rührt den Ansatz noch etwa 90 Minuten bei 70 °C nach und setzt anschließend weitere 20 Vol.-Teile der Wasserstoffperoxidlösung hinzu. Nach weiterem kurzen Nachrühren versetzt man den Ansatz mit 100 Teilen Wasser, kühlt ihn auf 0 °C ab und isoliert nach einiger Zeit den feinkristallinen Niederschlag.

Es werden 79,5 Teile der Verbindung der Formel

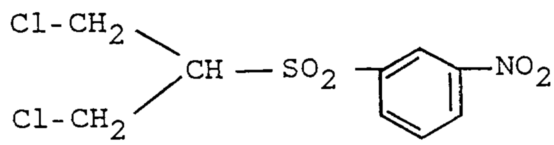

mit einem Festpunkt von 110 °C erhalten.

| Elementaranalyse: | | | | | | |
|---|---|---|---|---|---|---|
| ber.: | C 36,2 %, | H 3,04 %, | N 4,7 %, | Cl 23,8 %, | S 10,7 %, | O 21,4 % ; |
| gef.: | C 36,6 %, | H 3,1 %, | N 4,7 %, | Cl 23,5 %, | S 10,6 %, | O 21,9 % . |

$^1$H-NMR-Analyse: 4,05 ppm (4H;m), 4,5 ppm (1H;t), 7,98 ppm (1H;m), 8,38 ppm (1H;m), 8,61 ppm (1H;m).

Beispiel K

Zur Herstellung von 1-Chlor-3-acetyloxy-isopropyl-(4'-amino-phenyl)-sulfon gibt langsam unter gutem Rühren zu einer Mischung aus 47 Teilen Wasser, 20 Vol.-Teilen Dioxan und 15 Teilen Zinkpulver bei 15 bis 20 °C gleichzeitig eine Lösung von 6 Teilen 1-Chlor-3-acetyloxy-isopropyl-(4'-nitro-phenyl)-sulfon (Beispiel C) in 47 Vol.-Teilen Dioxan und eine Mischung aus 47 Vol.-Teilen konzentrierter wäßriger Salzsäure, 20 Teilen Wasser und 14 Vol.-Teilen Dioxan hinzu. Man rührt noch etwa 15 Minuten bei 15 bis 20 °C nach, filtriert den Niederschlag ab, stellt das Filtrat mit Natriumacetat auf einen pH-Wert von 4 ein und isoliert nach einiger Zeit das ausgefallene Produkt. Es werden 5 Teile der Verbindung 1-Chlor-3-acetyloxy-isopropyl-(4'-amino-phenyl)-sulfon als feines weißes Kristallpulver mit einem Festpunkt von 124 °C erhalten.

| Elementaranalyse: | | | | |
|---|---|---|---|---|
| ber.: | C 45,3 %, | H 4,4 %, | N 4,8 %, | Cl 12,15 % ; |
| gef.: | C 45,0 %, | H 4,75 %, | N 4,8 %, | Cl 12,4 % . |

$^1$H-NMR-Analyse: 1,90 ppm (3H;s), 3,82 ppm (2H;m), 3,98 ppm (1H;m), 4,38 ppm (2H;m), 6,2 ppm (2H;br), 6,65 ppm (2H;d), 7,45 ppm (2H;d).

Beispiel L

Zur Herstellung von 1-Brom-3-chlor-isopropyl-(4'-nitro-phenyl)-sulfidleitet man bei -10 °C in eine Lösung von 31 Teilen Bis-(4-nitro-phenyl)-disulfid in 150 Vol.-Teilen Dichlormethan Chlor ein. Man rührt den Ansatz noch einige Zeit nach und leitet sodann während etwa 15 Minuten trockenen Stickstoff durch und gibt anschließend langsam bei -40 °C 40 Teile Allylbromid hinzu. Man rührt noch 1 Stunde nach, erwärmt die Lösung auf etwa 20 °C und entfernt flüchtige Bestandteile unter reduziertem Druck. Man erhält 51 Teile eines braunen kristallinen, Rohproduktes, das ohne weitere Reinigung zur Synthese der entsprechenden Sulfonylverbindung (s. Beispiel M) eingesetzt werden kann.

Eine kleine Menge dieses Rohproduktes wurde aus einem Gemisch von gleichen Teilen Diethylether und Dichlormethan umkristallisiert. Es besitzt einen Festpunkt von 60 °C; folgende Analysenwerte wurden erhalten:

| Elementaranalyse: | | | |
|---|---|---|---|
| ber.: | C 34,8 %, | H 2,9 %, | N 4,5 % ; |
| gef.: | C 34,9 %, | H 2,9 %, | N 4,6 % . |

$^1$H-NMR-Analyse: 3,9 ppm (2H;m), 4,02 ppm (2H;m), 4,27 ppm (1H;quint.), 7,77 ppm (2H;d), 8,19 ppm (2H;d).

Beispiel M

Zur Herstellung von 1-Brom-3-chlor-isopropyl-(4'-nitro-phenyl)-sulfonversetzt man eine 70 °C warme Lösung von 75 Teilen 1-Brom-3-chlor-isopropyl-(4'-nitro-phenyl)-sulfid in 100 Vol.-Teilen Eisessig, nach Zugabe von 0,5 Teilen Natriumwolframat, langsam mit 86 Vol.-Teilen einer 35 %igen wäßrigen Wasserstoffperoxidlösung. Man rührt noch kurze Zeit nach kühlt den Reaktionsansatz auf 0 °C ab und isoliert nach etwa 2 Stunden das ausgefallene Produkt.

Es werden 66 Teile der Verbindung der Formel

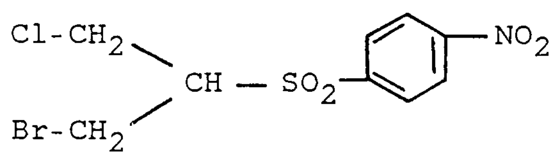

in Form leicht gelblicher Kristalle mit einem Festpunkt von 108 °C erhalten.

| C,H,N-Analyse: | | | |
|---|---|---|---|
| ber.: | C 31,5 %, | H 2,6 %, | N 4,1 % ; |
| gef.: | C 30,7 %, | H 2,6 %, | N 3,9 % . |

$^1$H-NMR-Analyse: 3,7 ppm (2H;m), 3,93 ppm (2H;m), 4,12 ppm (1H;d), 8,15 ppm (2H;d), 8,43 ppm (2H;d).

Beispiel N

Zur Herstellung von 1,3-Dichlor-isopropyl-(4'-aminophenyl)-sulfon hydriert man 29,8 Teile 1,3-Dichlor-isopropyl-(4'-nitrophenyl)-sulfon, gelöst in 300 Vol.-Teilen Eisessig, unter Zusatz von 1,5 Teilen eines Palladium/Aktivkohle-Katalysators (10 % Palladium auf Aktivkohle) bei einem Wasserstoffdruck von 10 bar. Nach beendeter Wasserstoffaufnahme wird der größte Teile der Essigsäure unter vermindertem Druck abdestilliert. Der Rückstand wird mit der 10-fachen Menge Wasser versetzt, abgesaugt und getrocknet. Man erhält die Verbindung der im Beispiel C angegebenen Formel als weißes Kristallpulver (24 Teile).

Beispiel P

Zur Herstellung von 1,3-Dichlor-isopropyl-(3'-aminophenyl)-sulfon hydriert man 25 Teile 1,3-Dichlor-isopropyl-(3'-nitrophenyl)-sulfon (Beispiel J), in 300 Vol.-Teilen Eisessig suspendiert, gemäß den Angaben des Beispiels N. Nach beendeter Wasserstoffaufnahme wird der größte Teil des Eisessigs abdestilliert und der erhaltene Rückstand in die 10-fache Menge Wasser eingerührt. Das reduzierte Produkt fällt zunächst harzig aus, kristallisiert jedoch nach einiger Zeit. Es werden etwa 20,8 Teile eines weißen Pulvers der Verbindung der Formel

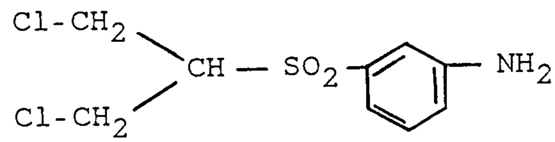

mit eines Festpunkt von 69 °C erhalten.

| C,H,N-Analyse: | | | | |
|---|---|---|---|---|
| ber.:<br>gef.: | C 40,3 %,<br>C 40,5 %, | H 4,1 %,<br>H 4,1 %, | N 5,2 %,<br>N 5,2 %, | Cl 26,4 % ;<br>Cl 26,3 % . |

$^1$H-NMR-Analyse: 4,0 ppm (4H;m), 4,07 ppm (1H;m), 5,65 ppm (2H;br), 6,91 ppm (1H;d), 7,00 ppm (1H;d), 7,09 ppm (1H;t), 7,29 ppm (1H;t).

Beispiel Q

76 Teile 4-(1-Chlor-3-acetyloxy-isopropylsulfonyl)-anilin suspendiert man in 200 Vol.-Teilen einer konzentrierten wäßrigen Salzsäure, erhitzt den Ansatz auf 65 bis 70 °C, hält ihn etwa zwei Stunden bei dieser Temperatur, kühlt ihn sodann ab, stellt einen pH-Wert zwischen 3 und 3,5 mittels wäßriger Natronlauge ein und saugt das ausgefallene Produkt ab. Man erhält 22 Teile eines feinen weißen Pulvers der Verbindung 4-(1-Chlor-3-hydroxy-isopropylsulfonyl)-anilin mit einem Festpunkt von 88 °C.

| Elementaranalyse: | | | |
|---|---|---|---|
| ber.:<br>gef.: | C 43,3 %,<br>C 42,3 %, | H 2,9 %,<br>H 4,5 %, | N 5,6 % ;<br>N 5,8 % . |

$^1$H-NMR-Analyse (in $CDCl_3$): 2,7 ppm (1H;t,br), 3,3 ppm (1H;m), 3,8 ppm (2H;n) 4,0-4,2 ppm (2H;m), 4,33 ppm (2H;s,br), 6,75 ppm (1H;d), 7,63 ppm (1H;d).

Beispiel R

16,5 Teile 4-(1-Chlor-3-hydroxy-isopropylsulfonyl)-anilin werden bei 10 bis 20 °C in 50 Vol.-Teile konzentrierter Schwefelsäure eingetragen. Man rührt noch 2 Stunden weiter und gießt die Mischung sodann in 300 Teile Eiswasser. Das ausgefallene Produkt wird abgesaugt und getrocknet.

Es werden 17,5 Teile eines weißen Pulvers der Verbindung 4-(1-Sulfato-3-chlor-isopropylsulfonyl)-anilin erhalten.

| Elementaranalyse: | | | |
|---|---|---|---|
| ber.:<br>gef.: | C 32,8 %,<br>C 32,5 %, | H 3,66 %,<br>H 3,6 %, | N 4,24 % ;<br>N 4,3 % . |

$^1$H-NMR-Analyse: 3,75 ppm (1H;m), 3,84 ppm (2H;d) 4,0 ppm (1H;m), 4,1 ppm (1H;m), 6,76 ppm (1H;d), 7,52 ppm (1H;d).

Beispiel S

In eine Lösung von 220 Teilen Thiophenol in 807 Teilen trockenem Chlorbenzol werden bei 0 °C unter Stickstoffatmosphäre während etwa drei Stunden 71 Teile Chlor eingeleitet. Danach wird der Ansatz noch 30 Minuten nachgerührt. Anschließend wird überschüssiges Chlor durch Hindurchleiten von trockenem Stickstoff ausgeblasen. Der Ansatz wird auf -40 °C abgekühlt. Innerhalb von etwa 30 Minuten gibt man 168 Teile Allylchlorid langsam und stetig hinzu. Man rührt noch 45 Minuten nach, erwärmt den Ansatz auf 20 °C und destilliert das Chlorbenzol unter reduziertem Druck ab.

443 Teile des erhaltenen hellgelben Öles werden in 734 Teilen Essigsäure gelöst. Man gibt 2 Teile Natriumwolframat-dihydrat hinzu, erhitzt den Ansatz auf 75 °C und läßt langsam und stetig unter Einhaltung einer Temperatur von 80 °C innerhalb von etwa einer Stunde 450 Teile einer 35 %igen wäßrigen Wasserstoffperoxidlösung zulaufen. Man rührt den Ansatz danach noch etwa 30 Minuten nach, kühlt ihn auf 0 °C ab, saugt den weißen Niederschlag ab, wäscht ihn dreimal mit etwa 70 Teilen Wasser und trocknet ihn unter reduziertem Druck.

506 Teile der erhaltenen Verbindung 1,3-Dichlorisopropyl-phenyl-sulfon werden in 950 Teilen 100 %iger Schwefelsäure gelöst; unter Rühren gibt man bei 40 bis 50 °C 610 Teile Nitriersäure, bestehend aus 70 % Schwefelsäure und 30 % Salzpetersäure, innerhalb von etwa einer Stunde langsam hinzu. Man rührt den Ansatz, der teilweise kristallisiert ist, noch etwa 30 Minuten nach und gibt ihn sodann in 4000 Teile Wasser. Das ausgefallene Produkt wird abgesaugt, mit 3000 Teilen Wasser gewaschen, fein gemahlen, nochmals mit 3000 Teilen Wasser gewaschen und getrocknet.

Die erhaltene Verbindung 1,3-Dichlor-isopropyl-(3'-nitrophenyl)-sulfon ist mit der des Beispieles J identisch.

Beispiel 1

Eine Suspension von 9 Teilen 4-(1,3-Dichlorisopropylsulfonyl)-anilin in einem Gemisch aus 40 Vol.-Teilen Aceton, 80 Teilen Wasser und 16 Vol.-Teilen konzentrierter wäßriger Salzsäure werden in üblicher Weise durch Zugabe von Natriumnitrit diazotiert. Anschließend gibt man eine neutrale Lösung des Natriumsalzes von 7,9 Teilen 1-(4'-Sulfo-phenyl)-3-carboxy-pyrazol-5-on in 55 Teilen Wasser hinzu und stellt mit Natriumacetat einen pH-Wert von 4 ein. Nach beendeter Kupplungsreaktion, die in üblicher Weise bei 15 bis 20 °C durchgeführt wird, wird die erhaltene erfindungsgemäße Azoverbindung durch Aussalzen mittels Natriumchlorid und Filtration isoliert.

Sie besitzt, in Form der freien Säure geschrieben die Formel

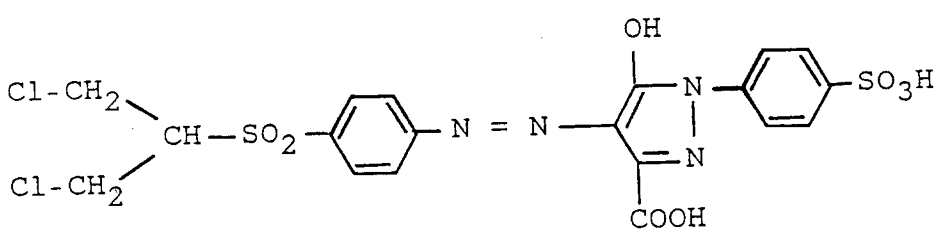

($\lambda_{max}$ = 412 nm)

und zeigt sehr gute faserreaktive Farbstoffeigenschaften. Bei Anwendung der für faserreaktive Farbstoffe bekannten Applikations- und Fixierverfahren erhält man mit ihr auf den in der Beschreibung genannten Materialien, wie insbesondere Cellulosefasermaterialen, beispielsweise Baumwolle, farbstarke, gelbe Färbungen und Drucke mit guten Echtheitseigenschaften, von denen insbesondere die Licht-, Naßlicht- und Schweißlichtechtheiten hervorgehoben werden können.

Beispiel 2

Man diazotiert 9 Teile 4-(1,3-Dichlorisopropylsulfony)1-anilin gemäß den Angaben des Beispiels 1. Zur erhaltenen Diazoniumsalzlösung gibt man eine neutrale Lösung des Natriumsalzes von 13,5 Teilen 2-Acetylamino-6-sulfo-8-naphthol in 30 Teilen Wasser hinzu, stellt den pH-Wert mit Natriumacetat auf 4,5 und führt die Kupplungsreaktion bei 15 bis 20 °C zu Ende.

Die ausgefallene erfindungsgemäße Azoverbindung der Formel

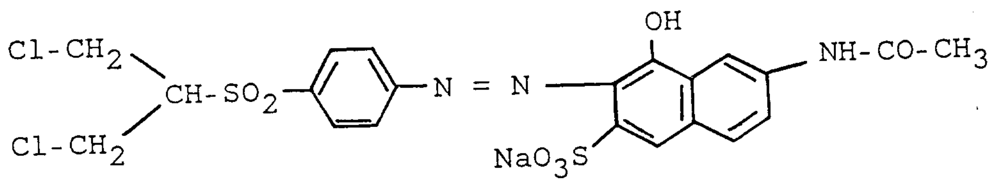

($\lambda_{max}$ = 490 nm)

wird abfiltriert und getrocknet. Sie besitzt sehr gute faserreaktive Farbstoffeigenschaften und liefert auf den in der Beschreibung genannten Materialien, wie insbesondere Cellulosefasermaterialien, beispielsweise Baumwolle, nach den für faserreaktive Farbstoffe üblichen Applikations- und Fixierverfahren orange Färbungen und Drucke mit guten Echtheitseigenschaften, von denen insbesondere die Licht-, Naßlicht- und Schweißlichtechtheiten hervorgehoben werden können.

Beispiel 3

10,7 Teile 4-(1',3'-Dichlorisopropylsulfonyl)-anilin werden gemäß den Angaben des Beispieles 1 diazotiert. Zur erhaltenen Diazoniumsalzlösung gibt man eine Suspension von 8 Teilen 2-Sulfo-5-acetylaminoanilin in 50 Teilen Wasser, stellt mit einer 10 %igen wäßrigen Natriumcarbonatlösung den pH auf einen Wert von 3 und führt die Kupplungsreaktion zunächst bei 20 °C während 30 Minuten und sodann bei 30 °C während 2 Stunden, jeweils unter Einhaltung des pH-Wertes 3 mittels Natriumacetat, zu Ende.

Die ausgefallene erfindungsgemäße Azoverbindung der Formel

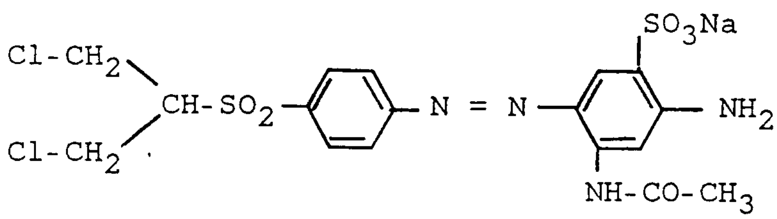

($\lambda_{max}$ = 490 nm)

wird abfiltriert und getrocknet. Sie besitzt sehr gute faserreaktive Farbstoffeigenschaften und liefert auf den in der Beschreibung genannten Materialien, wie insbesondere Cellulosefasermaterialien, beispielsweise Baumwolle, nach den für faserreaktive Farbstoffe üblichen Applikations- und Fixierverfahren goldgelbe Färbungen und Drucke mit guten Echtheitseigenschaften, von denen insbesondere die Licht- und Schweißlichtechtheiten hervorgehoben werden können.

Beispiel 4

8,1 Teile 4-(1'-Chlor-3'-acetyloxy-isopropylsulfonyl)-anilin werden in einem Gemisch aus 30 Vol.-Teilen Aceton, 70 Teilen Wasser und 16 Vol.-Teilen konzentrierter wäßriger Salzsäure suspendiert und gemäß den Angaben des Beispieles 1 diazotiert. Zu der erhaltenen Diazoniumsalzlösung gibt man eine neutrale Lösung von 9,4 Teilen 1-(4'-Sulfo-phenyl)-3-carboxy-pyrazol-5-on in 50 Teilen Wasser und führt die Kupplungsreaktion bei einem pH-Wert von 4,5 und einer Temperatur zwischen 10 und 25 °C durch.

Die erfindungsgemäße Azoverbindung der Formel (in Form der freien Säure geschrieben)

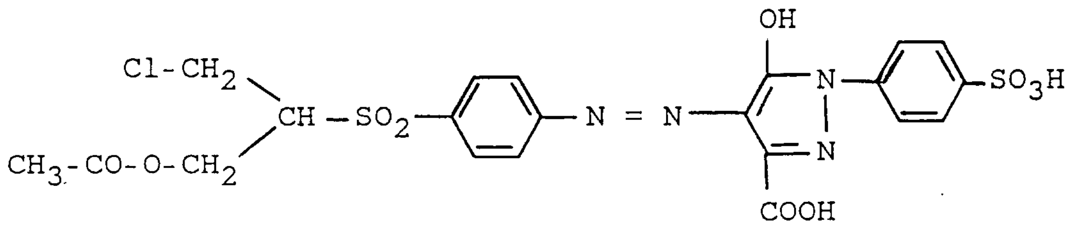

$(\lambda_{max} = 412\ nm)$

wird mit 6 Teilen Natriumchlorid als Natriumsalz ausgesalzen und isoliert. Sie besitzt sehr gute faserreaktive Farbstoffeigenschaften und liefert auf den in der Beschreibung genannten Materialien, wie insbesondere Cellulosefasermaterialien, beispielsweise Baumwolle, nach den für faserreaktive Farbstoffe üblichen Applikations- und Fixierverfahren gelbe Färbungen und Drucke mit guten Echtheitseigenschaften, von denen insbesondere die Licht-, Naßlicht- und Schweißlichtechtheiten hervorgehoben werden können.

Beispiel 5

Eine Suspension von 9 Teilen 3-(1,3-Dichlor-isopropyl-sulfonyl)-anilin in einem Gemisch aus 40 Vol.-Teilen Aceton, 80 Teilen Wasser und 16 Vol.-Teilen konzentrierter wäßriger Salzsäure werden in üblicher Weise durch Zugabe von Natriumnitrit diazotiert. Anschließend gibt man zur erhaltenen Diazoniumsalzlösung eine neutrale Lösung des Natriumsalzes von 13,5 Teilen 2-Acetylamino-6-sulfo-8-naphthol in 30 Teilen Wasser hinzu, stellt den pH-Wert mit Natriumacetat auf 4,5 ein und führt die Kupplungsreaktion bei 15 bis 20 °C zu Ende.

Die ausgefallene erfindungsgemäße Azoverbindung der Formel

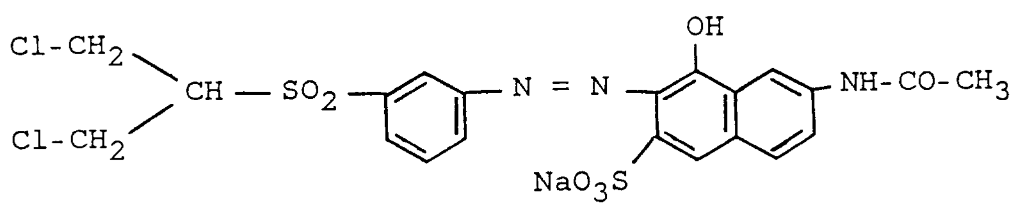

$(\lambda_{max} = 488\ nm)$

wird abfiltriert und getrocknet. Sie besitzt sehr gute faserreaktive Farbstoffeigenschaften und liefert auf den in der Beschreibung genannten Materialien, wie insbesondere Cellulosefasermaterialien, nach den für faserreaktive Farbstoffe üblichen Applikations- und Fixierverfahren farbstarke orange Färbungen und Drucke mit guten Echtheitseigenschaften, von denen insbesondere die Licht-, Naßlicht- und Schweißlichtechtheiten hervorgehoben werden können.

Beispiel 6

Eine analog den Angaben des Beispieles 1 hergestellte Diazoniumsalzlösung von 10,7 Teilen 4-(1,3-Dichlor-isopropylsulfonyl)-anilin gibt man zu 40 Vol.-Teilen einer wäßrigen Lösung von 16,8 Teilen N,N-Bis-($\beta$-sulfatoethyl)-3-chlor-anilin. Man stellt den Kupplungsansatz mit Salzsäure auf einen pH-Wert von 2 ein und rührt ihn noch etwa 30 Minuten zur Beendigung der Kupplungsreaktion. Zur vollständigen Ausfällung des erfindungsgemäßen Azofarbstoffes gibt man noch 10 Teile Natriumchlorid hinzu, filtriert den Niederschlag ab und trocknet ihn.

Man erhält die erfindungsgemäße Azoverbindung der Formel (in Form der freien Säure geschrieben)

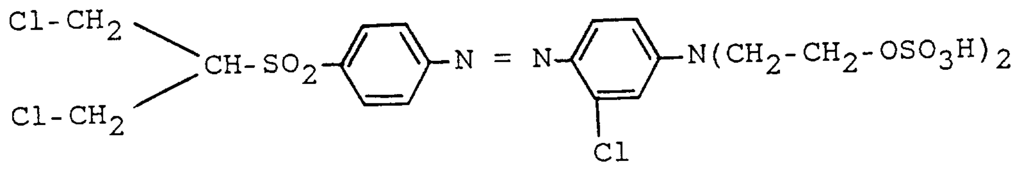

($\lambda_{max}$ = 462 nm)

als Natriumsalz in Form eines nur geringe Mengen Elektrolytsalz (Natriumchlorid) enthaltenden gelben Pulvers. Die erfindungsgemäße Azoverbindung besitzt sehr gute faserreaktive Farbstoffeigenschaften. Sie liefert auf den in der Beschreibung genannten Materialien, wie insbesondere Cellulosefasermaterialien, beispielsweise Baumwolle, nach den für faserreaktive Farbstoffe üblichen Färbe- und Druckverfahren farbstarke gelbe Färbungen und Drucke mit guten Echtheitseigenschaften, von denen insbesondere die Licht-, Naßlicht- und Schweißlichtechtheiten hervorgehoben werden können.

Beispiel 7

Man löst 4 Teile 4-(1,3-Dichlor-isopropylsulfonyl)-anilin in 8 Teilen konzentrierter Schwefelsäure und diazotiert durch langsame Zugabe eines Lösung der notwendigen Menge an Natriumnitrit in 40 Teilen Eiswasser. Die erhaltene Diazoniumsalzlösung wird mittels Kieselgur und Filtration geklärt; überschüssige salpetrige Säure wird in üblicher Weise mittels Amidosulfonsäure zerstört. Sodann gibt man eine Suspension von 8,4 Teilen der Monoazoverbindung 2-[4'-($\beta$-Sulfatoethylsulfonyl)-phenyl]-azo-1-amino-8-hydroxy-naphthalin-3,6-disulfonsäure als Natriumsalz in 100 Teilen Wasser hinzu, stellt den Ansatz auf einen pH-Wert von 4,5 und führt die Kupplungsreaktion bei etwa 20 °C und diesem pH-Wert zu Ende.

Die erhaltene erfindungsgemäße Disazoverbindung, die, in Form der freien Säure geschrieben, der Formel

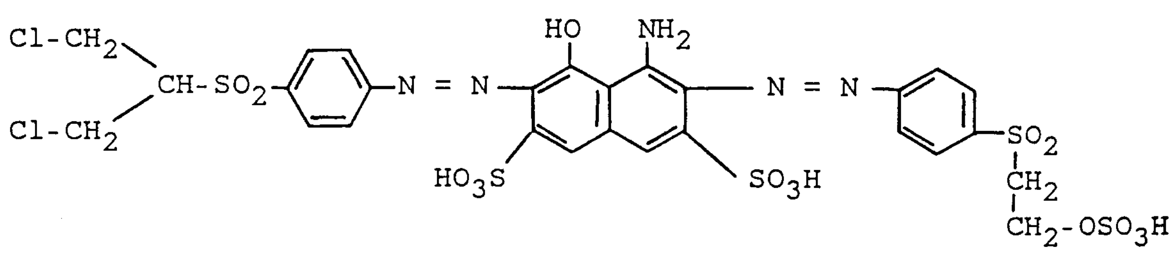

($\lambda_{max}$ = 594 nm)

entspricht, wird als Natriumsalz durch Aussalzen mit Natriumchlorid isoliert. Sie besitzt sehr gute faserreaktive Farbstoffeigenschaften und liefert beispielsweise auf Cellulosefasermaterialien nach den für faserreaktive Farbstoffe üblichen Applikations- und Fixierverfahren marineblaue Färbungen und Drucke mit guten Echtheitseigenschaften, von denen insbesondere die Licht-, Naßlicht- und Schweißlichtechtheiten hervorgehoben werden können.

Beispiel 8

26,6 Teile 4-(1,3-Dichlor-isopropylsulfonyl)-anilin werden mit 40 Volumenteilen einer wäßrigen konzentrierten Salzsäure zu einer homogenen Paste angerührt, die portionsweise im Laufe einer Stunde unter Rühren in eine Mischung aus 5 Volumenteilen einer wäßrigen 5N-Natriumnitritlösung in 200 Teilen Eiswasser eingetragen wird. Anschließend gibt man noch langsam 15 Volumenteile einer wäßrigen 5N-Natriumnitritlösung hinzu. Die erhaltene Diazoniumsalzlösung wird geklärt und in eine Lösung mit einem pH-Wert von 6 von 23,3 Teilen 1-($\beta$-Sulfoethyl)-4-methyl-6-hydroxy-2-pyridon in 250 Teilen Wasser eingerührt. Man führt die Kupplungsreaktion bei einer Temperatur von etwa 20 °C und einem pH-Wert von 6 zu Ende, saugt die erhaltene erfindungsgemäße Azoverbindung ab und trocknet sie.

Man erhält ein gelbes Farbstoffpulver des Natriumsalzes der Verbindung der Formel

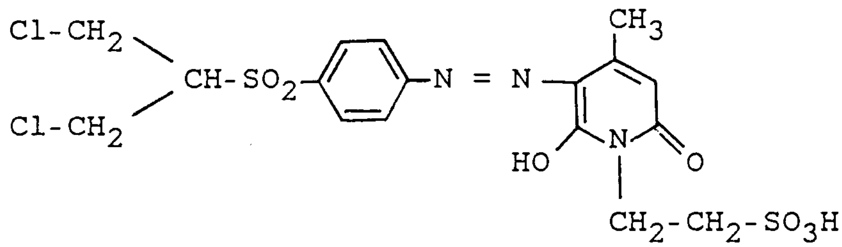

($\lambda_{max}$ = 409 nm)

die sehr gute faserreaktive Farbstoffeigenschaften besitzt und insbesondere auf Cellulosefasermaterialien, wie beispielsweise Baumwolle, nach den für faserreaktive üblichen Farbstoffe üblichen Applikations- und Fixierverfahren farbstarke gelbe Färbungen und Drucke mit guten Echtheitseigenschaften liefert, von denen insbesondere die guten Licht-, Naßlicht- und Schweißlichtechtheiten hervorgehoben werden können.

Beispiel 9

Eine Suspension von 9 Teilen 3-(1,3-Dichlorisopropylsulfonyl)-anilin in einem Gemisch aus 40 Vol.-Teilen Aceton, 80 Teilen Wasser und 16 Vol.-Teilen konzentrierter wäßriger Salzsäure wird in üblicher Weise durch Zugabe von Natriumnitrit diazotiert. Anschließend gibt man eine neutrale Lösung des Natriumsalzes von 7,9 Teilen 1-(4'-Sulfo-phenyl)-3-carboxy-pyrazol-5-on in 55 Teilen Wasser hinzu und stellt mit Natriumacetat einen pH-Wert von 4 ein. Nach beendeter Kupplungsreaktion, die in üblicher Weise bei 15 bis 20 °C durchgeführt wird, wird die erhaltene erfindungsgemäße Azoverbindung durch Aussalzen mittels Natriumchlorid und Filtration isoliert.

Sie besitzt, in Form der freien Säure geschrieben die Formel

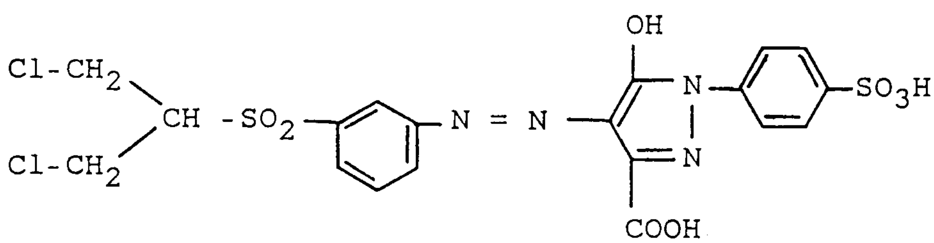

($\lambda_{max}$ = 410 nm)

und zeigt sehr gute faserreaktive Farbstoffeigenschaften. Bei Anwendung der für faserreaktive Farbstoffe bekannten Applikations- und Fixierverfahren erhält man mit ihr auf den in der Beschreibung genannten Materialien, wie insbesondere Cellulosefasermaterialen, beispielsweise Baumwolle, farbstarke, gelbe Färbungen und Drucke mit guten Echtheitseigenschaften, von denen insbesondere die Licht-, Naßlicht- und Schweißlichtechtheiten hervorgehoben werden können.

Beispiele 10 bis 59

In den nachfolgenden Tabellenbeispielen sind weitere erfindungsgemäße Azoverbindungen entsprechend der allgemeinen Formel (1) mit Hilfe der in dem jeweiligen Tabellenbeispiel angegebenen Komponenten beschrieben, wobei $D^1$, $D^2$ und $D^3$ die folgenden Bedeutungen haben:

$D^1$ = 4-(1',3'-Dichlor-isopropylsulfonyl)-phenyl

$D^2$ = 3-(1',3'-Dichlor-isopropylsulfonyl)-phenyl

$D^3$ = 4-(1'-Chlor-3'-acetyloxy-isopropylsulfonyl)-phenyl .

Diese erfindungsgemäßen Azoverbindungen lassen sich in erfindungsgemäßer Weise, beispielsweise analog einem der obigen Ausführungsbeispiele mit den aus den Tabellenbeispiel ersichtlichen Diazo- und Kupplungskomponenten, herstellen. Sie besitzen sehr gute faserreaktive Farbstoffeigenschaften und färben insbesondere Cellulosefasermaterialien nach den für faserreaktive Farbstoffe üblichen Applikations- und Fixierverfahren in dem für das jeweilige Tabellenbeispiel angegebenen Farbton mit guten Echtheitseigenschaften.

Azoverbindung der Formel (1)

| Bsp. | Rest D | Rest K | Farbton |
|---|---|---|---|
| 10 | $D^1$ | 1-(4'-ß-Sulfatoethylsulfonyl-phenyl)-3-methyl-pyrazol-5-on-4-yl | gelb (418) |
| 11 | $D^2$ | dito | gelb (415) |
| 12 | $D^1$ | 1-(4'-ß-Sulfatoethylsulfonyl-phenyl)-3-carboxy-pyrazol-5-on-4-yl | gelb (419) |
| 13 | $D^2$ | dito | gelb (407) |
| 14 | $D^1$ | 1-(4'-Sulfo-phenyl)-3-methyl-pyrazol-5-on-4-yl | gelb (421) |
| 15 | $D^2$ | dito | gelb (419) |
| 16 | $D^1$ | 3-Acetylamino-6-sulfo-8-hydroxy-naphth-7-yl | gelborange (480) |
| 17 | $D^1$ | 2-Benzoylamino-6-sulfo-8-hydroxy-naphth-7-yl | rot (491) |
| 18 | $D^1$ | 2-(N-Methyl-N-acetyl)-amino-6-sulfo-8-hydroxy-naphth-7-yl | orange (495) |
| 19 | $D^1$ | 1-Acetylamino-3,6-disulfo-8-hydroxy-naphth-7-yl | rot (498) |
| 20 | $D^1$ | 1-Benzoylamino-4,6-disulfo-8-hydroxy-naphth-7-yl | rot (499) |
| 21 | $D^1$ | 1-Benzoylamino-3,6-disulfo-8-hydroxy-naphth-7-yl | rot (500) |
| 22 | $D^1$ | 1-Acetylamino-4,6-disulfo-8-hydroxy-naphth-7-yl | rot (497) |

Azoverbindung der Formel (1)

| Bsp. | Rest D | Rest K | Farbton |
|---|---|---|---|
| 23 | $D^1$ | 3,6-Disulfo-2-hydroxy-naphth-1-yl | orange (483) |
| 24 | $D^2$ | 2-Acetylamino-5-sulfo-4-amino-phenyl | gelb (408) |
| 25 | $D^1$ | 2-Benzoylamino-5-sulfo-4-amino-phenyl | gelb (411) |
| 26 | $D^2$ | dito | gelb (410) |
| 27 | $D^1$ | 2-Ureido-5-amino-phenyl | gelb (390) |
| 28 | $D^2$ | dito | gelb (388) |
| 29 | $D^3$ | 2-Acetylamino-6-sulfo-8-hydroxy-naphth-7-yl | orange (489) |
| 30 | $D^2$ | 3-Acetylamino-6-sulfo-8-hydroxy-naphth-7-yl | gelborange (477) |
| 31 | $D^2$ | 2-Benzoylamino-6-sulfo-8-hydroxy-naphth-7-yl | orange (489) |
| 32 | $D^2$ | 2-(N-Methyl-N-acetyl)-amino-6-sulfo-8-hydroxy-naphth-7-yl | orange (492) |
| 33 | $D^2$ | 1-Acetylamino-3,6-disulfo-8-hydroxy-naphth-7-yl | rot (470) |
| 34 | $D^2$ | 1-Benzoylamino-4,6-disulfo-8-hydroxy-naphth-7-yl | rot (497) |
| 35 | $D^2$ | 1-Benzoylamino-3,6-disulfo-8-hydroxy-naphth-7-yl | rot (497) |

Azoverbindung der Formel (1)

| Bsp. | Rest D | Rest K | Farbton |
|---|---|---|---|
| 36 | $D^2$ | 1-Acetylamino-4,6-disulfo-8-hydroxy-naphth-7-yl | rot (498) |
| 37 | $D^2$ | 3,6-Disulfo-2-hydroxy-naphth-1-yl | orange (496) |
| 38 | $D^2$ | 2-Acetylamino-5-sulfo-4-amino-phenyl | gelb (480) |
| 39 | $D^1$ | 4-[N,N-Di-(ß-hydroxyethyl)]-amino-phenyl | gelb |
| 40 | $D^2$ | dito | gelb (430) |
| 41 | $D^2$ | 2-Chlor-4-[N,N-di-(ß-sulfato-ethyl)]-amino-phenyl | gelb (420) |
| 42 | $D^1$ | 4-[N,N-Di-(ß-sulfatoethyl)]-amino-phenyl | gelb (432) |
| 43 | $D^2$ | dito | gelb (430) |
| 44 | $D^1$ | 2-Chlor-5-methoxy-4-[N,N-di-(ß-hydroxyethyl)]-amino-phenyl | orangegelb (437) |
| 45 | $D^2$ | dito | orangegelb (435) |
| 46 | $D^2$ | 2-[4'-(ß-Sulfatoethylsulfonyl)-phenyl]-azo-1-amino-3,6-disulfo-8-hydroxy-naphth-7-yl | marineblau (593) |
| 47 | $D^1$ | 2-(4'-Sulfophenyl)-azo-1-amino-3,6-disulfo-8-hydroxy-naphth-7-yl | marineblau (596) |

Azoverbindung der Formel (1)

| Bsp. | Rest D | Rest K | Farbton |
|---|---|---|---|
| 48 | $D^2$ | dito | marineblau (594) |
| 49 | $D^1$ | 2-(2',5'-Disulfophenyl)-azo-1-amino-3,6-disulfo-8-hydroxy-naphth-7-yl | marineblau (590) |
| 50 | $D^2$ | dito | marineblau (588) |
| 51 | $D^2$ | N-(ß-Sulfoethyl)-4-methyl-2-hydroxy-pyrid-6-on-3-yl | gelb (407) |
| 52 | $D^1$ | 1-N-Ethyl-4-methyl-5-carbamoyl-2-hydroxy-pyrid-6-on-3-yl | gelb (404) |
| 53 | $D^2$ | dito | gelb (402) |
| 54 | $D^1$ | N-Ethyl-4-methyl-5-sulfomethyl-2-hydroxy-pyrid-6-on-3-yl | gelb (408) |
| 55 | $D^2$ | dito | gelb (406) |
| 56 | $D^1$ | N-Ethyl-4-methyl-5-cyano-2-hydroxy-pyrid-6-on-3-yl | gelb (405) |
| 57 | $D^2$ | dito | gelb (403) |
| 58 | $D^1$ | N-Ethyl-4-methyl-5-sulfo-2-hydroxy-pyrid-6-on-3-yl | gelb (404) |
| 59 | $D^2$ | dito | gelb (402) |

**Patentansprüche**

**Patentansprüche für folgende Vertragsstaaten : DE, GB, FR, IT, CH, LI**

**1.** Wasserlösliche Azoverbindung entsprechend der allgemeinen Formel (1)

$$D-N=N-K \quad (1)$$

in welcher bedeuten:

D ist ein Rest der allgemeinen Formel (2)

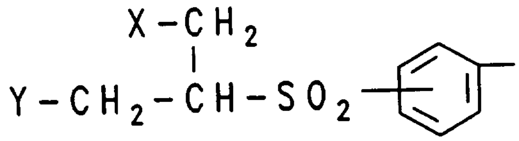

(2)

in welcher

Y ein Substituent ist, der durch Alkali unter Bildung der Vinylgruppe eliminierbar ist, wobei die Sulfonylgruppe bevorzugt in meta- oder para-Stellung zur freien Bindung an den Benzolkern gebunden ist, und

X Chlor oder Brom ist;

K ist ein Rest einer einfach ankuppelbaren, bevorzugt wasserlöslichen, Kupplungskomponente, die noch eine Azogruppe enthalten kann, oder der Rest einer doppelankuppelbaren, bevorzugt wasserlöslichen, Kupplungskomponente, jeweils aus der Reihe der Aminobenzole, der Phenole, der Naphthole, der Aminonaphthole oder der Acylamino-naphthole mit dem Acylrest einer Alkan- oder Alkencarbonsäure mit jeweils 1 bis 4 bzw. 2 bis 4 C-Atomen im Alkyl- bzw. Alkenylrest oder einer aromatischen Carbonsäure oder einer aromatischen Sulfonsäure oder einer N-substituierten Carbaminsäure oder aus der Reihe der Dihydroxynaphthalinsulfonsäuren, der Phenylazo- und Naphthylazo-aminonaphtholsulfonsäuren, der 5-Pyrazolone und 5-Aminopyrazole, der Acetoacetylarylide, der 2-Hydroxy-6-pyridone und der Hydroxychinoline, wobei K neben den in Farbstoffen üblichen Substituenten auch eine faserreaktive Gruppe der allgemeinen Formel $-SO_2-Y^1$, in welcher $Y^1$ die Vinylgruppe oder eine Gruppe der Formel $-CH_2-CH_2-Y$ mit Y einer der obigen Bedeutungen ist, enthalten kann.

**2.** Azoverbindung nach Anspruch 1, dadurch gekennzeichnet, daß K ein Rest der allgemeinen Formel (3c), (3f), (3h), (3i), (3p) oder (3q)

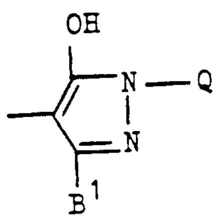

(3c)

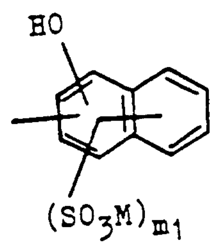

(3f)

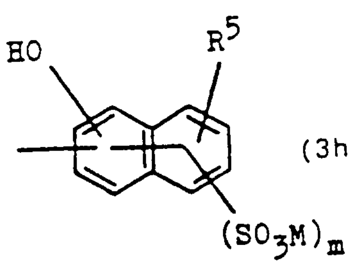

(3h)

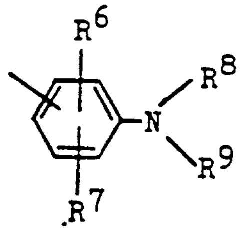

(3i)

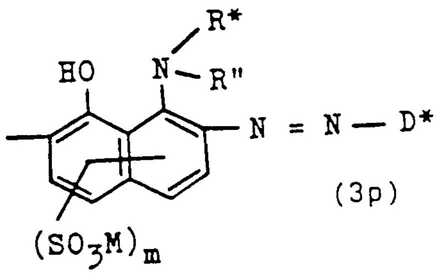
(3p)

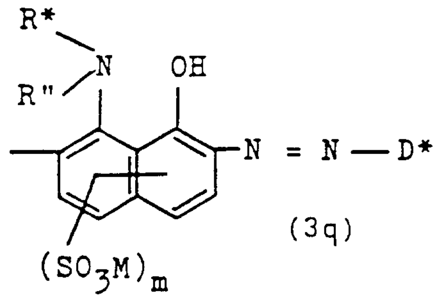
(3q)

ist, in welchen bedeuten:

- M ist Wasserstoff oder ein Alkalimetall;
- $B^1$ ist Alkyl von 1 bis 4 C-Atomen, Carboxy, Carbalkoxy von 2 bis 5 C-Atomen, Carbamoyl, Phenyl oder durch 1 oder 2 Substituenten aus der Gruppe Sulfo, Carboxy, Methyl, Ethyl, Methoxy, Ethoxy und Chlor substituiertes Phenyl;
- Q ist Phenyl, das durch 1, 2 oder 3 Substituenten aus der Gruppe Chlor, Brom, Methyl, Ethyl, Methoxy, Ethoxy, Carboxy, Sulfo und Alkanoylamino von 2 bis 5 C-Atomen und/oder durch eine Gruppe der allgemeinen Formel $-SO_2-Y^1$ substituiert sein kann, worin $Y^1$ die Vinylgruppe bedeutet oder die Ethylgruppe ist, die in $\beta$-Stellung einen alkalisch eliminierbaren Substituenten enthält;
- R* ist Wasserstoff oder Alkyl von 1 bis 4 C-Atomen, das durch Phenyl oder durch Sulfo und/oder eine Gruppe der allgemeinen Formel $-SO_2-Y^1$ mit $Y^1$ der obigen Bedeutung substituiertes Phenyl substituiert sein kann oder ist Alkanoyl von 2 bis 5 C-Atomen, gegebenenfalls substituiertes Phenylsulfonyl oder gegebenenfalls substituiertes Benzoyl;
- R" ist Wasserstoff oder Alkyl von 1 bis 4 C-Atomen, das durch Phenyl, Sulfophenyl oder eine Gruppe der allgemeinen Foreml $-SO_2-Y^1$ mit $Y^1$ der obigen Bedeutung substituiert sein kann, oder ist Phenyl oder durch 1 oder 2 Substituenten aus der Gruppe Alkyl von 1 bis 4 C-Atomen, Alkoxy von 1 bis 4 C-Atomen, Chlor, Brom, Sulfo und $-SO_2-Y^1$ mit $Y^1$ der obigen Bedeutung substituiertes Phenyl;
- $R^5$ ist Phenylureido, dessen Phenylrest durch eine Gruppe der Formel $-SO_2-Y^1$ mit $Y^1$ der obigen Bedeutung substituiert sein kann, oder ist Alkanoylamino von 2 bis 5 C-Atomen, das im Alkylrest durch eine Gruppe der Formel $-SO_2-Y^1$ mit $Y^1$ der obigen Bedeutung substituiert sein kann, oder ist Alkenoylamino von 3 bis 5 C-Atomen oder ist Benzoylamino, das durch Substituenten aus der Gruppe Chlor, Methyl, Methoxy, Nitro, Sulfo, Carboxy und $-SO_2-Y^1$ mit $Y^1$ der obigen Bedeutung substituiert sein kann;
- $R^6$ ist Wasserstoff, Alkyl von 1 bis 4 C-Atomen, Alkoxy von 1 bis 4 C-Atomen, Sulfo, Carboxy, Carbalkoxy von 2 bis 5 C-Atomen, Halogen oder Alkoxy von 1 bis 4 C-Atomen, das durch Hydroxy, Acetyloxy, Carboxy, Carbamoyl, Cyano oder Halogen substituiert ist;
- $R^7$ ist Wasserstoff, Alkyl von 1 bis 4 C-Atomen, Alkoxy von 1 bis 4 C-Atomen, Halogen, Cyano, Trifluormethyl, Alkoxy von 1 bis 4 C-Atomen, das durch Hydroxy, Acetyloxy, Carboxy, Carbamoyl, Cyano oder Halogen oder durch eine Gruppe der Formel $-SO_2-Y^1$ mit $Y^1$ der obigen Bedeutung substituiert ist, oder ist Alkanoylamino von 2 bis 5 C-Atomen, das durch Chlor, Brom, Alkoxy von 1 bis 4 C-Atomen, Phenoxy, Phenyl, Hydroxy, Carboxy oder Sulfo oder eine Gruppe der Formel $-SO_2-Y^1$ mit $Y^1$ der obigen Bedeutung substituiert sein kann, oder ist Alkenoylamino von 3 bis 5 C-Atomen, das durch Chlor, Brom, Carboxy oder Sulfo substituiert sein kann, oder ist Benzoylamino, das im Benzolkern durch Substituenten aus der Gruppe Chlor, Methyl, Sulfo und eine Gruppe der Formel $-SO_2-Y^1$ mit $Y^1$ der obigen Bedeutung substituiert sein kann, oder ist Alkylsulfonyl von 1 bis 4 C-Atomen oder Phenylsulfonyl, das im Benzolkern durch Substituenten aus der Gruppe Chlor, Methyl, Sulfo und eine Gruppe der Formel $-SO_2-Y^1$ mit $Y^1$ der obigen Bedeutung substituiert sein kann, oder ist Alkylsulfonylamino von 1 bis 4 C-Atomen, das durch Hydroxy, Sulfato, Chlor, Brom, Alkoxy von 1 bis 4 C-Atomen oder eine Gruppe der Formel $-SO_2-Y^1$ mit $Y^1$ der obigen Bedeutung substituiert sein kann, oder ist Phenylsulfonylamino, das im Benzolkern durch Substituenten aus der Gruppe Chlor, Methyl, Sulfo und eine Gruppe der Formel $SO_2$-$Y^1$ mit $Y^1$ der obigen Bedeutung substituiert sein kann, oder ist Carbamoyl, das am Stickstoffatom durch 1 oder 2 Substituenten mono- oder disubstituiert sein kann, wobei die Substituenten der Gruppe Alkyl von 1 bis 4 C-Atomen, durch Hydroxy, Sulfo, Carboxy, Sulfato, Phenyl oder eine Gruppe der Formel -$SO_2$-$Y^1$ mit $Y^1$ der obigen Bedeutung substituiertes Alkyl von 1 bis 4 C-Atomen, Cycloalkyl mit 5 bis 8 C-Atomen, Phenyl und durch Substituenten aus der Gruppe Chlor, Sulfo, Methyl, Methoxy, Carboxy und eine Gruppe der Formel -$SO_2$-$Y^1$ mit $Y^1$ der obigen Bedeutung substituiertes Phenyl angehören, oder ist Sulfamoyl, das am Stickstoffatom durch 1 oder 2 Substituenten mono- oder disubstituiert sein kann, wobei die Substituenten der Gruppe Alkyl von 1 bis 4 C-Atomen, durch Hydroxy, Sulfo, Carboxy, Sulfato, Phenyl oder eine Gruppe der Formel -$SO_2$-$Y^1$ mit $Y^1$ der obigen Bedeutung substituiertes Alkyl von 1 bis 4 C-Atomen, Cycloalkyl mit 5 bis 8 C-Atomen, Phenyl und durch Substituenten aus der Gruppe Chlor, Sulfo, Methyl, Methoxy, Carboxy und eine Gruppe der Formel -$SO_2$-$Y^1$ mit $Y^1$ der obigen Bedeutung substituiertes Phenyl angehören, oder ist Ureido oder Ureido, das am endständigen Stickstoffatom durch 1 oder 2 Substituenten mono- oder disubstituiert sein kann, wobei die Substituenten der Gruppe Alkyl von 1 bis 4 C-Atomen, durch Hydroxy, Sulfo, Carboxy, Sulfato, Phenyl oder eine Gruppe der Formel -$SO_2$-$Y^1$ mit $Y^1$ der obigen Bedeutungen substituiertes Alkyl von 1 bis 4 C-Atomen, Cycloalkyl mit 5 bis 8 C-Atomen, Phenyl und durch Substituenten aus der Gruppe Chlor, Sulfo, Methyl, Methoxy, Carboxy und eine Gruppe der Formel -$SO_2$-$Y^1$ mit $Y^1$ der obigen Bedeutung substituiertes Phenyl angehören;

$R^8$ ist Wasserstoff oder Alkyl von 1 bis 4 C-Atomen, das durch Hydroxy Sulfo, Carboxy, Sulfato, eine Gruppe -$SO_2$-$Y^1$ mit $Y^1$ der obigen Bedeutung, Phenyl oder Sulfophenyl substituiert sein kann, oder ist Alkenyl von 2 bis 4 C-Atomen, das durch Carboxy, Sulfo, Chlor oder Brom substituiert sein kann, oder ist Cycloalkyl von 5 bis 8 C-Atomen;

$R^9$ ist Wasserstoff oder Alkyl von 1 bis 4 C-Atomen, das durch Hydroxy, Sulfo, Carboxy, Sulfato, Phenyl oder -$SO_2$-$Y^1$ mit $Y^1$ obiger Bedeutung substituiert sein kann, oder ist Alkenyl von 2 bis 5 C-Atomen, das durch Carboxy, Sulfo oder -$SO_2$-$Y^1$ mit $Y^1$ obiger Bedeutung oder durch Chlor oder Brom substituiert sein kann, oder

$R^9$ ist Cycloalkyl von 5 bis 8 C-Atomen oder Phenyl, das durch Substituenten aus der Gruppe Chlor, Sulfo, Methyl, Methoxy, Carboxy und -$SO_2$-$Y^1$ mit $Y^1$ obiger Bedeutung substituiert sein kann, oder ist Naphthyl, das durch 1, 2 oder 3 Sulfo oder durch 1 oder 2 Sulfo und 1 oder 2 Gruppen der Formel -$SO_2$-$Y^1$ mit $Y^1$ der obigen Bedeutung oder nur durch eine solche Gruppe -$SO_2$-$Y^1$ substituiert ist, oder

$R^8$ und $R^9$ stellen zusammen mit dem Stickstoffatom den N-Piperidino-, N-Morpholino- oder N-Piperazino-Rest dar;

m ist die Zahl 1 oder 2;

$m_1$ ist die Zahl 1, 2 oder 3;

D* ist eine Gruppe der in Anspruch 1 definierten allgemeinen Formel (2), (2a) oder (2b) oder ist Phenyl, das durch 1, 2 oder 3 Substituenten aus der Gruppe Alkyl von 1 bis 4 C-Atomen, Alkoxy von 1 bis 4 C-Atomen, Chlor, Brom, Hydroxy, Carboxy, Sulfo, Carbamoyl, Sulfamoyl und Alkanoylamino von 2 bis 5 C-Atomen und/oder durch eine Gruppe der Formel -$SO_2$-$Y^1$ mit $Y^1$ der obengenannten Bedeutung substituiert sein kann, wobei bevorzugt einer dieser Substituenten eine Sulfo- oder Carboxygruppe ist und die Gruppe -$SO_2$-$Y^1$ bevorzugt in meta- oder para-Stellung zur Azogruppe steht, oder D* ist Naphthyl, das durch 1, 2 oder 3 Sulfogruppen oder durch 1 oder 2 Sulfogruppen und 1 oder 2 Gruppen der allgemeinen Formel -$SO_2$-$Y^1$ mit $Y^1$ der obengenannten Bedeutung oder nur durch eine solche Gruppe -$SO_2$-$Y^1$ substituiert ist,

wobei D und D* zueinander gleiche oder voneinander verschiedene Bedeutungen besitzen können.

**3.** Azoverbindung nach Anspruch 1, dadurch gekennzeichnet, daß K ein Rest der allgemeinen Formel (3d), (3e), 3(k) oder (3m)

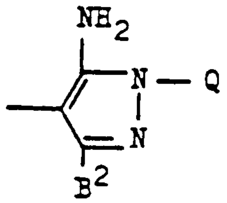

(3d)

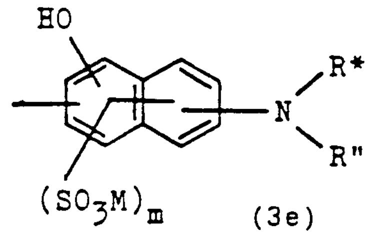

(3e)

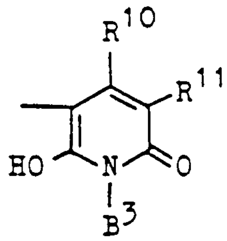

(3k)

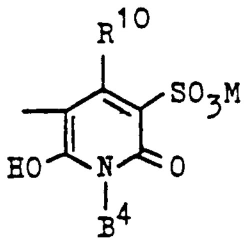

(3m)

in welchen bedeuten:

- M ist Wasserstoff oder ein Alkalimetall;
- $B^2$ ist Alkyl von 1 bis 4 C-Atomen, Carbalkoxy von 2 bis 5 C-Atomen, Carbamoyl, Phenyl oder durch 1 oder 2 Substituenten aus der Gruppe Alkyl von 1 bis 4 C-Atomen, Alkoxy von 1 bis 4 C-Atomen, Chlor, Brom und Sulfo substituiertes Phenyl;
- Q ist Phenyl, das durch 1, 2 oder 3 Substituenten aus der Gruppe Chlor, Brom, Methyl, Ethyl, Methoxy, Ethoxy, Carboxy, Sulfo und Alkanoylamino von 2 bis 5 C-Atomen und/oder durch eine Gruppe der allgemeinen Formel $-SO_2-Y^1$ substituiert sein kann, worin $Y^1$ die Vinylgruppe bedeutet oder die Ethylgruppe ist, die in β-Stellung einen alkalisch eliminierbaren Substituenten enthält;
- R* ist Wasserstoff oder Alkyl von 1 bis 4 C-Atomen, das durch Phenyl oder durch Sulfo und/oder eine Gruppe der allgemeinen Formel $-SO_2-Y^1$ mit $Y^1$ der obigen Bedeutung substituiertes Phenyl substituiert sein kann oder ist Alkanoyl von 2 bis 5 C-Atomen, gegebenenfalls substituiertes Phenylsulfonyl oder gegebenenfalls substituiertes Benzoyl;
- R" ist Wasserstoff oder Alkyl von 1 bis 4 C-Atomen, das durch Phenyl, Sulfophenyl oder eine Gruppe der allgemeinen Foreml $-SO_2-Y^1$ mit $Y^1$ der obigen Bedeutung substituiert sein kann, oder ist Phenyl oder durch 1 oder 2 Substituenten aus der Gruppe Alkyl von 1 bis 4 C-Atomen, Alkoxy von 1 bis 4 C-Atomen, Chlor, Brom, Sulfo und $-SO_2-Y^1$ mit $Y^1$ der obigen Bedeutung substituiertes Phenyl;
- m ist die Zahl 1 oder 2;
- $B^3$ ist Wasserstoff oder Alkyl von 1 bis 4 C-Atomen, das durch Phenyl, Sulfo, Sulfophenyl oder eine Gruppe $-SO_2-Y^1$ mit $Y^1$ der in Anspruch 1 genannten Bedeutung substituiert sein kann;
- $R^{10}$ ist Wasserstoff oder Alkyl von 1 bis 4 C-Atomen oder durch Alkoxy von 1 bis 4 C-Atomen oder Cyano substituiertes Alkyl von 1 bis 4 C-Atomen;
- $R^{11}$ ist Wasserstoff, Carboxy, Sulfo, Sulfoalkyl mit einem Alkylenrest von 1 bis 4 C-Atomen, Cyano oder Carbamoyl;
- $B^4$ ist Wasserstoff, Alkyl von 1 bis 4 C-Atomen oder durch Alkoxy von 1 bis 4 C-Atomen, Sulfo, Carboxy, Sulfato, Phenyl, Sulfophenyl, Acetylamino, Benzoylamino, Cyano oder eine Gruppe der Formel $-SO_2-Y^1$ mit $Y^1$ obiger Bedeutung substituiertes Alkyl von 1 bis 4 C-Atomen oder ist Alkenyl von 2 bis 4 C-Atomen, Cyclohexyl, Phenyl oder durch Substituenten aus der Gruppe Carboxy, Sulfo, Benzoylamino, Acetylamino, $-SO_2-Y^1$ mit $Y^1$ obiger Bedeutung und Chlor substituiertes Phenyl.

**4.** Azoverbindung nach Anspruch 2, dadurch gekennzeichnet, daß K ein Rest der allgemeinen Formel (3c) oder (3i) ist, in welchen

- $B^1$ Methyl, Carboxy oder Carbalkoxy von 2 bis 5 C-Atomen ist,
- Q Phenyl ist, das durch Sulfo und/oder eine Gruppe der Formel $-SO_2-Y^1$ substituiert ist, worin

$Y^1$ die in Anspruch 2 genannte Bedeutung besitzt, und bevorzugt β-Sulfatoethyl ist,
$R^6$ Wasserstoff, Sulfo, Methyl, Ethyl, Methoxy oder Ethoxy ist,
$R^7$ Wasserstoff, Methyl, Ethyl, Methoxy, Ethoxy, Chlor, Amino, substituiertes Amino, Alkanoylamino von 2 bis 5 C-Atomen, Benzoylamino oder Phenylsulfonylamino ist,
$R^8$ Wasserstoff, Methyl, Ethyl oder durch Hydroxy, Sulfo, Sulfato oder Carboxy substituiertes Alkyl von 2 bis 4 C-Atomen ist und
$R^9$ Wasserstoff, Methyl, Ethyl oder Alkyl von 2 bis 4 C-Atomen, das durch Hydroxy, Sulfo, Sulfato oder Carboxy substituiert ist, bedeutet.

**5.** Azoverbindung nach Anspruch 3, dadurch gekennzeichnet, daß K ein Rest der allgemeinen Formel (3e), (3k) oder (3m) ist, in welchen
$R^*$ Acetyl, Sulfobenzoyl oder Benzoyl ist,
R" Wasserstoff oder Alkyl von 1 bis 4 C-Atomen ist,
m die Zahl 1 oder 2 ist,
M Wasserstoff oder ein Alkalimetall ist,
$R^{10}$ Wasserstoff oder bevorzugt Methyl ist,
$R^{11}$ Wasserstoff, Cyano, Sulfomethyl, Carbamoyl oder Carboxy ist,
$B^3$ Wasserstoff, Ethyl oder durch Sulfo, Phenyl oder Sulfophenyl substituiertes Alkyl von 2 bis 4 C-Atomen ist und
$B^4$ Wasserstoff, Methyl, Ethyl oder durch Sulfo, Phenyl oder Sulfophenyl substituiertes Alkyl von 2 bis 4 C-Atomen ist.

**6.** Azoverbindung nach Anspruch 3, dadurch gekennzeichnet, daß K ein Rest der allgemeinen Formel (3p) ist, in welcher
$R^*$ Acetyl, Sulfobenzoyl oder Benzoyl oder bevorzugt Wasserstoff ist,
R" Alkyl von 1 bis 4 C-Atomen oder bevorzugt Wasserstoff ist,
m die Zahl 2 ist und
M Wasserstoff oder ein Alkalimetall ist.

**7.** Azoverbindung nach mindestens einem der Ansprüche 1 bis 6, dadurch gekennzeichnet, daß X Chlor ist.

**8.** Azoverbindung nach mindestens einem der Ansprüche 1 bis 7, dadurch gekennzeichnet, daß Y Sulfato oder bevorzugt Chlor ist.

**9.** Verfahren zur Herstellung einer Azoverbindung der in Anspruch 1 definierten allgemeinen Formel (1), dadurch gekennzeichnet, daß man eine Diazoniumverbindung einer Aminoverbindung der allgemeinen Formel (4)

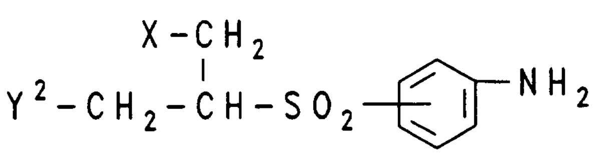

(4)

in welcher X die in Anspruch 1 genannte Bedeutung hat und $Y^2$ für die Hydroxygruppe steht oder eine der Bedeutungen von Y besitzt, mit einer Kupplungskomponente der allgemeinen Formel H-K mit K der in Anspruch 1 genannten Bedeutung umsetzt, oder daß man im Falle der Herstellung einer Disazoverbindung entsprechend der allgemeinen Formel (1), in welcher K einen Rest der allgemeinen Formel (3q) bedeutet, eine Monoazoverbindung der allgemeinen Formel

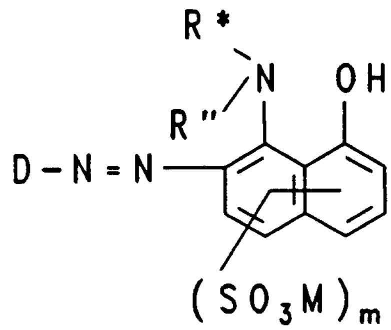

in welcher D, R*, R'', M und m eine der in Anspruch 1 bzw. Anspruch 2 genannten Bedeutungen haben, mit einer Diazoniumverbindung eines Amins der allgemeinen Formel $D^*$-$NH_2$ mit $D^*$ der in Anspruch 2 genannten Bedeutungen kuppelt,
und daß man im Falle der Verwendung einer Verbindung der Formel (4) mit $Y^2$ gleich einer Hydroxygruppe in der erhaltenen Azoverbindung die Hydroxygruppe $Y^2$ in eine Gruppe Y gemäß Formel (1) überführt.

**10.** Verwendung einer Verbindung entsprechend der allgemeinen Formel (1) von Anspruch 1 zum Färben (einschließlich Bedrucken) von hydroxy- und/oder carbonamidgruppenhaltigem Material, insbesondere Fasermaterial.

**11.** Verfahren zum Färben (einschließlich Bedrucken) von hydroxy- und/oder carbonamidgruppenhaltigem Material, insbesondere Fasermaterial, bei welchem man einen Farbstoff auf das Material aufbringt oder darin einbringt und ihn mittels Wärme und/oder mit Hilfe eines alkalisch wirkenden Mittels fixiert, dadurch gekennzeichnet, daß man als Farbstoff eine Verbindung entsprechend der allgemeinen Formel (1) von Anspruch 1 einsetzt.

**12.** Verbindung entsprechend der allgemeinen Formel (7)

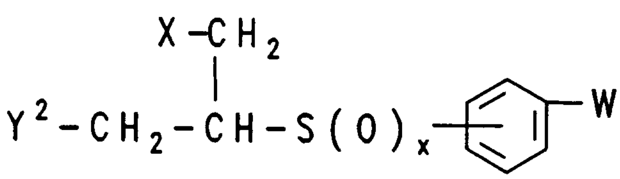

(7)

in welcher
- X Chlor oder Brom ist,
- x die Zahl Null oder 2 bedeutet,
- W Nitro oder Amino ist und
- $Y^2$ die Hydroxygruppe oder ein Substituent ist, der durch Alkali unter Bildung der Vinylgruppe eliminierbar ist.

**13.** Verbindung nach Anspruch 12 entsprechend der allgemeinen Formel (4)

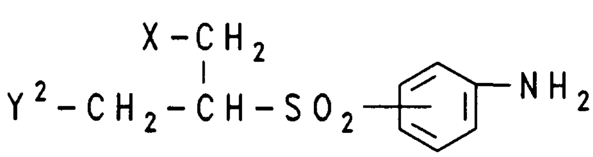

(4)

in welcher X und $Y^2$ die in Anspruch 12 genannte Bedeutung haben.

**14.** Verbindung nach Anspruch 12 oder 13, dadurch gekennzeichnet, daß X Chlor ist.

**15.** Verbindung nach mindestens einem der Ansprüche 12 bis 14, dadurch gekennzeichnet, daß $Y^2$ Chlor, Brom, Cyano, Alkanoyloxy von 2 bis 5 C-Atomen oder Sulfato oder bevorzugt Chlor, Brom, Cyano oder Acetyloxy ist.

**16.** Verfahren zur Herstellung einer Verbindung entsprechend der in Anspruch 12 genannten und definierten allgemeinen Formel (7), dadurch gekennzeichnet, daß man ein Nitrobenzol-sulfenylhalogenid mit einer Verbindung der allgemeinen Formel (8)

$Y^2 - CH_2 - CH = CH_2$ (8)

in welcher $Y^2$ die in Anspruch 12 genannte Bedeutung hat, zur Verbindung der Formel (7), in welcher $Y^2$ und X die in Anspruch 12 genannten Bedeutungen haben, x die Zahl Null ist und W die Nitrogruppe bedeutet, umsetzt, und daß man im Falle der Herstellung einer Verbindung der allgemeinen Formel (7), in welcher $Y^2$ und X die obengenannten Bedeutungen haben, x die Zahl 2 bedeutet und W für die Nitrogruppe steht, die Verbindung entsprechend der allgemeinen Formel (7), in welcher $Y^2$ und X die obengenannten Bedeutungen haben, x die Zahl Null ist und W die Nitrogruppe bedeutet, mit einem Oxidationsmittel umsetzt, und daß man im Falle der Herstellung einer Verbindung der allgemeinen Formel (7), in welcher $Y^2$ und X die obengenannten Bedeutungen haben, x für die Zahl 2 steht und W die Aminogruppe ist, in der Verbindung der Formel (7), in welcher $Y^2$ und X die obengenannten Bedeutungen haben, x die Zahl 2 bedeutet und W die Nitrogruppe ist, diese Nitrogruppe reduziert.

**17.** Verwendung einer Verbindung der allgemeinen Formel (7) von Anspruch 12, in welcher $Y^2$ und X die in Anspruch 12 genannten Bedeutungen haben, x gleich 2 ist und W die Aminogruppe bedeutet, als Diazokomponente zur Synthese von Azoverbindungen.

**Patentansprüche für folgenden Vertragsstaat : ES**

**1.** Verfahren zur Herstellung einer wasserlöslichen Azoverbindung entsprechend der allgemeinen Formel (1)

$D - N = N - K$ (1)

in welcher bedeuten:

D ist ein Rest der allgemeinen Formel (2)

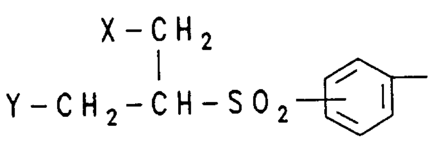

(2)

in welcher

Y ein Substituent ist, der durch Alkali unter Bildung der Vinylgruppe eliminierbar ist, wobei die Sulfonylgruppe bevorzugt in meta- oder para-Stellung zur freien Bindung an den Benzolkern gebunden ist, und

X Chlor oder Brom ist;

K ist ein Rest einer einfach ankuppelbaren, bevorzugt wasserlöslichen, Kupplungskomponente, die noch eine Azogruppe enthalten kann, oder der Rest einer doppelankuppelbaren, bevorzugt wasserlöslichen, Kupplungskomponente, jeweils aus der Reihe der Aminobenzole, der Phenole, der Naphthole, der Aminonaphthole oder der Acylamino-naphthole mit dem Acylrest einer Alkan- oder Alkencarbonsäure mit jeweils 1 bis 4 bzw. 2 bis 4 C-Atomen im Alkyl- bzw. Alkenylrest oder einer aromatischen Carbonsäure oder einer aromatischen Sulfonsäure oder einer N-substituierten Carbaminsäure oder aus der Reihe der Dihydroxynaphthalinsulfonsäuren, der Phenylazo- und Naphthylazoaminonaphtholsulfonsäuren, der 5-Pyrazolone und 5-Aminopyrazole, der Acetoacetylarylide, der 2-Hydroxy-6-pyridone und der Hydroxychinoline, wobei K neben den in Farbztoffen üblichen Substituenten auch eine faserreaktive Gruppe der allgemeinen Formel $-SO_2-Y^1$, in welcher $Y^1$ die Vinylgruppe oder eine Gruppe der Formel $-CH_2-CH_2-Y$ mit Y einer der obigen Bedeutungen ist, enthalten kann,

dadurch gekennzeichnet, daß man eine Diazoniumverbindung einer Aminoverbindung der allgemeinen Formel (4)

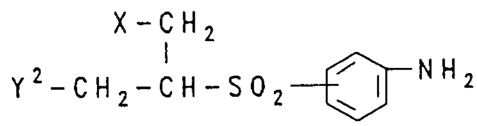

(4)

in welcher X die oben genannte Bedeutung hat und $Y^2$ für die Hydroxygruppe steht oder eine der Bedeutungen von Y besitzt, mit einer Kupplungskomponente der allgemeinen Formel H-K mit K der obengenannten Bedeutung umsetzt, oder daß man im Falle der Herstellung einer Disazoverbindung entsprechend der allgemeinen Formel (1), in welcher K ein Rest der allgemeinen Formel (3q)

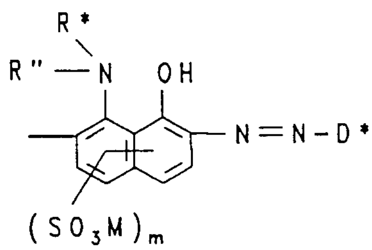

(3q)

ist, worin

- M Wasserstoff oder ein Alkalimetall ist,
- m die Zahl 1 oder 2 ist,
- R* Wasserstoff oder Alkyl von 1 bis 4 C-Atomen ist, das durch Phenyl oder durch Sulfo und/oder eine Gruppe der allgemeinen Formel $-SO_2-Y^1$ mit $Y^1$ der obigen Bedeutung substituiertes Phenyl substituiert sein kann, oder Alkanoyl von 2 bis 5 C-Atomen, gegebenenfalls substituiertes Phenylsulfonyl oder gegebenenfalls substituiertes Benzoyl ist,
- R" Wasserstoff oder Alkyl von 1 bis 4 C-Atomen ist, das durch Phenyl, Sulfophenyl oder eine Gruppe der allgemeinen Formel $-SO_2-Y^1$ mit $Y^1$ der obigen Bedeutung substituiert sein kann, oder Phenyl oder durch 1 oder 2 Substituenten aus derGruppe Alkyl von 1 bis 4 C-Atomen, Alkoxy von 1 bis 4 C-Atomen, Chlor, Brom, Sulfo und $-SO_2-Y^1$ mit $Y^1$ der obigen Bedeutung substituiertes Phenyl ist und
- D* eine Gruppe der definierten allgemeinen Formel (2) oder Phenyl ist, das durch 1, 2 oder 3 Substituenten aus der Gruppe Alkyl von 1 bis 4 C-Atomen, Alkoxy von 1 bis 4 C-Atomen, Chlor, Brom, Hydroxy, Carboxy, Sulfo, Carbamoyl, Sulfamoyl und Alkanoylamino von 2 bis 5 C-Atomen und/oder durch eine Gruppe der Formel $-SO_2-Y^1$ mit $Y^1$ der obengenannten Bedeutung substituiert sein kann, wobei bevorzugt einer dieser Substituenten eine Sulfo- oder Carboxygruppe ist und die Gruppe $-SO_2-Y^1$ bevorzugt in meta- oder para-Stellung zur Azogruppe steht, oder D* Naphthyl ist, das durch 1, 2 oder 3 Sulfogruppen oderdurch 1 oder 2 Sulfogrppen und 1 oder 2 Gruppen der allgemeinen Formel $-SO_2-Y^1$ mit $Y^1$ der obengenannten Bedeutung oder nur durch eine solche Gruppe $-SO_2-Y^1$ substituiert ist,

wobei D und D* zueinander gleiche oder voneinander verschiedene Bedeutungen besitzen können, eine Monoazoverbindung der allgemeinen Formel

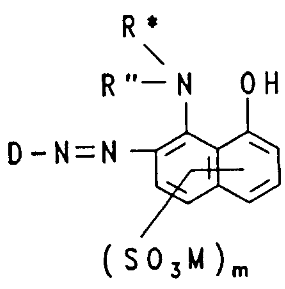

in welcher D, R*, R", M und m die obengenannten Bedeutungen haben, mit einer Dazoniumverbindung eines Amins der allgemeinen Formel $D^*$-$NH_2$ mit $D^*$ der obengenannten Bedeutung kuppelt, und daß man im Falle der Verwendung einer Verbindung der Formel (4) mit $Y^2$ gleich einer Hydroxygruppe in der erhaltenen Azoverbindung die Hydroxygruppe $Y^2$ in eine Gruppe Y gemäß Formel (1) überführt.

**2.** Verfahren nach Anspruch 1, dadurch gekennzeichnet, daß K ein Rest der allgemeinen Formel (3c), (3f), (3h), (3i), (3p) oder (3q)

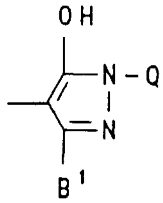
(3c)

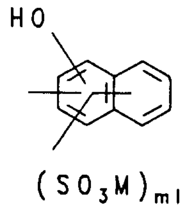
(3f)

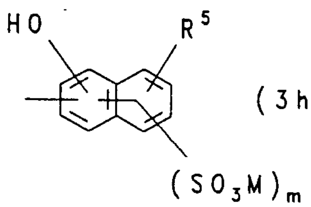
(3h)

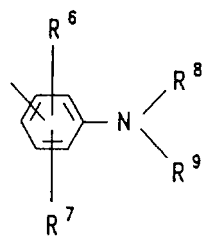
(3i)

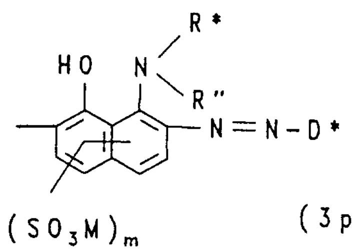
(3p)

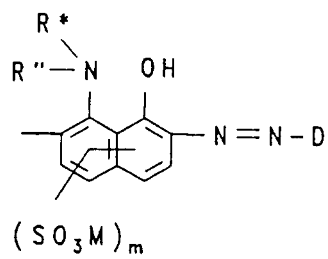
(3q)

ist, in welchen bedeuten:

| | |
|---|---|
| M | ist Wasserstoff oder ein Alkalimetall; |
| $B^1$ | ist Alkyl von 1 bis 4 C-Atomen, Carboxy, Carbalkoxy von 2 bis 5 C-Atomen, Carbamoyl, Phenyl oder durch 1 oder 2 Substituenten aus der Gruppe Sulfo, Carboxy, Methyl, Ethyl, Methoxy, Ethoxy und Chlor substituiertes Phenyl; |
| Q | ist Phenyl, das durch 1, 2 oder 3 Substituenten aus der Gruppe Chlor, Brom, Methyl, Ethyl, Methoxy, Ethoxy, Carboxy, Sulfo und Alkanoylamino von 2 bis 5 C-Atomen und/oder durch eine Gruppe der allgemeinen Formel -$SO_2$-$Y^1$ substituiert sein kann, worin $Y^1$ die Vinylgruppe bedeutet oder die Ethylgruppe ist, die in $\beta$-Stellung einen alkalisch eliminierbaren Substituenten enthält; |
| R* | ist Wasserstoff oder Alkyl von 1 bis 4 C-Atomen, das durch Phenyl oder durch Sulfo und/oder eine Gruppe der allgemeinen Formel -$SO_2$-$Y^1$ mit $Y^1$ der obigen Bedeutung substituiertes Phenyl substituiert sein kann, oder ist Alkanoyl von 2 bis 5 C-Atomen, gegebenenfalls substituiertes Phenylsulfonyl oder gegebenenfalls substituiertes Benzoyl; |
| R" | ist Wasserstoff oder Alkyl von 1 bis 4 C-Atomen, das durch Phenyl, Sulfophenyl oder eine Gruppe der allgemeinen Formel -$SO_2$-$Y^1$ mit $Y^1$ der obigen Bedeutung substituiert sein kann, oder ist Phenyl oder durch 1 oder 2 Substituenten aus der Gruppe Alkyl von 1 bis 4 C-Atomen, Alkoxy von 1 bis 4 C-Atomen, Chlor, Brom, Sulfo und -$S0_2$-$Y^1$ mit $Y^1$ der obigen Bedeutung substituiertes Phenyl; |
| $R^5$ | ist Phenylureido, dessen Phenylrest durch eine Gruppe der Formel -$SO_2$-$Y^1$ mit $Y^1$ der obigen Bedeutung substituiert sein kann, oder ist Alkanoylamino von 2 bis 5 C- |

Atomen, das im Alkylrest durch eine Gruppe der Formel $-S0_2-Y^1$ mit $Y^1$ der obigen Bedeutung substituiert sein kann, oder ist Alkenoylamino von 3 bis 5 C-Atomen oder ist Benzoylamino, das durch Substituenten aus der Gruppe Chlor, Methyl, Methoxy, Nitro, Sulfo, Carboxy und $-S0_2-Y^1$ mit $Y^1$ der obigen Bedeutung substituiert sein kann;

$R^6$ ist Wasserstoff, Alkyl von 1 bis 4 C-Atomen, Alkoxy von 1 bis 4 C-Atomen, Sulfo, Carboxy, Carbalkoxy von 2 bis 5 C-Atomen, Halogen oder Alkoxy von 1 bis 4 C-Atomen, das durch Hydroxy, Acetyloxy, Carboxy, Carbamoyl, Cyano oder Halogen substituiert ist;

$R^7$ ist Wasserstoff, Alkyl von 1 bis 4 C-Atomen, Alkoxy von 1 bis 4 C-Atomen, Halogen, Cyano, Trifluormethyl, Alkoxy von 1 bis 4 C-Atomen, das durch Hydroxy, Acetyloxy, Carboxy, Carbamoyl, Cyano oder Halogen oder durch eine Gruppe der Formel $-S0_2-Y^1$ mit $Y^1$ der obigen Bedeutung substituiert ist, oder ist Alkanoylamino von 2 bis 5 C-Atomen, das durch Chlor, Brom, Alkoxy von 1 bis 4 C-Atomen, Phenoxy, Phenyl, Hydroxy, Carboxy oder Sulfo oder eine Gruppe der Formel $-SO_2-Y^1$ mit $Y^1$ der obigen Bedeutung substituiert sein kann, oder ist Alkenoylamino von 3 bis 5 C-Atomen, das durch Chlor, Brom, Carboxy oder Sulfo substituiert sein kann, oder ist Benzoylamino, das im Benzolkern durch Substituenten aus der Gruppe Chlor, Methyl, Sulfo und eine Gruppe der Formel $-S0_2-Y^1$ mit $Y^1$ der obigen Bedeutung substituiert sein kann, oder ist Alkylsulfonyl von 1 bis 4 C-Atomen oder Phenylsulfonyl, das im Benzolkern durch Substituenten aus der Gruppe Chlor, Methyl, Sulfo und eine Gruppe der Formel $-S0_2Y^1$ mit $Y^1$ der obigen Bedeutung substituiert sein kann, oder ist Alkylsulfonylamino von 1 bis 4 C-Atomen, das durch Hydroxy, Sulfato, Chlor, Brom, Alkoxy von 1 bis 4 C-Atomen oder eine Gruppe der Formel $-S0_2-Y^1$ mit $Y^1$ der obigen Bedeutung substituiert sein kann, oder ist Phenylsulfonylamino, das im Benzolkern durch Substituenten aus der Gruppe Chlor, Methyl, Sulfo und eine Gruppe der Formel $-S0_2-Y^1$ mit $Y^1$ der obigen Bedeutung substituiert sein kann, oder ist Carbamoyl, das am Stickstoffatom durch 1 oder 2 Substituenten mono- oder disubstituiert sein kann, wobei die Substituenten der Gruppe Alkyl von 1 bis 4 C-Atomen, durch Hydroxy, Sulfo, Carboxy, Sulfato, Phenyl oder eine Gruppe der Formel $-S0_2-Y^1$ mit $Y^1$ der obigen Bedeutung substituiertes Alkyl von 1 bis 4 C-Atomen, Cycloalkyl mit 5 bis 8 C-Atomen, Phenyl und durch Substituenten aus der Gruppe Chlor, Sulfo, Methyl, Methoxy, Carboxy und eine Gruppe der Formel $-S0_2-Y^1$ mit $Y^1$ der obigen Bedeutung substituiertes Phenyl angehören, oder ist Sulfamoyl, das am Stickstoffatom durch 1 oder 2 Substituenten mono- oder disubstituiert sein kann, wobei die Substituenten der Gruppe Alkyl von 1 bis 4 C-Atomen, durch Hydroxy, Sulfo, Carboxy, Sulfato, Phenyl oder eine Gruppe der Formel $-S0_2-Y^1$ mit $Y^1$ der obigen Bedeutung substituiertes Alkyl von 1 bis 4 C-Atomen, Cycloalkyl mit 5 bis 8 C-Atomen, Phenyl und durch Substituenten aus der Gruppe Chlor, Sulfo, Methyl, Methoxy, Carboxy und eine Gruppe der Formel $-S0_2-Y^1$ mit $Y^1$ der obigen Bedeutung substituiertes Phenyl angehören, oder ist Ureido oder Ureido, das am endständigen Stickstoffatom durch 1 oder 2 Substituenten mono- oder disubstituiert sein kann, wobei die Substituenten der Gruppe Alkyl von 1 bis 4 C-Atomen, durch Hydroxy, Sulfo, Carboxy, Sulfato, Phenyl oder eine Gruppe der Formel $-S0_2-Y^1$ mit $Y^1$ der obigen Bedeutungen substituiertes Alkyl von 1 bis 4 C-Atomen, Cycloalkyl mit 5 bis 8 C-Atomen, Phenyl und durch Substituenten aus der Gruppe Chlor, Sulfo, Methyl, Methoxy, Carboxy und eine Gruppe der Formel $-S0_2-Y^1$ mit $Y^1$ der obigen Bedeutung substituiertes Phenyl angehören;

$R^8$ ist Wasserstoff oder Alkyl von 1 bis 4 C-Atomen, das durch Hydroxy, Sulfo, Carboxy, Sulfato, eine Gruppe $-S0_2-Y^1$ mit $Y^1$ der obigen Bedeutung, Phenyl oder Sulfophenyl substituiert sein kann, oder ist Alkenyl von 2 bis 4 C-Atomen, das durch Carboxy, Sulfo, Chlor oder Brom substituiert sein kann, oder ist Cycloalkyl von 5 bis 8 C-Atomen;

$R^9$ ist Wasserstoff oder Alkyl von 1 bis 4 C-Atomen, das durch Hydroxy, Sulfo, Carboxy, Sulfato, Phenyl oder $-S0_2-Y^1$ mit $Y^1$ obiger Bedeutung substituiert sein kann, oder ist Alkenyl von 2 bis 5 C-Atomen, das durch Carboxy, Sulfo oder $-S0_2-Y^1$ mit $Y^1$ obiger Bedeutung oder durch Chlor oder Brom substituiert sein kann, oder

$R^9$ ist Cycloalkyl von 5 bis 8 C-Atomen oder Phenyl, das durch Substituenten aus der Gruppe Chlor, Sulfo, Methyl, Methoxy, Carboxy und $-S0_2-Y^1$ mit $Y^1$ obiger Bedeutung

substituiert sein kann, oder ist Naphthyl, das durch 1, 2 oder 3 Sulfo oder durch 1 oder 2 Sulfo und 1 oder 2 Gruppen der Formel $-S0_2-Y^1$ mit $Y^1$ der obigen Bedeutung oder nur durch eine solche Gruppe $-S0_2-Y^1$ substituiert ist, oder

$R^8$ und $R^9$ stellen zusammen mit dem Stickstoffatom den N-Piperidino-, N-Morpholino- oder N-Piperazino-Rest dar;

m ist die Zahl 1 oder 2;

$m_1$ ist die Zahl 1, 2 oder 3;

D* ist eine Gruppe der in Anspruch 1 definierten allgemeinen Formel (2) oder ist Phenyl, das durch 1, 2 oder 3 Substituenten aus der Gruppe Alkyl von 1 bis 4 C-Atomen, Alkoxy von 1 bis 4 C-Atomen, Chlor, Brom, Hydroxy, Carboxy, Sulfo, Carbamoyl, Sulfamoyl und Alkanoylamino von 2 bis 5 C-Atomen und/oder durch eine Gruppe der Formel $-S0_2-Y^1$ mit $Y^1$ der obengenannten Bedeutung substituiert sein kann, wobei bevorzugt einer dieser Substituenten eine Sulfo- oder Carboxygruppe ist und die Gruppe $-S0_2-Y^1$ bevorzugt in meta- oder para-Stellung zur Azogruppe steht, oder D* ist Naphthyl, das durch 1 , 2 oder 3 Sulfogruppen oder durch 1 oder 2 Sulfogruppen und 1 oder 2 Gruppen der allgemeinen Formel $-S0_2-Y^1$ mit $Y^1$ der obengenannten Bedeutung oder nur durch eine solche Gruppe $-S0_2-Y^1$ substituiert ist,

wobei D und D* zueinander gleiche oder voneinander verschiedene Bedeutungen besitzen können.

3. Verfahren nach Anspruch 1, dadurch gekennzeichnet, daß K ein Rest der allgemeinen Formel (3d), (3e), (3k) oder (3m)

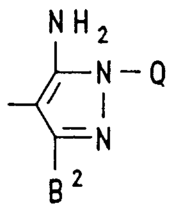
(3d)

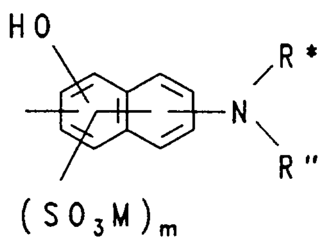
(3e)

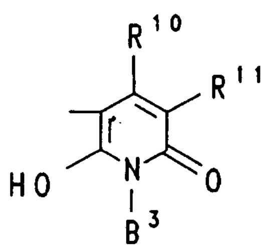
(3k)

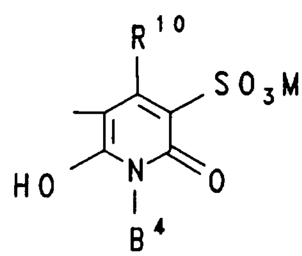
(3m)

ist, in welchen bedeuten:

M ist Wasserstoff oder ein Alkalimetall;

$B^2$ ist Alkyl von 1 bis 4 C-Atomen, Carbalkoxy von 2 bis 5 C-Atomen, Carbamoyl, Phenyl oder durch 1 oder 2 Substituenten aus der Gruppe Alkyl von 1 bis 4 C-Atomen, Alkoxy von 1 bis 4 C-Atomen, Chlor, Brom und Sulfo substituiertes Phenyl;

Q ist Phenyl, das durch 1, 2 oder 3 Substituenten aus der Gruppe Chlor, Brom, Methyl, Ethyl, Methoxy, Ethoxy, Carboxy, Sulfo und Alkanoylamino von 2 bis 5 C-Atomen und/oder durch eine Gruppe der allgemeinen Formel $-S0_2-Y^1$ substituiert sein kann, worin $Y^1$ die Vinylgruppe bedeutet oder die Ethylgruppe ist, die in $\beta$-Stellung einen alkalisch eliminierbaren Substituenten enthält;

R* ist Wasserstoff oder Alkyl von 1 bis 4 C-Atomen, das durch Phenyl oder durch Sulfo und/oder eine Gruppe der allgemeinen Formel $-SO_2-Y^1$ mit $Y^1$ der obigen Bedeutung substituiertes Phenyl substituiert sein kann, oder ist Alkanoyl von 2 bis 5 C-Atomen, gegebenenfalls substituiertes Phenylsulfonyl oder gegebenenfalls substituiertes Benzoyl;

R'' ist Wasserstoff oder Alkyl von 1 bis 4 C-Atomen, das durch Phenyl, Sulfophenyl oder eine

Gruppe der allgemeinen Formel $-SO_2-Y^1$ mit $Y^1$ der obigen Bedeutung substituiert sein kann, oder ist Phenyl oder durch 1 oder 2 Substituenten aus der Gruppe Alkyl von 1 bis 4 C-Atomen, Alkoxy von 1 bis 4 C-Atomen, Chlor, Brom, Sulfo und $-SO_2-Y^1$ mit $Y^1$ der obigen Bedeutung substituiertes Phenyl;

m ist die Zahl 1 oder 2;

$B^3$ ist Wasserstoff oder Alkyl von 1 bis 4 C-Atomen, das durch Pheny, Sulfo, Sulfophenyl oder eine Gruppe $-SO_2-Y^1$ mit $Y^1$ der in Anspruch 1 genannten Bedeutung substituiert sein kann;

$R^{10}$ ist Wasserstoff oder Alkyl von 1 bis 4 C-Atomen oder durch Alkoxy von 1 bis 4 C-Atomen oder Cyano substituiertes Alkyl von 1 bis 4 C-Atomen;

$R^{11}$ ist Wasserstoff, Carboxy, Sulfo, Sulfoalkyl mit einem Alkylenrest von 1 bis 4 C-Atomen, Cyano oder Carbamoyl;

$B^4$ ist Wasserstoff, Alkyl von 1 bis 4 C-Atomen oder durch Alkoxy von 1 bis 4 C-Atomen, Sulfo, Carboxy, Sulfato, Phenyl, Sulfophenyl, Acetylamino, Benzoylamino, Cyano oder eine Gruppe der Formel $-SO_2-Y^1$ mit $Y^1$ obiger Bedeutung substituiertes Alkyl von 1 bis 4 C-Atomen oder ist Alkenyl von 2 bis 4 C-Atomen, Cyclohexyl, Phenyl oder durch Substituenten aus der Gruppe Carboxy, Sulfo, Benzoylamino, Acetylamino, $-SO_2-Y^1$ mit $Y^1$ obiger Bedeutung und Chlor substituiertes Phenyl.

**4.** Verfahren nach Anspruch 2, dadurch gekennzeichnet, daß K ein Rest der allgemeinen Formel (3c) oder (3i) ist, in welchen

$B^1$ Methyl, Carboxy oder Carbalkoxy von 2 bis 5 C-Atomen ist,

Q Phenyl ist, das durch Sulfo und/oder eine Gruppe der Formel $-SO_2-Y^1$ substituiert ist, worin $Y^1$ die in Anspruch 2 genannte Bedeutung besitzt, und bevorzugt $\beta$-Sulfatoethyl ist,

$R^6$ Wasserstoff, Sulfo, Methyl, Ethyl, Methoxy oder Ethoxy ist,

$R^7$ Wasserstoff, Methyl, Ethyl, Methoxy, Ethoxy, Chlor, Amino, substituiertes Amino, Alkanoylamino von 2 bis 5 C-Atomen, Benzoylamino oder Phenylsulfonylamino ist,

$R^8$ Wasserstoff, Methyl, Ethyl oder durch Hydroxy, Sulfo, Sulfato oder Carboxy substituiertes Alkyl von 2 bis 4 C-Atomen ist und

$R^9$ Wasserstoff, Methyl, Ethyl oder Alkyl von 2 bis 4 C-Atomen, das durch Hydroxy, Sulfo, Sulfato oder Carboxy substituiert ist, bedeutet.

**5.** Verfahren nach Anspruch 3, dadurch gekennzeichnet, daß K ein Rest der allgemeinen Formel (3e), (3k) oder (3m) ist, in welchen

R* Acetyl, Sulfobenzoyl oder Benzoyl ist,

R'' Wasserstoff oder Alkyl von 1 bis 4 C-Atomen ist,

m die Zahl 1 oder 2 ist,

M Wasserstoff oder ein Alkalimetall ist,

$R^{10}$ Wasserstoff oder bevorzugt Methyl ist,

$R^{11}$ Wasserstoff, Cyano, Sulfomethyl, Carbamoyl oder Carboxy ist,

$B^3$ Wasserstoff, Ethyl oder durch Sulfo, Phenyl oder Sulfophenyl substituiertes Alkyl von 2 bis 4 C-Atomen ist und

$B^4$ Wasserstoff, Methyl, Ethyl oder durch Sulfo, Phenyl oder Sulfophenyl substituiertes Alkyl von 2 bis 4 C-Atomen ist.

**6.** Verfahren nach Anspruch 3, dadurch gekennzeichnet, daß K ein Rest der allgemeinen Formel (3p) ist, in welcher

R* Acetyl, Sulfobenzoyl oder Benzoyl oder bevorzugt Wasserstoff ist,

R'' Alkyl von 1 bis 4 C-Atomen oder bevorzugt Wasserstoff ist,

m die Zahl 2 ist und

M Wasserstoff oder ein Alkalimetall ist.

**7.** Verfahren nach mindestens einem der Ansprüche 1 bis 6, dadurch gekennzeichnet, daß R Wasserstoff ist.

**8.** Verfahren nach mindestens einem der Ansprüche 1 bis 7, dadurch gekennzeichnet, daß X Chlor ist.

**9.** Verfahren nach mindestens einem der Ansprüche 1 bis 8, dadurch gekennzeichnet, daß Y Sulfato oder bevorzugt Chlor ist.

**10.** Verfahren zum Färben (einschließlich Bedrucken) von hydroxy- und/oder carbonamidgruppenhaltigem Material, insbesondere Fasermaterial, bei welchem man einen Farbstoff auf das Material aufbringt oder darin einbringt und ihn mittels Wärme und/oder mit Hilfe eines alkalisch wirkenden Mittels fixiert, dadurch gekennzeichnet, daß man als Farbstoff eine in Anspruch 1 genannte und definierte Verbindung der allgemeinen Formel (1) oder eine nach Anspruch 1 hergestellte Verbindung der allgemeinen Formel (1) einsetzt.

**11.** Verfahren zur Herstellung einer Verbindung entsprechend der allgemeinen Formel (7)

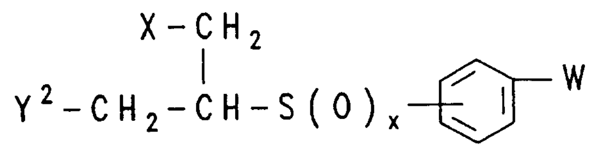
(7)

in welcher
X Chlor oder Brom ist,
x die Zahl Null oder 2 bedeutet,
W Nitro oder Amino ist und
$Y^2$ die Hydroxygruppe oder ein Substituent ist, der durch Alkali unter Bildung der Vinylgruppe eliminierbar ist,

dadurch gekennzeichnet; daß man ein Nitrobenzol-sulfenylhalogenid mit einer Verbindung der allgemeinen Formel (8)

$Y^2 - CH_2 - CH = CH_2$ (8)

in welcher $Y^2$ die oben genannte Bedeutung hat, zur Verbindung der Formel (7), in welcher $Y^2$ und X die oben genannten Bedeutungen haben, x die Zahl Null ist und W die Nitrogruppe bedeutet, umsetzt, und daß man im Falle der Herstellung einer Verbindung der allgemeinen Formel (7), in welcher Y2 und X die obengenannten Bedeutungen haben, x die Zahl 2 bedeutet und W für die Nitrogruppe steht, die Verbindung entsprechend der allgemeinen Formel (7), in welcher $Y^2$ und x die obengenannten Bedeutungen haben, x die Zahl Null ist und W die Nitrogruppe bedeutet, mit einem Oxidationsmittel umsetzt, und daß man im Falle der Herstellung einer Verbindung der allgemeinen Formel (7), in welcher $Y^2$ und X die obengenannten Bedeutungen haben, x für die Zahl 2 steht und W die Aminogruppe ist, in der Verbindung der Formel (7), in welcher Y2 und X die obengenannten Bedeutungen haben, x die Zahl 2 bedeutet und W die Nitrogruppe ist, diese Nitrogruppe reduziert.

**12.** Verfahren nach Anspruch 11, dadurch gekennzeichnet, daß die Verbindung der Formel (7) eine Verbindung der allgemeinen Formel (4)

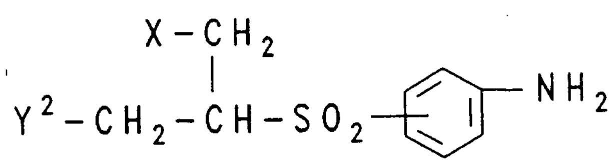
(4)

ist, in welcher x und $Y^2$ die in Anspruch 11 genannte Bedeutung haben.

**13.** Verbindung nach Anspruch 11 oder 12, dadurch gekennzeichnet, daß X Chlor ist.

**14.** Verbindung nach mindestens einem der Ansprüche 11 bis 13, dadurch gekennzeichnet, daß $Y^2$ Chlor, Brom, Cyano, Alkanoyloxy von 2 bis 5 C-Atomen oder Sulfato oder bevorzugt Chlor, Brom, Cyano oder Acetyloxy ist.

**Claims**

**Claims for the following Contracting States : DE, GB, FR, IT, CH, LI**

1. A water soluble azo compound conforming to the formula (1)

   D - N = N - K (1)

   where

   D is a radical of the formula (2)

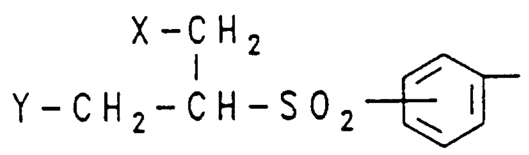

(2)

where

Y is a substituent which is eliminable by alkali to form a vinyl group, the sulfonyl group preferably being attached to the benzene nucleus meta or para to the free bond, and

X is chlorine or bromine;

K is a radical of a singly couplable, preferably water soluble, coupling component, which may additionally contain an azo group, or the radical of a doubly couplable, preferably water soluble, coupling component, each selected from the series of the aminobenzenes, the phenols, the naphthols, the aminonaphthols or the acylaminonaphthols, having the acyl radical of an alkane- or alkenecarboxylic acid having from 1 to 4 or from 2 to 4 carbon atoms in the alkyl or alkenyl radical respectively or of an aromatic carboxylic acid or of an aromatic sulfonic acid or of an N-substituted carbamic acid, or from the series of the dihydroxynaphthalenesulfonic acids, the phenylazo- and naphthylazo-aminonaphtholsulfonic acids, the 5-pyrazolones and 5-aminopyrazoles, the acetoacetylarylides, the 2-hydroxy-6-pyridones and the hydroxyquinolines, and K may in addition to the substituents customary in dyes also contain a fiber reactive group of the formula $-SO_2-Y^1$, where $Y^1$ is vinyl or a group of the formula $-CH_2-CH_2-Y$, where Y has one of the above meanings.

2. An azo compound as claimed in claim 1, wherein K is a radical of the formula (3c), (3f), (3h), (3i), (3p) or (3q)

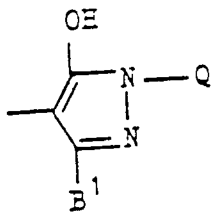

(3c)

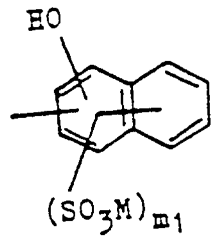

(3f)

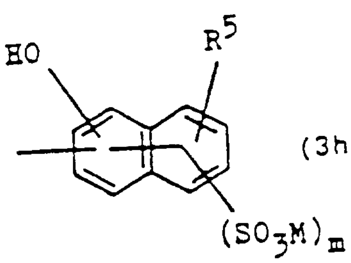

(3h)

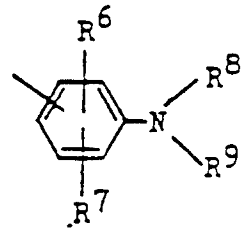

(3i)

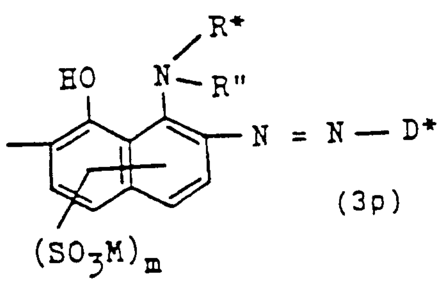

(3p)

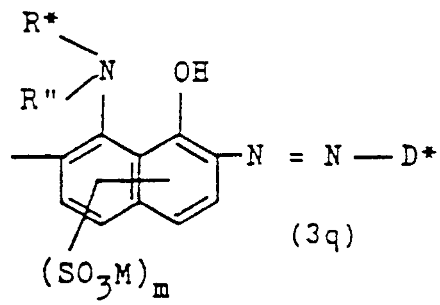

(3q)

where

M is hydrogen or an alkali metal;

$B^1$ is alkyl of from 1 to 4 carbon atoms, carboxyl, carbalkoxy of from 2 to 5 carbon atoms, carbamoyl, phenyl or phenyl which is substituted by 1 or 2 substituents selected from the group consisting of sulfo, carboxyl, methyl, ethyl, methoxy, ethoxy and chlorine;

Q is phenyl which may be substituted by 1, 2 or 3 substituents selected from the group consisting of chlorine, bromine, methyl, ethyl, methoxy, ethoxy, carboxyl, sulfo and alkanoylamino of from 2 to 5 carbon atoms and/or by a group of the formula $-SO_2-Y^1$, where $Y^1$ is vinyl or an ethyl group which contains an alkali eliminable substituent in the $\beta$-position;

$R^*$ is hydrogen or alkyl of from 1 to 4 carbon atoms, which may be substituted by phenyl or phenyl which is substituted by sulfo and/or a group of the formula $-SO_2-Y^1$, where $Y^1$ is as defined above, or is alkanoyl of from 2 to 5 carbon atoms, substituted or unsubstituted phenylsulfonyl or substituted or unsubstituted benzoyl;

R'' is hydrogen or alkyl of from 1 to 4 carbon atoms, which may be substituted by phenyl, sulfophenyl or a group of the formula $-SO_2-Y^1$, where $Y^1$ is as defined above, or is phenyl or phenyl which is substituted by 1 or 2 substituents selected from the group consisting of alkyl of from 1 to 4 carbon atoms, alkoxy of from 1 to 4 carbon atoms, chlorine, bromine, sulfo and $-SO_2-Y^1$, where $Y^1$ is as defined above;

$R^5$ is phenylureido, whose phenyl radical may be substituted by a group of the formula $-SO_2-Y^1$, where $Y^1$ is as defined above, or is alkanoylamino of from 2 to 5 carbon atoms, which may be substituted in the alkyl radical by a group of the formula $-SO_2-$

$Y^1$, where $Y^1$ is as defined above, or is alkenoylamino of from 3 to 5 carbon atoms or is benzoylamino, which may be substituted by substituents selected from the group consisting of chlorine, methyl, methoxy, nitro, sulfo, carboxyl and $-SO_2-Y^1$, where $Y^1$ is as defined above;

$R^6$ is hydrogen, alkyl of from 1 to 4 carbon atoms, alkoxy of from 1 to 4 carbon atoms, sulfo, carboxyl, carbalkoxy of from 2 to 5 carbon atoms, halogen or alkoxy of from 1 to 4 carbon atoms which is substituted by hydroxyl, acetyloxy, carboxyl, carbamoyl, cyano or halogen;

$R^7$ is hydrogen, alkyl of from 1 to 4 carbon atoms, alkoxy of from 1 to 4 carbon atoms, halogen, cyano, trifluoromethyl, alkoxy of from 1 to 4 carbon atoms, which is substituted by hydroxyl, acetyloxy, carboxyl, carbamoyl, cyano or halogen or by a group of the formula $-SO_2-Y^1$, where $Y^1$ is as defined above, or is alkanoylamino of from 2 to 5 carbon atoms, which may be substituted by chlorine, bromine, alkoxy of from 1 to 4 carbon atoms, phenoxy, phenyl, hydroxyl, carboxyl or sulfo or a group of the formula $-SO_2-Y^1$, where $Y^1$ is as defined above, or is alkenoylamino of from 3 to 5 carbon atoms, which may be substituted by chlorine, bromine, carboxyl or sulfo, or is benzoylamino, which may be substituted in the benzene nucleus by substituents selected from the group consisting of chlorine, methyl, sulfo and a group of the formula $-SO_2-Y^1$, where $Y^1$ is as defined above, or is alkylsulfonyl of from 1 to 4 carbon atoms or phenylsulfonyl, which may be substituted in the benzene nucleus by substituents selected from the group consisting of chlorine, methyl, sulfo and a group of the formula $-SO_2Y^1$, where $Y^1$ is as defined above, or is alkylsulfonylamino of from 1 to 4 carbon atoms, which may be substituted by hydroxyl, sulfato, chlorine, bromine, alkoxy of from 1 to 4 carbon atoms or a group of the formula $-SO_2-Y^1$, where $Y^1$ is as defined above, or is phenylsulfonylamino, which may be substituted in the benzene nucleus by substituents selected from the group consisting of chlorine, methyl, sulfo and a group of the formula $-SO_2-Y^1$, where $Y^1$ is as defined above, or is carbamoyl, which may be mono- or disubstituted on the nitrogen atom by 1 or 2 substituents, which substituents belong to the group consisting of alkyl of from 1 to 4 carbon atoms, alkyl of from 1 to 4 carbon atoms which is substituted by hydroxyl, sulfo, carboxyl, sulfato, phenyl or a group of the formula $-SO_2-Y^1$, where $Y^1$ is as defined above, cycloalkyl of from 5 to 8 carbon atoms, phenyl and phenyl which is substituted by substituents selected from the group consisting of chlorine, sulfo, methyl, methoxy, carboxyl and a group of the formula $-SO_2-Y^1$, where $Y^1$ is as defined above, or is sulfamoyl, which may be mono- or disubstituted on the nitrogen atom by 1 or 2 substituents, which substituents belong to the group consisting of alkyl of from 1 to 4 carbon atoms, alkyl of from 1 to 4 carbon atoms which is substituted by hydroxyl, sulfo, carboxyl, sulfato, phenyl or a group of the formula $-SO_2-Y^1$, where $Y^1$ is as defined above, cycloalkyl of from 5 to 8 carbon atoms, phenyl and phenyl which is substituted by substituents selected from the group consisting of chlorine, sulfo, methyl, methoxy, carboxyl and a group of the formula $-SO_2-Y^1$, where $Y^1$ is as defined above, or is ureido or ureido which may be mono- or disubstituted on the terminal nitrogen atom by 1 or 2 substituents, which substituents belong to the group consisting of alkyl of from 1 to 4 carbon atoms, alkyl of from 1 to 4 carbon atoms which is substituted by hydroxyl, sulfo, carboxyl, sulfato, phenyl or a group of the formula $-SO_2-Y^1$, where $Y^1$ is as defined above, cycloalkyl of from 5 to 8 carbon atoms, phenyl and phenyl which is substituted by substituents selected from the group consisting of chlorine, sulfo, methyl, methoxy, carboxyl and a group of the formula $-SO_2-Y^1$, where $Y^1$ is as defined above;

$R^8$ is hydrogen or alkyl of from 1 to 4 carbon atoms, which may be substituted by hydroxyl, sulfo, carboxyl, sulfato, a group $-SO_2-Y^1$, where $Y^1$ is as defined above, phenyl or sulfophenyl, or is alkenyl of from 2 to 4 carbon atoms, which may be substituted by carboxyl, sulfo, chlorine or bromine, or is cycloalkyl of from 5 to 8 carbon atoms;

$R^9$ is hydrogen or alkyl of from 1 to 4 carbon atoms, which may be substituted by hydroxyl, sulfo, carboxyl, sulfato, phenyl or $-SO_2-Y^1$, where $Y^1$ is as defined above, or is alkenyl of from 2 to 5 carbon atoms, which may be substituted by carboxyl, sulfo or $-SO_2-Y^1$, where $Y^1$ is as defined above, or by chlorine or bromine, or

$R^9$ is cycloalkyl of from 5 to 8 carbon atoms or phenyl, which may be substituted by substituents selected from the group consisting of chlorine, sulfo, methyl, methoxy, carboxyl and $-SO_2-Y^1$, where $Y^1$ is as defined above, or is naphthyl which is substituted by 1, 2 or 3 sulfo groups or by 1 or 2 sulfo groups and 1 or 2 groups of the formula $-SO_2-Y^1$, where $Y^1$ is as defined above, or only by one such group $-SO_2-Y^1$, or

$R^8$ and $R^9$ together with the nitrogen atom are N-piperidino, N-morpholino or N-piperazino;

m is 1 or 2;

$m_1$ is 1, 2 or 3;

D* is a group of the formula (2), (2a) or (2b) as defined in claim 1 or is phenyl which may be substituted by 1, 2 or 3 substituents selected from the group consisting of alkyl of from 1 to 4 carbon atoms, alkoxy of from 1 to 4 carbon atoms, chlorine, bromine, hydroxyl, carboxyl, sulfo, carbamoyl, sulfamoyl and alkanoylamino of from 2 to 5 carbon atoms and/or by a group of the formula $-SO_2-Y^1$, where $Y^1$ is as defined above, one of these substituents preferably being a sulfo or carboxyl group and the group $-SO_2-Y^1$ preferably being meta or para to the azo group, or D* is naphthyl which is substituted by 1, 2 or 3 sulfo groups or by 1 or 2 sulfo groups and 1 or 2 groups of the formula $-SO_2-Y^1$, where $Y^1$ is as defined above, or only by one such group $-SO_2-Y^1$,

it being possible for D and D* to have meanings identical to or different from each other.

**3.** An azo compound as claimed in claim 1, wherein K is a radical of the formula (3d), (3e), (3k) or (3m)

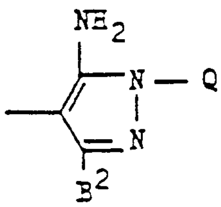

(3d)

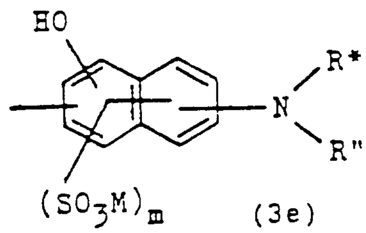

(3e)

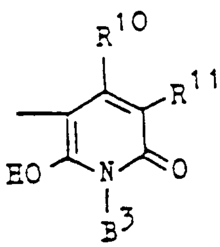

(3k)

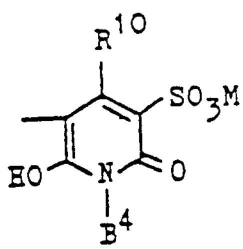

(3m)

where

M is hydrogen or an alkali metal;

$B^2$ is alkyl of from 1 to 4 carbon atoms, carbalkoxy of from 2 to 5 carbon atoms, carbamoyl, phenyl or phenyl which is substituted by 1 or 2 substituents selected from the group consisting of alkyl of from 1 to 4 carbon atoms, alkoxy of from 1 to 4 carbon atoms, chlorine, bromine and sulfo;

Q is phenyl which may be substituted by 1, 2 or 3 substituents selected from the group consisting of chlorine, bromine, methyl, ethyl, methoxy, ethoxy, carboxyl, sulfo and alkanoylamino of from 2 to 5 carbon atoms and/or by a group of the formula $-SO_2-Y^1$, where $Y^1$ is vinyl or an ethyl group which contains an alkali eliminable substituent in the $\beta$-position;

R* is hydrogen or alkyl of from 1 to 4 carbon atoms, which may be substituted by phenyl or phenyl which is substituted by sulfo and/or a group of the formula $-SO_2-Y^1$, where $Y^1$ is as defined above, or is alkanoyl of from 2 to 5 carbon atoms, substituted or unsubstituted phenylsulfonyl or substituted or unsubstituted benzoyl;

R'' is hydrogen or alkyl of from 1 to 4 carbon atoms, which may be substituted by phenyl, sulfophenyl or a group of the formula $-SO_2-Y^1$, where $Y^1$ is as defined above, or is phenyl or phenyl which is substituted by 1 or 2 substituents selected from the group consisting of alkyl of from 1 to 4 carbon atoms, alkoxy of from 1 to 4 carbon atoms, chlorine, bromine, sulfo and $-SO_2-Y^1$, where $Y^1$ is as defined above;

m is 1 or 2;

$B^3$ is hydrogen or alkyl of from 1 to 4 carbon atoms, which may be substituted by phenyl, sulfo, sulfophenyl or a group $-SO_2-Y^1$, where $Y^1$ is as defined in claim 1;

$R^{10}$ is hydrogen or alkyl of from 1 to 4 carbon atoms or alkyl of from 1 to 4 carbon atoms which is substituted by alkoxy of from 1 to 4 carbon atoms or cyano;

$R^{11}$ is hydrogen, carboxyl, sulfo, sulfoalkyl having an alkylene radical of from 1 to 4 carbon atoms, cyano or carbamoyl;

$B^4$ is hydrogen, alkyl of from 1 to 4 carbon atoms or alkyl of from 1 to 4 carbon atoms which is substituted by alkoxy of from 1 to 4 carbon atoms, sulfo, carboxyl, sulfato, phenyl, sulfophenyl, acetylamino, benzoylamino, cyano or a group of the formula $-SO_2-Y^1$, where $Y^1$ is as defined above, or is alkenyl of from 2 to 4 carbon atoms, cyclohexyl, phenyl or phenyl which is substituted by substituents selected from the group consisting of carboxyl, sulfo, benzoylamino, acetylamino, $-SO_2-Y^1$, where $Y^1$ is as defined above, and chlorine.

**4.** An azo compound as claimed in claim 2, wherein K is a radical of the formula (3c) or (3i) where

$B^1$ is methyl, carboxyl or carbalkoxy of from 2 to 5 carbon atoms,

Q is phenyl which is substituted by sulfo and/or a group of the formula $-SO_2-Y^1$, where $Y^1$ is as defined in claim 2, but preferably is $\beta$-sulfatoethyl,

$R^6$ is hydrogen, sulfo, methyl, ethyl, methoxy or ethoxy,

$R^7$ is hydrogen, methyl, ethyl, methoxy, ethoxy, chlorine, amino, substituted amino, alkanoylamino of from 2 to 5 carbon atoms, benzoylamino or phenylsulfonylamino,

$R^8$ is hydrogen, methyl, ethyl or hydroxyl-, sulfo-, sulfato- or carboxyl-substituted alkyl of from 2 to 4 carbon atoms, and

$R^9$ is hydrogen, methyl, ethyl or alkyl of from 2 to 4 carbon atoms, which is substituted by hydroxyl, sulfo, sulfato or carboxyl.

**5.** An azo compound as claimed in claim 3, wherein K is a radical of the formula (3e), (3k) or (3m) where

R* is acetyl, sulfobenzoyl or benzoyl,

R'' is hydrogen or alkyl of from 1 to 4 carbon atoms,

m is 1 or 2,

M is hydrogen or an alkali metal,

$R^{10}$ is hydrogen or preferably methyl,

$R^{11}$ is hydrogen, cyano, sulfomethyl, carbamoyl or carboxyl,

$B^3$ is hydrogen, ethyl or sulfo-, phenyl- or sulfophenyl-substituted alkyl of from 2 to 4 carbon atoms, and

$B^4$ is hydrogen, methyl, ethyl or sulfo-, phenyl- or sulfophenyl-substituted alkyl of from 2 to 4 carbon atoms.

**6.** An azo compound as claimed in claim 3, wherein K is a radical of the formula (3p) where

R* is acetyl, sulfobenzoyl or benzoyl or preferably hydrogen,

R'' is alkyl of from 1 to 4 carbon atoms or preferably hydrogen,

m is 2, and

M is hydrogen or an alkali metal.

**7.** An azo compound as claimed in at least one of claims 1 to 6, wherein X is chlorine.

**8.** An azo compound as claimed in at least one of claims 1 to 7, wherein Y is sulfato or preferably chlorine.

**9.** A process for preparing an azo compound of the formula (1) as defined in claim 1, which comprises reacting a diazonium compound of an amino compound of the formula (4)

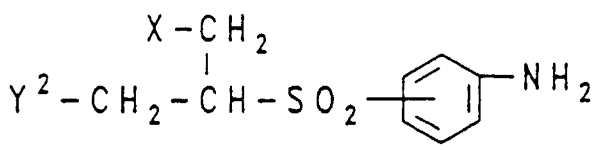

(4)

where X is as defined in claim 1 and $Y^2$ is hydroxyl or has one of the meanings of Y, with a coupling component of the formula H-K, where K is as defined in claim 1, or coupling in the case of the preparation of a disazo compound conforming to the formula (1) where K is a radical of the formula (3q) a monoazo compound of the formula

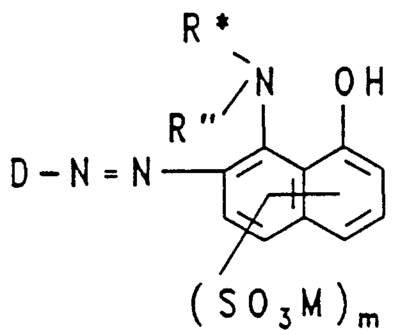

where D, R*, R'', M and m are each as defined in claim 1 or claim 2, with a diazonium compound of an amine of the formula $D^*$-$NH_2$, where D* is as defined in claim 2, and converting in the case of use of a compound of the formula (4) where $Y^2$ is hydroxyl in the resulting azo compound the hydroxyl group $Y^2$ into a group Y of the formula (1).

**10.** The use of a compound conforming to the formula (1) of claim 1 for dyeing (including printing) hydroxyland/or carboxamido-containing material, in particular fiber material.

**11.** A process for dyeing (including printing) hydroxyl- and/or carboxamido-containing material, in particular fiber material, by introducing a dye to or into the material and fixing it by means of heat and/or with the aid of an alkali agent, which comprises using as the dye a compound conforming to the formula (1) of claim 1.

**12.** A compound conforming to the formula (7)

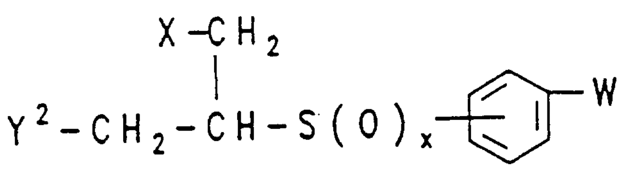

(7)

where

X is chlorine or bromine,

x is zero or 2,

W is nitro or amino, and

$Y^2$ is hydroxyl or a substituent which is eliminable by means of alkali to form a vinyl group.

**13.** A compound as claimed in claim 12 conforming to the formula (4)

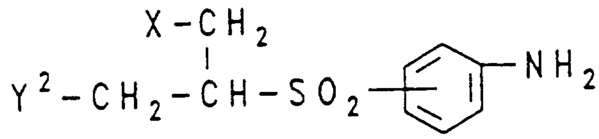

(4)

where X and $Y^2$ are each as defined in claim 12.

**14.** A compound as claimed in claim 12 or 13, wherein X is chlorine.

**15.** A compound as claimed in at least one of claims 12 to 14, wherein $Y^2$ is chlorine, bromine, cyano, alkanoyloxy of from 2 to 5 carbon atoms or sulfato or preferably chlorine, bromine, cyano or acetyloxy.

**16.** A process for preparing a compound conforming to the formula (7) mentioned and defined in claim 12, which comprises reacting a nitrobenzenesulfenyl halide with a compound of the formula (8)

$Y^2 - CH_2 - CH = CH_2$ (8)

where $Y^2$ is as defined in claim 12, to form the compound of the formula (7), where $Y^2$ and X are each as defined in claim 12, x is zero and W is nitro, and in the case of the preparation of a compound of the formula (7) where $Y^2$ and X are each as defined above, x is 2 and W is nitro, reacting the compound conforming to the formula (7) where $Y^2$ and X are each as defined above, x is zero and W is nitro with an oxidizing agent, and in the case of the preparation of a compound of the formula (7) where $Y^2$ and X are each as defined above, x is 2 and W is amino, reducing, in the compound of the formula (7) where $Y^2$ and X are each as defined above, x is 2 and W is nitro, this nitro group.

**17.** The use of a compound of the formula (7) of claim 12, where $Y^2$ and X are each as defined in claim 12, x is 2 and W is amino, as a diazo component for synthesizing azo compounds.

**Claims for the following Contracting State : ES**

**1.** A process for preparing a water soluble azo compound conforming to the formula (1)

D - N = N - K (1)

where

D is a radical of the formula (2)

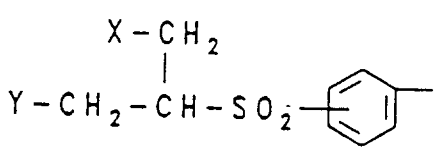

(2)

where

Y is a substituent which is eliminable by alkali to form a vinyl group, the sulfonyl group preferably being attached to the benzene nucleus meta or para to the free bond, and

X is chlorine or bromine;

K is a radical of a singly couplable, preferably water soluble, coupling component, which may additionally contain an azo group, or the radical of a doubly couplable, preferably water soluble, coupling component, each selected from the series of the aminobenzenes, the phenols, the naphthols, the aminonaphthols or the acylaminonaphthols, having the acyl radical of an alkane- or alkene-carboxylic acid having from 1 to 4 or from 2 to 4 carbon atoms in the alkyl or alkenyl radical respectively or of an aromatic carboxylic acid or of an aromatic sulfonic acid or of an N-substituted carbamic acid, or from the series of the dihydroxynaphthalenesulfonic acids, the phenylazo- and naphthylazo-amino-naphtholsulfonic acids, the 5-pyrazolones and 5-aminopyrazoles, the acetoacetylarylides, the 2-hydroxy-6-pyridones and the hydroxyquinolines, and K may in addition to the substituents customary in dyes also contain a fiber reactive group of the formula $-SO_2-Y^1$, where $Y^1$ is vinyl or a group of the formula $-CH_2-CH_2-Y$, where Y has one of the above meanings,

which comprises reacting a diazonium compound of an amino compound of the formula (4)

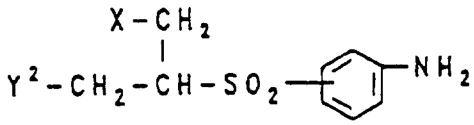

(4)

where X is as defined above and $Y^2$ is hydroxyl or has one of the meanings of Y, with a coupling component of the formula H-K, where K is as defined above, or in the case of the preparation of a disazo compound conforming to the formula (1) where K is a radical of the formula (3q)

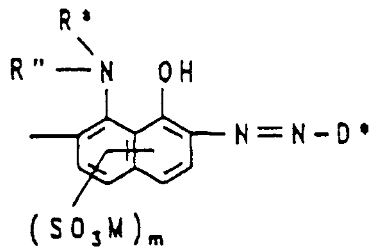

(3q)

where

- M is hydrogen or an alkali metal;
- m is 1 or 2;
- R* is hydrogen or alkyl of from 1 to 4 carbon atoms, which may be substituted by phenyl or phenyl which is substituted by sulfo and/or a group of the formula $-SO_2-Y^1$, where $Y^1$ is as defined above, or is alkanoyl of from 2 to 5 carbon atoms, substituted or unsubstituted phenylsulfonyl or substituted or unsubstituted benzoyl;
- R" is hydrogen or alkyl of from 1 to 4 carbon atoms, which may be substituted by phenyl, sulfophenyl or a group of the formula $-SO_2-Y^1$, where $Y^1$ is as defined above, or is phenyl or phenyl which is substituted by 1 or 2 substituents selected from the group consisting of alkyl of from 1 to 4 carbon atoms, alkoxy of from 1 to 4 carbon atoms, chlorine, bromine, sulfo and $-SO_2-Y^1$, where $Y^1$ is as defined above; and
- D* is a group of the formula (2) or is phenyl which may be substituted by 1, 2 or 3 substituents selected from the group consisting of alkyl of from 1 to 4 carbon atoms, alkoxy of from 1 to 4 carbon atoms, chlorine, bromine, hydroxyl, carboxyl, sulfo, carbamoyl, sulfamoyl and alkanoylamino of from 2 to 5 carbon atoms and/or by a group of the formula $-SO_2-Y^1$, where $Y^1$ is as defined above, one of these substituents preferably being a sulfo or carboxyl group and the $-SO_2-Y^1$ group preferably being meta or para to the azo group, or D* is naphthyl which is substituted by 1, 2 or 3 sulfo groups or by 1 or 2 sulfo groups and 1 or 2 groups of the formula $-SO_2-Y^1$, where $Y^1$ is as defined above, or only by one such group $-SO_2-Y^1$,

it being possible for D and D* to have meanings identical to or different from each other, a coupling monoazo compound of the formula

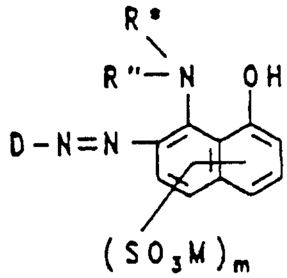

where D, R*, R'', M and m are each as defined above, with a diazonium compound of an amine of the formula $D^*\text{-}NH_2$ where D* has the abovementioned meaning, and in the case of the use of a compound of the formula (4) where $Y^2$ is a hydroxyl group converting the hydroxyl group $Y^2$ in the azo compound obtained into a group Y as per the formula (1).

**2.** The process as claimed in claim 1, wherein K is a radical of the formula (3c), (3f), (3h), (3i), (3p) or (3q)

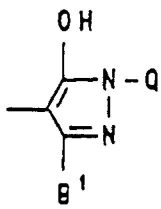
(3c)

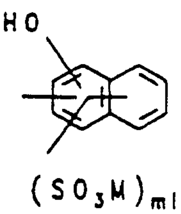
(3f)

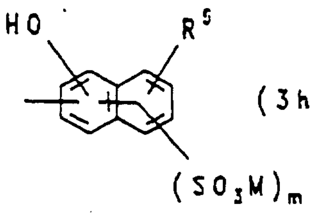
(3h)

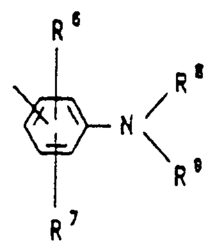
(3i)

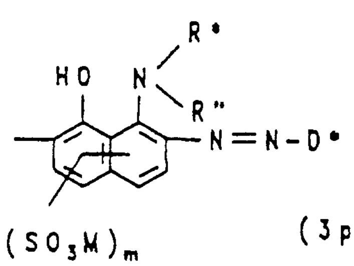
(3p)

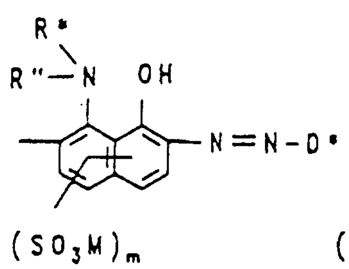
(3q)

where

| | |
|---|---|
| M | is hydrogen or an alkali metal; |
| $B^1$ | is alkyl of from 1 to 4 carbon atoms, carboxyl, carbalkoxy of from 2 to 5 carbon atoms, carbamoyl, phenyl or phenyl which is substituted by 1 or 2 substituents selected from the group consisting of sulfo, carboxyl, methyl, ethyl, methoxy, ethoxy and chlorine; |
| Q | is phenyl which may be substituted by 1, 2 or 3 substituents selected from the group consisting of chlorine, bromine, methyl, ethyl, methoxy, ethoxy, carboxyl, sulfo and alkanoylamino of from 2 to 5 carbon atoms and/or by a group of the formula $-SO_2-Y^1$, where $Y^1$ is vinyl or an ethyl group which contains an alkali eliminable substituent in the $\beta$-position; |

R* is hydrogen or alkyl of from 1 to 4 carbon atoms, which may be substituted by phenyl or phenyl which is substituted by sulfo and/or a group of the formula $-SO_2-Y^1$, where $Y^1$ is as defined above, or is alkanoyl of from 2 to 5 carbon atoms, substituted or unsubstituted phenylsulfonyl or substituted or unsubstituted benzoyl;

R'' is hydrogen or alkyl of from 1 to 4 carbon atoms, which may be substituted by phenyl, sulfophenyl or a group of the formula $-SO_2-Y^1$, where $Y^1$ is as defined above, or is phenyl or phenyl which is substituted by 1 or 2 substituents selected from the group consisting of alkyl of from 1 to 4 carbon atoms, alkoxy of from 1 to 4 carbon atoms, chlorine, bromine, sulfo and $-SO_2-Y^1$, where $Y^1$ is as defined above;

$R^5$ is phenylureido, whose phenyl radical may be substituted by a group of the formula $-SO_2-Y^1$, where $Y^1$ is as defined above, or is alkanoylamino of from 2 to 5 carbon atoms, which may be substituted in the alkyl radical by a group of the formula $-SO_2-Y^1$, where $Y^1$ is as defined above, or is alkenoylamino of from 3 to 5 carbon atoms or is benzoylamino, which may be substituted by substituents selected from the group consisting of chlorine, methyl, methoxy, nitro, sulfo, carboxyl and $-SO_2-Y^1$, where $Y^1$ is as defined above;

$R^6$ is hydrogen, alkyl of from 1 to 4 carbon atoms, alkoxy of from 1 to 4 carbon atoms, sulfo, carboxyl, carbalkoxy of from 2 to 5 carbon atoms, halogen or alkoxy of from 1 to 4 carbon atoms which is substituted by hydroxyl, acetyloxy, carboxyl, carbamoyl, cyano or halogen;

$R^7$ is hydrogen, alkyl of from 1 to 4 carbon atoms, alkoxy of from 1 to 4 carbon atoms, halogen, cyano, trifluoromethyl, alkoxy of from 1 to 4 carbon atoms, which is substituted by hydroxyl, acetyloxy, carboxyl, carbamoyl, cyano or halogen or by a group of the formula $-SO_2-Y^1$, where $Y^1$ is as defined above, or is alkanoylamino of from 2 to 5 carbon atoms, which may be substituted by chlorine, bromine, alkoxy of from 1 to 4 carbon atoms, phenoxy, phenyl, hydroxyl, carboxyl or sulfo or a group of the formula $-SO_2-Y^1$, where $Y^1$ is as defined above, or is alkenoylamino of from 3 to 5 carbon atoms, which may be substituted by chlorine, bromine, carboxyl or sulfo, or is benzoylamino, which may be substituted in the benzene nucleus by substituents selected from the group consisting of chlorine, methyl, sulfo and a group of the formula $-SO_2-Y^1$, where $Y^1$ is as defined above, or is alkylsulfonyl of from 1 to 4 carbon atoms or phenylsulfonyl, which may be substituted in the benzene nucleus by substituents selected from the group consisting of chlorine, methyl, sulfo and a group of the formula $-SO_2-Y^1$, where $Y^1$ is as defined above, or is alkylsulfonylamino of from 1 to 4 carbon atoms, which may be substituted by hydroxyl, sulfato, chlorine, bromine, alkoxy of from 1 to 4 carbon atoms or a group of the formula $-SO_2-Y^1$, where $Y^1$ is as defined above, or is phenylsulfonylamino, which may be substituted in the benzene nucleus by substituents selected from the group consisting of chlorine, methyl, sulfo and a group of the formula $-SO_2-Y^1$, where $Y^1$ is as defined above, or is carbamoyl, which may be mono- or disubstituted on the nitrogen atom by 1 or 2 substituents, which substituents belong to the group consisting of alkyl of from 1 to 4 carbon atoms, alkyl of from 1 to 4 carbon atoms which is substituted by hydroxyl, sulfo, carboxyl, sulfato, phenyl or a group of the formula $-SO_2-Y^1$, where $Y^1$ is as defined above, cycloalkyl of from 5 to 8 carbon atoms, phenyl and phenyl which is substituted by substituents selected from the group consisting of chlorine, sulfo, methyl, methoxy, carboxyl and a group of the formula $-SO_2-Y^1$, where $Y^1$ is as defined above, or is sulfamoyl, which may be mono- or disubstituted on the nitrogen atom by 1 or 2 substituents, which substituents belong to the group consisting of alkyl of from 1 to 4 carbon atoms, alkyl of from 1 to 4 carbon atoms which is substituted by hydroxyl, sulfo, carboxyl, sulfato, phenyl or a group of the formula $-SO_2-Y^1$, where $Y^1$ is as defined above, cycloalkyl of from 5 to 8 carbon atoms, phenyl and phenyl which is substituted by substituents selected from the group consisting of chlorine, sulfo, methyl, methoxy, carboxyl and a group of the formula $-SO_2-Y^1$, where $Y^1$ is as defined above, or is ureido or ureido which may be mono- or disubstituted on the terminal nitrogen atom by 1 or 2 substituents, which substituents belong to the group consisting of alkyl of from 1 to 4 carbon atoms, alkyl of from 1 to 4 carbon atoms which is substituted by hydroxyl, sulfo, carboxyl, sulfato, phenyl or a group of the formula $-SO_2-Y^1$, where $Y^1$ is as defined above, cycloalkyl of from 5 to 8 carbon

atoms, phenyl and phenyl which is substituted by substituents selected from the group consisting of chlorine, sulfo, methyl, methoxy, carboxyl and a group of the formula $-SO_2-Y^1$, where $Y^1$ is as defined above;

$R^8$ is hydrogen or alkyl of from 1 to 4 carbon atoms, which may be substituted by hydroxyl, sulfo, carboxyl, sulfato, a group $-SO_2-Y^1$, where $Y^1$ is as defined above, phenyl or sulfophenyl, or is alkenyl of from 2 to 4 carbon atoms, which may be substituted by carboxyl, sulfo, chlorine or bromine, or is cycloalkyl of from 5 to 8 carbon atoms;

$R^9$ is hydrogen or alkyl of from 1 to 4 carbon atoms, which may be substituted by hydroxyl, sulfo, carboxyl, sulfato, phenyl or $-SO_2-Y^1$, where $Y^1$ is as defined above, or is alkenyl of from 2 to 5 carbon atoms, which may be substituted by carboxyl, sulfo or $-SO_2-Y^1$, where $Y^1$ is as defined above, or by chlorine or bromine, or

$R^9$ is cycloalkyl of from 5 to 8 carbon atoms or phenyl, which may be substituted by substituents selected from the group consisting of chlorine, sulfo, methyl, methoxy, carboxyl and $-SO_2-Y^1$, where $Y^1$ is as defined above, or is naphthyl which is substituted by 1, 2 or 3 sulfo groups or by 1 or 2 sulfo groups and 1 or 2 groups of the formula $-SO_2-Y^1$, where $Y^1$ is as defined above, or only by one such group $-SO_2-Y^1$, or

$R^8$ and $R^9$ together with the nitrogen atom are N-piperidino, N-morpholino or N-piperazino;

m is 1 or 2;

$m_1$ is 1, 2 or 3;

D* is a group of the formula (2) as defined in claim 1 or is phenyl which may be substituted by 1, 2 or 3 substituents selected from the group consisting of alkyl of from 1 to 4 carbon atoms, alkoxy of from 1 to 4 carbon atoms, chlorine, bromine, hydroxyl, carboxyl, sulfo, carbamoyl, sulfamoyl and alkanoylamino of from 2 to 5 carbon atoms and/or by a group of the formula $-SO_2-Y^1$, where $Y^1$ is as defined above, one of these substituents preferably being a sulfo or carboxyl group and the group $-SO_2-Y^1$ preferably being meta or para to the azo group, or D* is naphthyl which is substituted by 1, 2 or 3 sulfo groups or by 1 or 2 sulfo groups and 1 or 2 groups of the formula $-SO_2-Y^1$, where $Y^1$ is as defined above, or only by one such group $-SO_2-Y^1$,

it being possible for D and D* to have meanings identical to or different from each other.

**3.** The process as claimed in claim 1, wherein K is a radical of the formula (3d), (3e), (3k) or (3m)

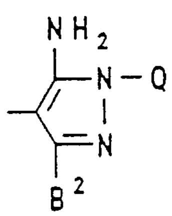

(3d)

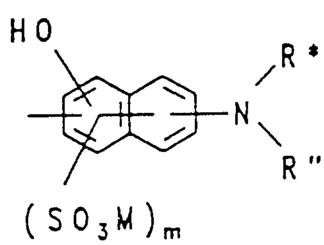

(3e)

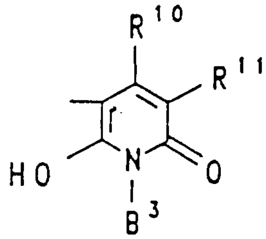

(3k)

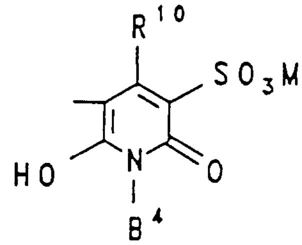

(3m)

where

M is hydrogen or an alkali metal;

$B^2$ is alkyl of from 1 to 4 carbon atoms, carbalkoxy of from 2 to 5 carbon atoms, carbamoyl, phenyl or phenyl which is substituted by 1 or 2 substituents selected from the group consisting of alkyl of from 1 to 4 carbon atoms, alkoxy of from 1 to 4 carbon atoms, chlorine, bromine and sulfo;

Q is phenyl which may be substituted by 1, 2 or 3 substituents selected from the group consisting of chlorine, bromine, methyl, ethyl, methoxy, ethoxy, carboxyl, sulfo and alkanoylamino of from 2 to 5 carbon atoms and/or by a group of the formula $-SO_2-Y^1$, where $Y^1$ is vinyl or an ethyl group which contains an alkali eliminable substituent in the $\beta$-position;

R* is hydrogen or alkyl of from 1 to 4 carbon atoms, which may be substituted by phenyl or phenyl which is substituted by sulfo and/or a group of the formula $-SO_2-Y^1$, where $Y^1$ is as defined above, or is alkanoyl of from 2 to 5 carbon atoms, substituted or unsubstituted phenylsulfonyl or substituted or unsubstituted benzoyl;

R'' is hydrogen or alkyl of from 1 to 4 carbon atoms, which may be substituted by phenyl, sulfophenyl or a group of the formula $-SO_2-Y^1$, where $Y^1$ is as defined above, or is phenyl or phenyl which is substituted by 1 or 2 substituents selected from the group consisting of alkyl of from 1 to 4 carbon atoms, alkoxy of from 1 to 4 carbon atoms, chlorine, bromine, sulfo and $-SO_2-Y^1$, where $Y^1$ is as defined above;

m is 1 or 2;

$B^3$ is hydrogen or alkyl of from 1 to 4 carbon atoms, which may be substituted by phenyl, sulfo, sulfophenyl or a group $-SO_2-Y^1$, where $Y^1$ is as defined in claim 1;

$R^{10}$ is hydrogen or alkyl of from 1 to 4 carbon atoms or alkyl of from 1 to 4 carbon atoms which is substituted by alkoxy of from 1 to 4 carbon atoms or cyano;

$R^{11}$ is hydrogen, carboxyl, sulfo, sulfoalkyl having an alkylene radical of from 1 to 4 carbon atoms, cyano or carbamoyl;

$B^4$ is hydrogen, alkyl of from 1 to 4 carbon atoms or alkyl of from 1 to 4 carbon atoms which is substituted by alkoxy of from 1 to 4 carbon atoms, sulfo, carboxyl, sulfato, phenyl, sulfophenyl, acetylamino, benzoylamino, cyano or a group of the formula $-SO_2-Y^1$, where $Y^1$ is as defined above, or is alkenyl of from 2 to 4 carbon atoms, cyclohexyl, phenyl or phenyl which is substituted by substituents selected from the group consisting of carboxyl, sulfo, benzoylamino, acetylamino, $-SO_2-Y^1$, where $Y^1$ is as defined above, and chlorine.

**4.** The process as claimed in claim 2, wherein K is a radical of the formula (3c) or (3i) where

$B^1$ is methyl, carboxyl or carbalkoxy of from 2 to 5 carbon atoms,

Q is phenyl which is substituted by sulfo and/or a group of the formula $-SO_2-Y^1$, where $Y^1$ is as defined in claim 2, but preferably is $\beta$-sulfatoethyl,

$R^6$ is hydrogen, sulfo, methyl, ethyl, methoxy or ethoxy,

$R^7$ is hydrogen, methyl, ethyl, methoxy, ethoxy, chlorine, amino, substituted amino, alkanoylamino of from 2 to 5 carbon atoms, benzoylamino or phenylsulfonylamino,

$R^8$ is hydrogen, methyl, ethyl or hydroxyl-, sulfo-, sulfato- or carboxyl-substituted alkyl of from 2 to 4 carbon atoms, and

$R^9$ is hydrogen, methyl, ethyl or alkyl of from 2 to 4 carbon atoms, which is substituted by hydroxyl, sulfo, sulfato or carboxyl.

**5.** The process as claimed in claim 3, wherein K is a radical of the formula (3e), (3k) or (3m) where

R* is acetyl, sulfobenzoyl or benzoyl,

R'' is hydrogen or alkyl of from 1 to 4 carbon atoms,

m is 1 or 2,

M is hydrogen or an alkali metal,

$R^{10}$ is hydrogen or preferably methyl,

$R^{11}$ is hydrogen, cyano, sulfomethyl, carbamoyl or carboxyl,

$B^3$ is hydrogen, ethyl or sulfo-, phenyl- or sulfophenyl-substituted alkyl of from 2 to 4 carbon atoms, and

$B^4$ is hydrogen, methyl, ethyl or sulfo-, phenyl- or sulfophenyl-substituted alkyl of 2 to 4 carbon atoms.

**6.** The process as claimed in claim 3, wherein K is a radical of the formula (3p) where

R* is acetyl, sulfobenzoyl or benzoyl or preferably hydrogen,

R'' is alkyl of from 1 to 4 carbon atoms or preferably hydrogen,

m is 2, and
M is hydrogen or an alkali metal.

**7.** The process as claimed in at least one of claims 1 to 6, wherein R is hydrogen.

**8.** The process as claimed in at least one of claims 1 to 7, wherein X is chlorine.

**9.** The process as claimed in at least one of claims 1 to 8, wherein Y is sulfato or preferably chlorine.

**10.** A process for dyeing (including printing) hydroxyl- and/or carboxamido-containing material, in particular fiber material, by introducing a dye to or into the material and fixing it by means of heat and/or with the aid of an alkali agent, which comprises using as the dye a compound as mentioned and defined in the formula (1) or a compound of the formula (1) prepared as claimed in claim 1.

**11.** A process for preparing a compound conforming to the formula (7)

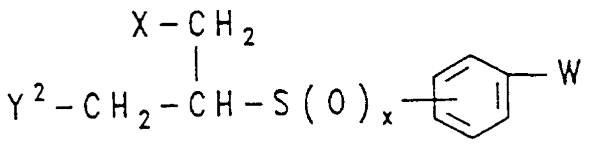

(7)

where
X is chlorine or bromine,
x is zero or 2,
W is nitro or amino, and
$Y^2$ is hydroxyl or a substituent which is eliminable by means of alkali to form a vinyl group,
which comprises reacting a nitrobenzenesulfenyl halide with a compound of the formula (8)

$Y^2 - CH_2 - CH = CH_2$ (8)

where $Y^2$ is as defined above, to form the compound of the formula (7), where $Y^2$ and X are each as defined above, x is zero and W is nitro, and in the case of the preparation of a compound of the formula (7) where $Y^2$ and X are each as defined above, x is 2 and W is nitro, reacting the compound conforming to the formula (7) where $Y^2$ and X are each as defined above, x is zero and W is nitro with an oxidizing agent, and in the case of the preparation of a compound of the formula (7) where $Y^2$ and X are each as defined above, x is 2 and W is amino, reducing, in the compound of the formula (7) where $Y^2$ and X are each as defined above, x is 2 and W is nitro, this nitro group.

**12.** The process as claimed in claim 11, wherein the compound of the formula (7) is a compound of the formula (4)

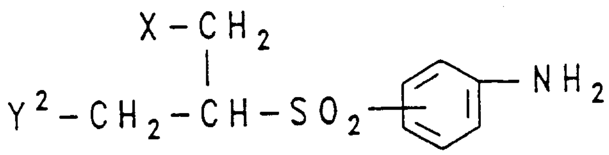

(4)

where X and $Y^2$ are each as defined in claim 11.

**13.** A compound as claimed in claim 11, wherein X is chlorine.

**14.** A compound as claimed in at least one of claims 11 to 13, wherein $Y^2$ is chlorine, bromine, cyano, alkanoyloxy of from 2 to 5 carbon atoms or sulfato or preferably chlorine, bromine, cyano or acetyloxy.

**Revendications**

**Revendications pour les Etats contractants suivants : DE, GB, FR, IT, CH, LI**

**1.** Composés azoïques répondant à la formule générale (1)

D - N = N - K (1)

dans laquelle représentent :

D représente un radical de formule générale (2)

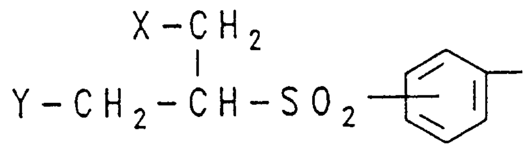

(2)

dans laquelle

Y représente un substituant éliminable en milieu alcalin en formant un groupe vinyle, le groupe sulfonyle étant lié de préférence en position méta ou para par rapport à la liaison libre sur le noyau de benzène, et

X représente le chlore ou le brome,

K représente un radical d'un composant de copulation simplement copulable, de préférence hydrosoluble, qui peut encore contenir un groupe azoïque, ou le radical d'un composant de copulation doublement copulable, de préférence hydrosoluble, chacun de la série des aminobenzènes, des phénols, des naphtols, des aminonaphtols ou des acylaminonaphtols avec le radical acyle d'un acide alcane- ou alcène-carboxylique avec chaque fois de 1 à 4, ou bien de 2 à 4 atomes de carbone dans le radical alkyle, respectivement alcényle ou d'un acide carboxylique aromatique ou d'un acide sulfonique aromatique ou d'un acide carbamique N-substitué ou de la série des acides dihydroxynaphtalènesulfoniques, des acides phénylazo- et naphtylazo-aminonaphtalènesulfoniques, des 5-pyrazolones et des 5-aminopyrazoles, des acétoacétylarylides, des 2-hydroxy-6-pyridones et des hydroxyquinoléines, K pouvant contenir, outre les substituants usuels pour les colorants aussi un groupe réactif vis-à-vis des fibres de formule générale $-SO_2-Y^1$, dans laquelle $Y^1$ représente le groupe vinyle ou un groupe de formule $-CH_2-CH_2-Y$ avec Y ayant la signification donnée ci-dessus.

**2.** Composé azoïque selon la revendication 1 caractérisé en ce que K représente un radical de formule générale (3c), (3f), (3h), (3i), (3p) ou (3q)

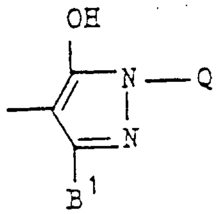 (3c)

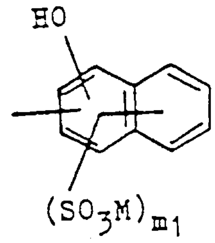 (3f)

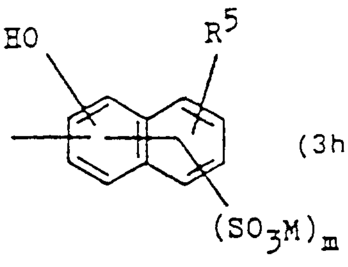 (3h)

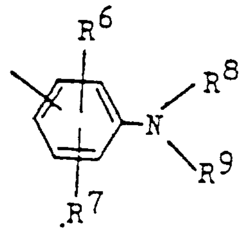 (3i)

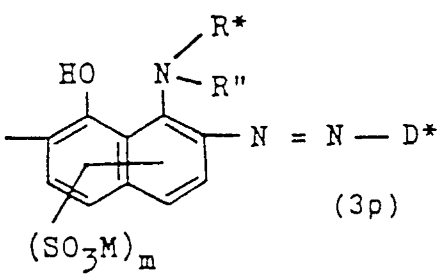 (3p)

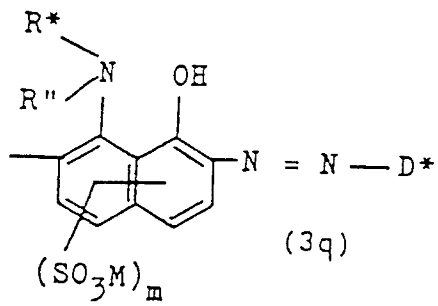 (3q)

dans lesquelles signifient :

- M représente l'hydrogène ou un métal alcalin ;
- $B^1$ représente alkyle avec 1 à 4 atomes de carbone, carboxy, carbalcoxy avec 2 à 5 atomes de carbone, carbamoyle, phényle ou phényle substitué par 1 ou 2 substituants pris dans le groupe comportant sulfo, carboxy, méthyle, éthyle, méthoxy, éthoxy et chlore ;
- Q représente phényle qui peut être substitué par 1, 2 ou 3 substituants pris dans le groupe comportant chlore, brome, méthyle, éthyle, méthoxy, éthoxy, carboxy, sulfo et alcanoylamino avec 2 à 5 atomes de carbone et/ou par un groupe de formule générale - $SO_2$-$Y^1$ dans laquelle $Y^1$ représente le groupe vinyle ou le groupe éthylè qui contient en position $\beta$ un substituant éliminable en milieu alcalin ;
- R* représente l'hydrogène ou alkyle avec 1 à 4 atomes de carbone, qui peut être substitué par phényle ou par phényle substitué par sulfo et/ou un groupe de formule générale -$SO_2$-$Y^1$ avec $Y^1$ ayant la signification donnée ci-dessus, ou est alcanoyle avec 2 à 5 atomes de carbone, phénylsulfonyle éventuellement substitué ou benzoyle éventuellement substitué ;
- R" représente l'hydrogène ou alkyle avec 1 à 4 atomes de carbone, qui peut être substitué par phényle, sulfophényle ou un groupe de formule générale -$SO_2$-$Y^1$ avec $Y^1$ ayant la signification donnée ci-dessus, ou est phényle ou phényle substitué par 1 ou 2 substituants pris dans le groupe comportant alkyle avec 1 à 4 atomes de carbone, alcoxy avec 1 à 4 atomes de carbone, chlore, brome, sulfo et -$SO_2$-$Y^1$ avec $Y^1$ ayant la signification donnée ci-dessus ;
- $R^5$ représente phényluréido, dont le radical phényle peut être substitué par un groupe de formule -$SO_2$-$Y^1$ avec $Y^1$ ayant la signification donnée ci-dessus, ou est alcanoylamino avec 2 à 5 atomes de carbone, qui peut être substitué dans le radical alkyle par un groupe de formule -$SO_2$-$Y^1$ avec $Y^1$ ayant la signification donnée ci-dessus, ou est alcénoylamino avec 3 à 5 atomes de carbone ou est benzoylamino qui peut être substitué par des substituants pris dans le groupe comportant chlore, méthyle, méthoxy, nitro, sulfo, carboxy et -$SO_2$-$Y^1$ avec $Y^1$ ayant la signification donnée ci-dessus ;

$R^6$ représente l'hydrogène, alkyle avec 1 à 4 atomes de carbone, alcoxy avec 1 à 4 atomes, sulfo, carboxy, carbalcoxy avec 2 à 5 atomes de carbone, halogène ou alcoxy avec 1 à 4 atomes de carbone qui est substitué par hydroxy, acétyloxy, carboxy, carbamoyle, cyano ou halogène ;

$R^7$ représente l'hydrogène, alkyle avec 1 à 4 atomes de carbone, alcoxy avec 1 à 4 atomes de carbone, halogène, cyano, trifluorométhyle, alcoxy avec 1 à 4 atomes de carbone qui est substitué par hydroxy, acétyloxy, carboxy, carbamoyle, cyano ou halogène ou par un groupe de formule $-SO_2-Y^1$ avec $Y^1$ ayant la signification donnée ci-dessus, ou est alcanoylamino avec 2 à 5 atomes de carbone qui peut être substitué par chlore, brome, alcoxy avec 1 à 4 atomes de carbone, phénoxy, phényle, hydroxy, carboxy ou sulfo ou un groupe de formule $-SO_2-Y^1$ avec $Y^1$ ayant la signification donnée ci-dessus, ou est alcénoylamino avec 3 à 5 atomes de carbone, qui peut être substitué par chlore, brome, carboxy ou sulfo, ou benzoylamino qui peut être substitué sur le noyau de benzène par des substituants pris dans le groupe comportant chlore, méthyle, sulfo et un groupe de formule $-SO_2-Y^1$ avec $Y^1$ ayant la signification donnée ci-dessus, ou est alkylsulfonyle avec 1 à 4 atomes de carbone ou phénylsulfonyle qui peut être substitué sur le noyau de benzène, par des substituants pris dans le groupe comportant chlore, méthyle, sulfo et un groupe de formule $-SO_2-Y^1$ avec $Y^1$ ayant la signification donnée ci-dessus, ou est alkylsulfonylamino avec 1 à 4 atomes de carbone qui peut être substitué par hydroxy, sulfato, chlore, brome, alcoxy avec 1 à 4 atomes de carbone ou un groupe de formule $-SO_2-Y^1$ avec $Y^1$ ayant la signification donnée ci-dessus, ou est phénylsulfonylamino qui peut être substitué sur le noyau de benzène par des substituants pris dans le groupe comportant chlore, méthyle, sulfo et un groupe de formule $-SO_2-Y^1$ avec $Y^1$ ayant la signification donnée ci-dessus, ou est carbamoyle qui peut être mono- ou disubstitué sur l'atome d'azote par 1 ou 2 substituants, les substituants appartenant au groupe comportant alkyle avec 1 à 4 atomes de carbone, alkyle comportant 1 à 4 atomes de carbone substitué par hydroxy, sulfo, carboxy, sulfato, phényle ou un groupe de formule $-SO_2-Y^1$ avec $Y^1$ ayant la signification donnée ci-dessus, cycloalkyle avec 5 à 8 atomes de carbone, phényle ou phényle substitué par des substituants pris dans le groupe comportant chlore, sulfo, méthyle, méthoxy, carboxy et un groupe de formule $-SO_2-Y^1$ avec $Y^1$ ayant la signification donnée ci-dessus, ou est sulfamoyle, qui peut être mono- ou disubstitué par 1 ou 2 substituants sur l'atome d'azote, les substituants appartenant au groupe comportant alkyle avec 1 à 4 atomes de carbone, alkyle avec 1 à 4 atomes de carbone substitué par hydroxy, sulfo, carboxy, sulfato, phényle ou un groupe de formule $-SO_2-Y^1$ avec $Y^1$ ayant la signification donnée ci-dessus, cycloalkyle avec 5 à 8 atomes de carbone, phényle ou phényle substitué par des substituants pris dans le groupe comportant chlore, sulfo, méthyle, méthoxy, carboxy et un groupe de formule $-SO_2-Y^1$ avec $Y^1$ ayant la signification donnée ci-dessus, ou est uréido ou uréido qui peut être mono-ou disubstitué sur l'atome d'azote terminal par 1 ou 2 substituants, les substituants appartenant au groupe comportant alkyle avec 1 à 4 atomes de carbone, alkyle avec 1 à 4 atomes substitué par hydroxy, sulfo, carboxy, sulfato, phényle ou un groupe de formule $-SO_2-Y^1$ avec $Y^1$ ayant la signification donnée ci-dessus, cycloalkyle avec 5 à 8 atomes de carbone, phényle et phényle substitué par des substituants pris dans le groupe comportant chlore, sulfo, méthyle, méthoxy, carboxy et un groupe de formule $-SO_2-Y^1$ avec $Y^1$ ayant la signification donnée ci-dessus ;

$R^8$ représente l'hydrogène ou alkyle avec 1 à 4 atomes de carbone qui peut être substitué par hydroxy, sulfo, carboxy, sulfato, un groupe de formule $-SO_2-Y^1$ avec $Y^1$ ayant la signification donnée ci-dessus, phényle ou sulfophényle, ou est alcényle avec 2 à 4 atomes de carbone, qui peut être substitué par carboxy, sulfo, chlore ou brome, ou est cycloalkyle avec 5 à 8 atomes de carbone ;

$R^9$ représente l'hydrogène ou alkyle avec 1 à 4 atomes de carbone qui peut être substitué par hydroxy, sulfo, carboxy, sulfato, phényle ou $-SO_2-Y^1$ avec $Y^1$ ayant la signification donnée ci-dessus ou est alcényle avec 2 à 5 atomes de carbone qui peut être substitué par carboxy, sulfo ou $-SO_2-Y^1$ avec $Y^1$ ayant la signification donnée ci-dessus ou par chlore ou brome, ou

$R^9$ représente cycloalkyle avec 5 à 8 atomes de carbone ou phényle, qui peut être substitué par des substituants pris dans le groupe comportant chlore, sulfo, méthyle,

méthoxy, carboxy et $-SO_2-Y^1$ avec $Y^1$ ayant la signification donnée ci-dessus, ou est naphtyle, qui est substitué par 1, 2 ou 3 sulfo ou par 1 ou 2 sulfo et 1 ou 2 groupes de formule $-SO_2-Y^1$ avec $Y^1$ ayant la signification donnée ci-dessus ou seulement par un tel groupe $-SO_2-Y^1$ ou

$R^8$ et $R^9$ forment ensemble avec l'atome d'azote le radical N-pipéridino, N-morpholino ou N-pipérazino ;

m vaut 1 ou 2 ;

$m_1$ vaut 1, 2 ou 3 ;

D* représente un groupe de formule générale (2), (2a) ou (2b) définie dans la revendication 1 ou est phényle, qui peut être substitué par 1, 2 ou 3 substituants pris dans le groupe comportant alkyle avec 1 à 4 atomes de carbone, alcoxy avec 1 à 4 atomes de carbone, chlore, brome, hydroxy, carboxy, sulfo, carbamoyle, sulfamoyle et alcanoylamino avec 2 à 5 atomes de carbone et/ou par un groupe de formule $-SO_2-Y^1$ avec $Y^1$ ayant la signification donnée ci-dessus, en préférant parmi ces substituants un groupe sulfo ou carboxy et le groupe $-SO_2-Y^1$ est de préférence en position méta ou para par rapport au groupe azo, ou D* est naphtyle, qui est substitué par 1, 2 ou 3 groupes sulfo ou par 1 ou 2 groupes sulfo et 1 ou 2 groupes de formule générale $-SO_2-Y^1$ avec $Y^1$ ayant la signification donnée ci-dessus ou seulement par un tel groupe $-SO_2-Y^1$,

D et D* pouvant avoir l'un par rapport à l'autre des significations identiques ou différentes.

**3.** Composé azoïque selon la revendication 1 caractérisé en ce que K représente un radical de formule générale (3d), (3e), (3k) ou (3m)

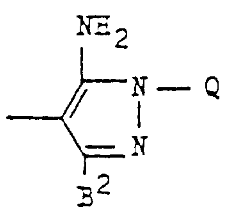

(3d)

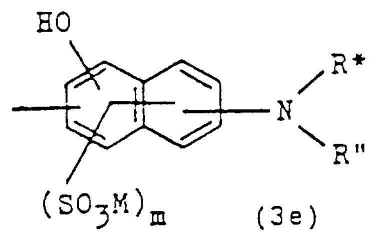

(3e)

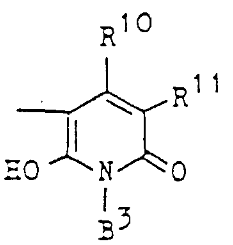

(3k)

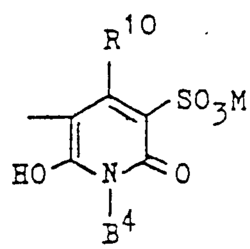

(3m)

dans lesquelles

M représente l'hydrogène ou un métal alcalin ;

$B^2$ représente alkyle avec 1 à 4 atomes de carbone, carbalcoxy avec 2 à 5 atomes de carbone, carbamoyle, phényle ou phényle substitué par 1 ou 2 substituants pris dans le groupe comportant alkyle avec 1 à 4 atomes de carbone, alcoxy avec 1 à 4 atomes de carbone, chlore, brome et sulfo ;

Q représente phényle qui peut être substitué par 1, 2 ou 3 substituants pris dans le groupe comportant chlore, brome, méthyle, éthyle, méthoxy, éthoxy, carboxy, sulfo et alcanoylamino avec 2 à 5 atomes de carbone et/ou par un groupe de formule générale $-SO_2-Y^1$ dans laquelle $Y^1$ représente le groupe vinyle ou le groupe éthyle, qui contient en position $\beta$ un substituant éliminable en milieu alcalin ;

R* représente l'hydrogène ou alkyle avec 1 à 4 atomes de carbone, qui peut être substitué par phényle ou par phényle substitué par sulfo et/ou un groupe de formule générale $-SO_2-Y^1$ avec $Y^1$ ayant la signification donnée ci-dessus, ou est alcanoyle avec 2 à 5 atomes de carbone, phénylsulfonyle éventuellement substitué ou benzoyle éventuellement substitué ;

R" représente l'hydrogène ou alkyle avec 1 à 4 atomes de carbone, qui peut être substitué par phényle, sulfophényle ou un groupe de formule générale $-SO_2-Y^1$ avec $Y^1$ ayant la signification donnée ci-dessus, ou est phényle ou phényle substitué par 1 ou 2 substituants pris

dans le groupe comportant alkyle avec 1 à 4 atomes de carbone, alcoxy avec 1 à 4 atomes de carbone, chlore, brome, sulfo et $-SO_2-Y^1$ avec $Y^1$ ayant la signification donnée ci-dessus ;

m vaut 1 ou 2 ;

$B^3$ représente l'hydrogène ou alkyle avec 1 à 4 atomes de carbone qui peut être substitué par phényle, sulfo, sulfophényle ou un groupe $-SO_2-Y^1$ avec $Y^1$ ayant la signification donnée dans la revendication 1 ;

$R^{10}$ représente l'hydrogène ou alkyle avec 1 à 4 atomes de carbone ou alkyle avec 1 à 4 atomes de carbone substitué par alcoxy avec 1 à 4 atomes de carbone ou cyano ;

$R^{11}$ représente l'hydrogène, carboxy, sulfo, sulfoalkyle avec un radical alkylène avec 1 à 4 atomes de carbone, cyano ou carbamoyle ;

$B^4$ représente l'hydrogène, alkyle avec 1 à 4 atomes de carbone ou alkyle avec 1 à 4 atomes de carbone substitué par alcoxy avec 1 à 4 atomes de carbone, sulfo, carboxy, sulfato, phényle, sulfophényle, acétylamino, benzoylamino, cyano ou un groupe de formule $-SO_2-Y^1$ avec $Y^1$ ayant la signification donnée ci-dessus, ou est alcényle avec 2 à 4 atomes de carbone, cyclohexyle, phényle ou phényle substitué par des substituants pris dans le groupe comportant carboxy, sulfo, benzoylamino, acétylamino, $-SO_2-Y^1$ avec $Y^1$ ayant la signification donnée ci-dessus, et chlore.

**4.** Composé azoïque selon la revendication 2, caractérisé en ce que K est un radical de formule (3c) ou (3i) dans lesquelles

$B^1$ représente méthyle, carboxy ou carbalcoxy avec 2 à 5 atomes de carbone,

Q représente phényle qui est substitué par sulfo et/ou un groupe de formule $-SO_2-Y^1$ dans laquelle $Y^1$ a la signification donnée dans la revendication 2, et est de préférence le $\beta$-sulfatoéthyle,

$R^6$ représente l'hydrogène, sulfo, méthyle, éthyle, méthoxy ou éthoxy,

$R^7$ représente l'hydrogène, méthyle, éthyle, méthoxy, éthoxy, chlore, amino, amino substitué, alcanoylamino avec 2 à 5 atomes de carbone, benzoylamino ou phénylsulfonylamino,

$R^8$ représente l'hydrogène, méthyle, éthyle ou alkyle avec 2 à 4 atomes de carbone substitué par hydroxy, sulfo, sulfato ou carboxy et

$R^9$ représente l'hydrogène, méthyle, éthyle ou alkyle avec 2 à 4 atomes de carbone qui est substitué par hydroxy, sulfo, sulfato ou carboxy.

**5.** Composé azoïque selon la revendication 3, caractérisé en ce que K est un radical de formule (3e), (3k) ou (3m) dans lesquelles

R* représente acétyle, sulfobenzoyle ou benzoyle,

R" représente l'hydrogène ou alkyle avec 1 à 4 atomes de carbone,

m vaut 1 ou 2,

M représente l'hydrogène ou un métal alcalin,

$R^{10}$ représente l'hydrogène ou de préférence méthyle,

$R^{11}$ représente l'hydrogène, cyano, sulfométhyle, carbamoyle ou carboxy,

$B^3$ représente l'hydrogène, éthyle ou alkyle avec 2 à 4 atomes de carbone substitué par sulfo, phényle ou sulfophényle et

$B^4$ représente l'hydrogène, méthyle, éthyle ou alkyle avec 2 à 4 atomes de carbone substitué par sulfo, phényle ou sulfophényle.

**6.** Composé azoïque selon la revendication 3, caractérisé en ce que K représente un radical de formule (3p) dans laquelle

R* représente acétyle, sulfobenzoyle ou benzoyle ou de préférence l'hydrogène,

R" représente alkyle avec 1 à 4 atomes de carbone ou de préférence l'hydrogène,

m vaut 2, et

M représente l'hydrogène ou un métal alcalin.

**7.** Composé azoïque selon au moins l'une des revendication 1 à 6, caractérisé en ce que X est chlore.

**8.** Composé azoïque selon au moins l'une des revendications 1 à 7, caractérisé en ce que Y est sulfato ou de préférence chlore.

9. Procédé pour la préparation d'un composé azoïque de formule générale (1) définie dans la revendication 1, caractérisé en ce qu'on fait réagir un composé de diazonium d'un composé amino de formule générale (4)

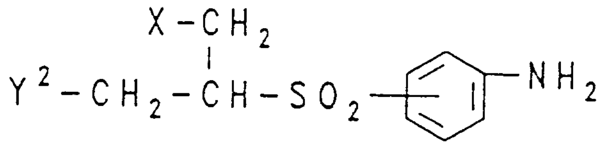

(4)

dans laquelle X a la signification donnée dans la revendication 1 et $Y^2$ représente un groupe hydroxy ou a l'une des significations de Y, avec un composant de copulation de formule générale H-K avec K ayant la signification donnée dans la revendication 1, ou en ce que, dans le cas de la préparation d'un composé disazoïque répondant à la formule générale (1) dans laquelle K représente un radical de formule générale (3q), on copule un composé monoazoïque de formule générale

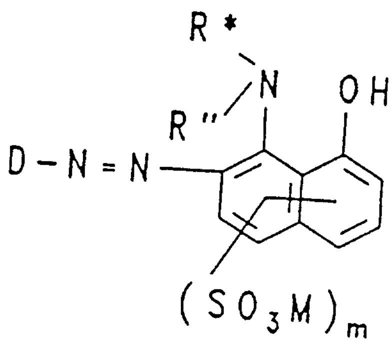

dans laquelle D, R*, R'', M et m ont les significations citées dans la revendication 1, respectivement revendication 2, avec un composé de diazonium d'une amine de formule générale $D^*-NH^2$ avec D* ayant les significations données dans les revendications 2, et en ce que, dans le cas de l'utilisation d'un composé de formule (4) avec $Y^2$ étant un groupe hydroxy, on transforme dans le composé azoïque obtenu le groupe hydroxy $Y^2$ en un groupe Y selon la formule (1).

10. Utilisation d'un composé répondant à la formule générale (1) de la revendication 1, pour la teinture (y compris l'impression) de matières contenant des groupes hydroxy et/ou carboxamido, notamment des matières fibreuses.

11. Utilisation pour la teinture (y compris l'impression) de matière contenant des groupes hydroxy et/ou carboxamido, notamment des matières fibreuses, selon lequel on applique un colorant à la matière ou on l'introduit dans celle-ci et on le fixe à l'aide de la chaleur et/ou à l'aide d'un agent à action alcaline caractérisé en ce qu'on utilise en tant que colorant un composé répondant à la formule générale (1) de la revendication 1.

12. Composé répondant à la formule générale (7)

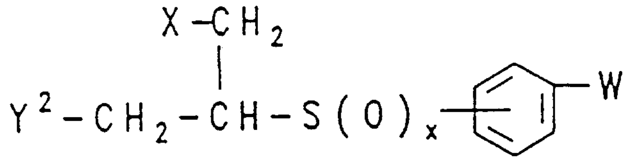

(7)

dans laquelle
X représente le chlore ou le brome,

x vaut 0 ou 2,
W représente nitro ou amino et
$Y^2$ représente le groupe hydroxy ou un substituant éliminable par des alcalis en formant le groupe vinyle.

**13.** Composé selon la revendication 12 répondant à la formule générale (4)

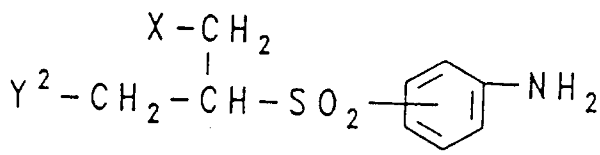

(4)

dans laquelle X et $Y^2$ ont la signification donnée dans la revendication 12.

**14.** Composé selon la revendication 12 ou 13, caractérisé en ce que X est chlore.

**15.** Composé selon au moins l'une des revendications 12 à 14, caractérisé en ce que $Y^2$ représente chlore, brome, cyano, alcanoyloxy avec 2 à 5 atomes de carbone ou sulfato ou de préférence chlore, brome, cyano ou acétyloxy.

**16.** Procédé pour la préparation d'un composé répondant à la formule générale (7) citée et définie dans la revendication 12, caractérisé en ce qu'on fait réagir un halogénure de nitrobenzènesulfényle avec un composé de formule générale (8)

$$Y^2 - CH_2 - CH = CH_2 \qquad (8)$$

dans laquelle $Y^2$ a la signification donnée dans la revendication 12, pour obtenir le composé de formule (7) dans laquelle $Y^2$ et X ont les significations données dans la revendication 12, x vaut 0 et W représente le groupe nitro, et en ce que dans le cas de la préparation d'un composé de formule générale (7) dans laquelle $Y^2$ et X ont les significations précitées, x vaut 2 et W représente le groupe nitro, composé correspondant à la formule générale (7) dans laquelle $Y^2$ et X ont les significations données ci-dessus, x vaut 0 et W représente le groupe nitro, avec un agent oxydant, et en ce que, dans le cas de la préparation d'un composé de formule générale (7), dans laquelle $Y^2$ et X ont les significations données ci-dessus, x vaut 2 et W est le groupe amino, en le composé de formule (7) dans laquelle $Y^2$ et X ont les significations données ci-dessus et x vaut 2 et W est le groupe nitro, on réduit ce groupe nitro.

**17.** Utilisation d'un composé de formule générale (7) de la revendication 12, dans laquelle $Y^2$ et X ont les significations données dans la revendication 12, x vaut 2 et W représente le groupe amino, en tant que composant de diazotation pour la synthèse de composés azoïques.

**Revendications pour l'Etat contractant suivant : ES**

**1.** Procédé pour la préparation d'un composé azoïque hydrosoluble répondant à la formule générale (1)

$$D - N = N - K \qquad (1)$$

sans laquelle représentent :

D représente un radical de formule générale (2)

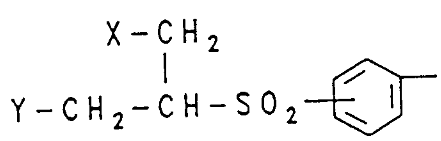

(2)

dans laquelle

Y représente un substituant éliminable en milieu alcalin sous la formation d'un groupe vinyle, le groupe sulfonyle étant lié de préférence en position méta ou para par rapport à la liaison libre sur le noyau de benzène, et

X représente le chlore ou le brome ;

K représente un radical d'un composant de copulation simplement copulable, de préférence hydrosoluble qui peut encore contenir un groupe azoïque, ou le radical d'un composant de copulation doublement copulable, de préférence hydrosoluble, chacun de la série des aminobenzènes, des phénols, des naphtols, des aminonaphtols ou des acylaminonaphtols, avec le radical acyle d'un acide alcane- ou alcènecarboxylique avec chaque fois de 1 à 4, respectivement de 2 à 4 atomes de carbone dans le radical alkyle, respectivement alcényle ou d'un acide carboxylique aromatique ou d'un acide sulfonique aromatique, ou d'un acide carbamique N-substitué ou de la série des acides dihydroxynaphtalènesulfoniques, des acides phénylazo- et naphtylazo-aminonaphtalènesulfoniques, des 5-pyrazolones et des 5-aminopyrazoles, des acétoacétylarylides, des 2-hydroxy-6-pyridones et de la hydroxyquinoléine, K pouvant contenir, outre les substituants usuels pour les colorants aussi un groupe réactif vis-à-vis des fibres de formule générale $-SO_2-Y^1$, dans laquelle $Y^1$ représente le groupe vinyle ou un groupe de formule $-CH_2-CH_2-Y$ avec Y ayant la signification donnée ci-dessus.

et est caractérisé en ce qu'on fait réagir un composé de diazonium d'un composé amino de formule générale (4)

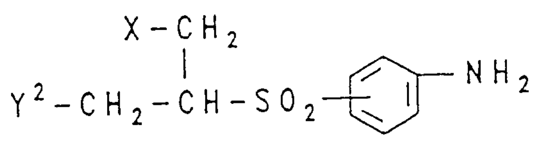

(4)

dans laquelle X a la signification donnée ci-dessus et $Y^2$ représente le groupe hydroxy ou a l'une des significations de Y avec un composant de copulation de formule générale H-K avec K ayant la signification donnée ci-dessus, ou en ce que, dans le cas de la préparation d'un composé disazoïque répondant à la formule générale (1) dans laquelle K représente un radical de formule générale (3q)

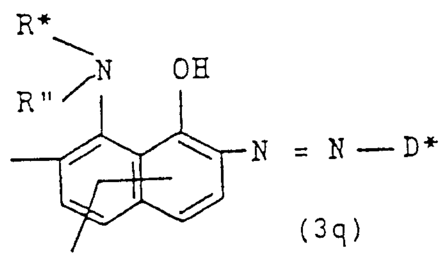

(3q)

dans laquelle

M représente l'hydrogène ou un métal alcalin ;

m vaut 1 ou 2 ;

R* représente l'hydrogène ou alkyle avec 1 à 4 atomes de carbone, qui peut être substitué par phényle ou sulfo et/ou un groupe de formule générale $-SO_2-Y^1$ avec $Y^1$ ayant la signification donnée ci-dessus, ou est alcanoyle avec 2 à 5 atomes de carbone, phénylsulfonyle éventuellement substitué ou benzoyle éventuellement substitué,

R'' représente l'hydrogène ou alkyle avec 1 à 4 atomes de carbone, qui peut être substitué par phényle, sulfophényle ou un groupe de formule générale $-SO_2-Y^1$ avec $Y^1$ ayant la signification donnée ci-dessus, ou est phényle ou phényle substitué par 1 ou 2 substituants pris dans le groupe comportant alkyle avec 1 à 4 atomes de carbone, alcoxy avec 1 à 4 atomes de carbone, chlore, brome, sulfo et $-SO_2-Y^1$ avec $Y^1$ ayant la signification donnée ci-dessus ;

D* représente un groupe dans la formule générale (2) définie ou est phényle, qui peut être substitué par 1, 2 ou 3 substituants pris dans le groupe comportant alkyle avec 1 à 4

atomes de carbone, alcoxy avec 1 à 4 atomes de carbone, chlore, brome, hydroxy, carboxy, sulfo, carbamoyle, sulfamoyle et alcanoylamino avec 2 à 5 atomes de carbone et/ou par un groupe de formule $-SO_2-Y^1$ avec $Y^1$ ayant la signification donnée ci-dessus, en préférant qu'un de ces substituants soit un groupe sulfo ou carboxy et que le groupe $-SO_2-Y^1$ soit de préférence en position méta ou para par rapport au groupe azo, ou D* est naphtyle, qui est substitué par 1, 2 ou 3 groupes sulfo ou par 1 ou 2 groupes sulfo et 1 ou 2 groupes de formule générale $-SO_2-Y^1$ avec $Y^1$ ayant la signification donnée ci-dessus ou seulement par un tel groupe $-SO_2-Y^1$,

D et D* pouvant avoir l'un par rapport à l'autre des significations identiques ou différentes, on copule un composé monoazoïque de formule générale

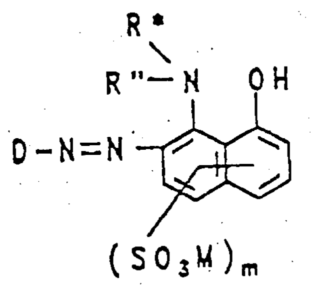

dans laquelle D, R*, R'', M et m ayant les significations donné ci-dessus, avec un composé de diazonium d'une amine de formule générale $D^*-NH_2$ avec D* ayant la signification donnée ci-dessus, et en ce que dans le cas de l'utilisation d'un composé de formule (4) avec $Y^2$ étant un groupe hydroxy, dans le composé azoïque obtenu on transforme le groupe hydroxy $Y^2$ en un groupe Y répondant à la formule (1).

**2.** Procédé selon la revendication 1, caractérisé en ce que K est un radical de formule générale (3c), (3f), (3h), (3i), (3p) ou (3q)

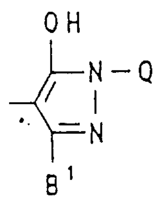

(3c)

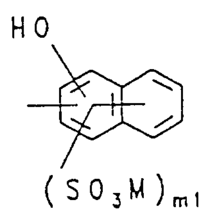

(3f)

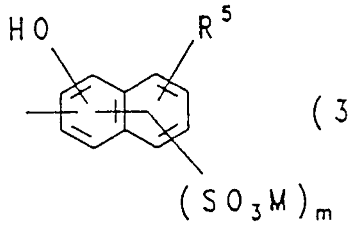

(3h)

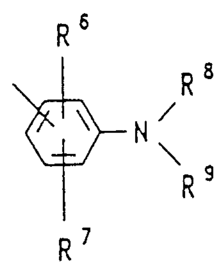

(3i)

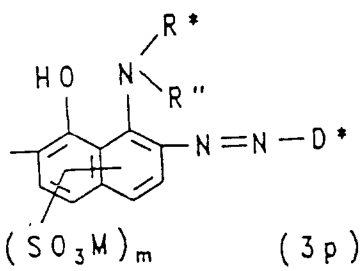

(3p)

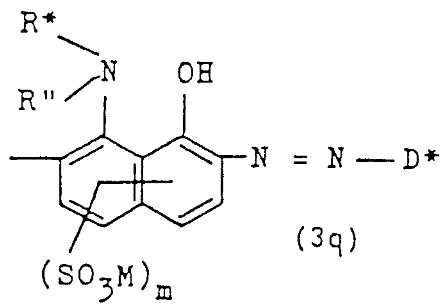

(3q)

dans lesquelles signifient :

M représente l'hydrogène ou un métal alcalin ;

$B^1$ représente alkyle avec 1 à 4 atomes de carbone, carboxy, carbalcoxy avec 2 à 5 atomes de carbone, carbamoyle, phényle ou phényle substitué par 1 ou 2 substituants pris dans le groupe comportant sulfo, carboxy, méthyle, éthyle, méthoxy, éthoxy et chlore ;

Q représente phényle qui peut être substitué par 1, 2 ou 3 substituants pris dans le groupe comportant chlore, brome, méthyle, éthyle, méthoxy, éthoxy, carboxy, sulfo et alcanoylamino avec 2 à 5 atomes de carbone et/ou par un groupe de formule générale - $SO_2$-$Y^1$ dans laquelle $Y^1$ représente le groupe vinyle ou le groupe éthyle qui contient en position $\beta$ un substituant éliminable en milieu alcalin ;

R* représente l'hydrogène ou alkyle avec 1 à 4 atomes de carbone, qui peut être substitué par phényle ou par phényle substitué par sulfo et/ou un groupe de formule générale -$SO_2$-$Y^1$ avec $Y^1$ ayant la signification donnée ci-dessus, ou est alcanoyle avec 2 à 5 atomes de carbone, phénylsulfonyle éventuellement substitué ou benzoyle éventuellement substitué ;

R" représente l'hydrogène ou alkyle avec 1 à 4 atomes de carbone, qui peut être substitué par phényle, sulfophényle ou un groupe de formule générale -$SO_2$-$Y^1$ avec $Y^1$ ayant la signification donnée ci-dessus, ou est phényle, ou phényle substitué par 1 ou 2 substituants pris dans le groupe comportant alkyle avec 1 à 4 atomes de carbone, alcoxy avec 1 à 4 atomes de carbone, chlore, brome, sulfo et -$SO_2$-$Y^1$ avec $Y^1$ ayant la signification donnée ci-dessus ;

$R^5$ représente phényluréido, dont le radical phényle peut être substitué par un groupe de formule -$SO_2$-$Y^1$ avec $Y^1$ ayant la signification donnée ci-dessus, ou est alcanoylamino avec 2 à 5 atomes de carbone, qui peut être substitué dans le radical alkyle par un groupe de formule -$SO_2$-$Y^1$ avec $Y^1$ ayant la signification donnée ci-dessus, ou est alcénoylamino avec 3 à 5 atomes de carbone ou est benzoylamino qui peut être substitué par des substituants pris dans le groupe comportant chlore, méthyle, méthoxy, nitro, sulfo, carboxy et -$SO_2$-$Y^1$ avec $Y^1$ ayant la signification donnée ci-dessus ;

$R^6$ représente l'hydrogène, alkyle avec 1 à 4 atomes de carbone, alcoxy avec 1 à 4 atomes, sulfo, carboxy, carbalcoxy avec 2 à 5 atomes de carbone, halogène ou alcoxy avec 1 à 4 atomes de carbone qui est substitué par hydroxy, acétyloxy, carboxy, carbamoyle, cyano ou halogène ;

$R^7$ est l'hydrogène, alkyle avec 1 à 4 atomes de carbone, alcoxy avec 1 à 4 atomes de carbone, halogène, cyano, trifluorométhyle, alcoxy avec 1 à 4 atomes de carbone et qui est substitué par hydroxy, acétyloxy, carboxy, carbamoyle, cyano ou halogène ou par un groupe de formule -$SO_2$-$Y^1$ avec $Y^1$ ayant la signification donnée ci-dessus, ou est alcanoylamino avec 2 à 5 atomes de carbone qui peut être substitué par chlore, brome, alcoxy avec 1 à 4 atomes de carbone, phénoxy, phényle, hydroxy, carboxy ou sulfo ou un groupe de formule -$SO_2$-$Y^1$ avec $Y^1$ ayant la signification donnée ci-dessus, ou est alcénoylamino avec 3 à 5 atomes de carbone, qui peut être substitué par chlore, brome, carboxy ou sulfo, ou benzoylamino qui peut être substitué sur le noyau de benzène par des substituants pris dans le groupe comportant le chlore, méthyle, sulfo et un groupe de formule -$SO_2$-$Y^1$ avec $Y^1$ ayant la signification donnée ci-dessus, ou est alkylsulfonyle avec 1 à 4 atomes de carbone ou phénylsulfonyle qui peut être substitué sur le noyau de benzène par des substituants pris dans le groupe comportant

chlore, méthyle, sulfo et un groupe de formule $-SO_2-Y^1$ avec $Y^1$ ayant la signification donnée ci-dessus, ou est alkylsulfonylamino avec 1 à 4 atomes de carbone qui peut être substitué par hydroxy, sulfato, chlore, brome, alcoxy avec 1 à 4 atomes de carbone ou un groupe de formule $-SO_2-Y^1$ avec $Y^1$ ayant la signification donnée ci-dessus, ou est phénylsulfonylamino qui peut être substitué sur le noyau de benzène par des substituants pris dans le groupe comportant chlore, méthyle, sulfo et un groupe de formule $-SO_2-Y^1$ avec $Y^1$ ayant la signification donnée ci-dessus, ou est carbamoyle qui peut être mono- ou disubstitué sur l'atome d'azote par 1 ou 2 substituants, les substituants appartenant au groupe comportant alkyle avec 1 à 4 atomes de carbone, alkyle avec 1 à 4 atomes de carbone substitués par hydroxy, sulfo, carboxy, sulfato, phényle ou un groupe de formule $-SO_2-Y^1$ avec $Y^1$ ayant la signification donnée ci-dessus, cycloalkyle avec 5 à 8 atomes de carbone, phényle ou phényle substitué par des substituants pris dans le groupe comportant chlore, sulfo, méthyle, méthoxy, carboxy et un groupe de formule $-SO_2-Y^1$ avec $Y^1$ ayant la signification donnée ci-dessus, ou est sulfamoyle, qui peut être mono- ou disubstitué par 1 ou 2 substituants sur l'atome d'azote, les substituants appartenant au groupe comportant alkyle avec 1 à 4 atomes de carbone, alkyle avec 1 à 4 atomes de carbone substitué par hydroxy, sulfo, carboxy, sulfato, phényle ou un groupe de formule $-SO_2-Y^1$ avec $Y^1$ ayant la signification donnée ci-dessus, cycloalkyle avec 5 à 8 atomes de carbone, phényle ou phényle substitué par des substituants pris dans le groupe comportant le chlore, sulfo, méthyle, méthoxy, carboxy et un groupe de formule $-SO_2-Y^1$ avec $Y^1$ ayant la signification donnée ci-dessus, ou est uréido ou uréido qui est mono- ou disubstitué sur l'atome d'azote terminal par 1 ou 2 substituants, les substituants appartenant au groupe comportant alkyle avec 1 à 4 atomes de carbone, alkyle avec 1 à 4 atomes substitué par hydroxy, sulfo, carboxy, sulfato, phényle ou un groupe de formule $-SO_2-Y^1$ avec $Y^1$ ayant la signification donnée ci-dessus, cycloalkyle avec 5 à 8 atomes de carbone, phényle et phényle substitué par des substituants pris dans le groupe comportant le chlore, sulfo, méthyle, méthoxy, carboxy et un groupe de formule $-SO_2-Y^1$ avec $Y^1$ ayant la signification donnée ci-dessus ;

$R^8$ est l'hydrogène ou alkyle avec 1 à 4 atomes de carbone qui peut être substitué par hydroxy, sulfo, carboxy, sulfato, un groupe de formule $-SO_2-Y^1$ avec $Y^1$ ayant la signification donnée ci-dessus, par phényle ou sulfophényle, ou est alcényle avec 2 à 4 atomes de carbone, qui peut être substitué par carboxy, sulfo, chlore ou brome, ou est cycloalkyle avec 5 à 8 atomes de carbone ;

$R^9$ est l'hydrogène ou alkyle avec 1 à 4 atomes de carbone qui peut être substitué par hydroxy, sulfo, carboxy, sulfato, phényle ou $-SO_2-Y^1$ avec $Y^1$ ayant la signification donnée ci-dessus ou est alcényle avec 2 à 5 atomes de carbone qui peut être substitué par carboxy, sulfo ou $-SO_2-Y^1$ avec $Y^1$ ayant la signification donnée ci-dessus ou par chlore ou brome, ou

$R^9$ est cycloalkyle avec 5 à 8 atomes de carbone ou phényle, qui peut être substitué par des substituants pris dans le groupe comportant chlore, sulfo, méthyle, méthoxy, carboxy et $-SO_2-Y^1$ avec $Y^1$ ayant la signification donnée ci-dessus, ou est naphtyle, qui est substitué par 1, 2 ou 3 sulfo ou par 1 ou 2 sulfo et 1 ou 2 groupes de formule $-SO_2-Y^1$ avec $Y^1$ ayant la signification donnée ci-dessus ou seulement par un tel groupe $-SO_2-Y^1$ ou

$R^8$ et $R^9$ forment ensemble avec l'atome d'azote le radical N-pipéridino, N-morpholino ou N-pipérazino ;

m vaut 1 ou 2 ;

$m_1$ vaut 1, 2 ou 3 ;

D* représente un groupe de formule générale (2) définie dans la revendication 1, ou est phényle, qui peut être substitué par 1, 2 ou 3 substituants pris dans le groupe comportant alkyle avec 1 à 4 atomes de carbone, alcoxy avec 1 à 4 atomes de carbone, chlore, brome, hydroxy, carboxy, sulfo, carbamoyle, sulfamoyle et alcanoylamino avec 2 à 5 atomes de carbone et/ou par un groupe de formule $-SO_2-Y^1$ avec $Y^1$ ayant la signification donnée ci-dessus, en préférant parmi ces substituants un groupe sulfo ou carboxy et le groupe $-SO_2-Y^1$ est de préférence en position méta ou para par rapport au groupe azo, ou D* représente naphtyle, qui est substitué par 1, 2 ou 3 groupes sulfo ou par 1 ou 2 groupes sulfo et 1 ou 2 groupes de formule générale $-SO_2-$

Y[1] avec Y[1] ayant la signification donnée ci-dessus ou seulement par un tel groupe $-SO_2-Y^1$,

D et D* pouvant avoir l'un par rapport à l'autre des significations identiques ou différentes.

3. Procédé selon la revendication 1, caractérisé en ce que K est un radical de formule générale (3d), (3e), (3k) ou (3m)

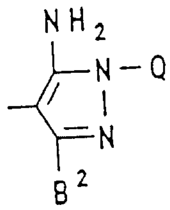
(3d)

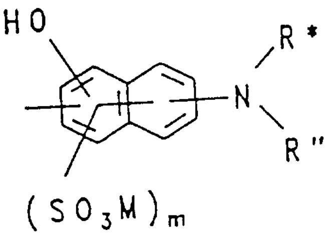
(3e)

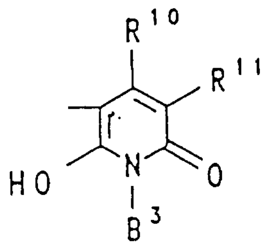
(3k)

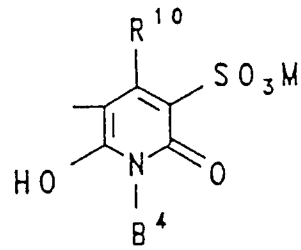
(3m)

dans lesquelles

- M représente l'hydrogène ou un métal alcalin ;
- $B^2$ représente alkyle avec 1 à 4 atomes de carbone, carbalcoxy avec 2 à 5 atomes de carbone, carbamoyle, phényle ou phényle substitué par 1 ou 2 substituants pris dans le groupe comportant alkyle avec 1 à 4 atomes de carbone, alcoxy avec 1 à 4 atomes de carbone, chlore, brome et sulfo ;
- Q représente phényle qui peut être substitué par 1, 2 ou 3 substituants pris dans le groupe comportant chlore, brome, méthyle, éthyle, méthoxy, éthoxy, carboxy, sulfo et alcanoylamino avec 2 à 5 atomes de carbone et/ou par un groupe de formule générale $-SO_2-Y^1$ dans laquelle $Y^1$ représente le groupe vinyle ou le groupe éthyle, qui contient en position $\beta$ un substituant éliminable en milieu alcalin ;
- R* représente l'hydrogène ou alkyle avec 1 à 4 atomes de carbone, qui peut être substitué par phényle ou par phényle substitue par sulfo et/ou un groupe de formule générale $-SO_2-Y^1$ avec $Y^1$ ayant la signification donnée ci-dessus, ou est alcanoyle avec 2 à 5 atomes de carbone, phénylsulfonyle éventuellement substitué ou benzoyle éventuellement substitué ;
- R" représente l'hydrogène ou alkyle avec 1 à 4 atomes de carbone, qui peut être substitué par phényle, sulfophényle ou un groupe de formule générale $-SO_2-Y^1$ avec $Y^1$ ayant la signification donnée ci-dessus, ou est phényle, ou phényle substitué par 1 ou 2 substituants pris dans le groupe comportant alkyle avec 1 à 4 atomes de carbone, alcoxy avec 1 à 4 atomes de carbone, chlore, brome, sulfo et $-SO_2-Y^1$ avec $Y^1$ ayant la signification donnée ci-dessus ;
- m vaut 1 ou 2 ;
- $B^3$ représente l'hydrogène ou alkyle avec 1 à 4 atomes de carbone qui peut être substitué par phényle, sulfo, sulfophényle ou un groupe $-SO_2-Y^1$ avec $Y^1$ ayant la signification donnée dans la revendication 1 ;
- $R^{10}$ représente l'hydrogène ou alkyle avec 1 à 4 atomes de carbone ou alkyle avec 1 à 4 atomes de carbone substitué par alcoxy avec 1 à 4 atomes de carbone ou cyano ;
- $R^{11}$ représente l'hydrogène, carboxy, sulfo, sulfoalkyle avec un radical alkylène avec 1 à 4 atomes de carbone, cyano ou carbamoyle ;
- $B^4$ représente l'hydrogène, alkyle avec 1 à 4 atomes de carbone ou alkyle avec 1 à 4 atomes de carbone substitué par alcoxy avec 1 à 4 atomes de carbone, sulfo, carboxy, sulfato, phényle, sulfophényle, acétylamino, benzoylamino, cyano ou un groupe de formule $-SO_2-Y^1$

avec $Y^1$ ayant la signification donnée ci-dessus, ou est alcényle avec 2 à 4 atomes de carbone, cyclohexyle, phényle ou phényle substitué par des substituants pris dans le groupe comportant carboxy, sulfo, benzoylamino, acétylamino, $-SO_2-Y^1$ avec $Y^1$ ayant la signification donnée ci-dessus, et chlore.

**4.** Procédé selon la revendication 2, caractérisé en ce que K est un radical de formule (3c) ou (3i) dans lesquelles
- $B^1$ représente méthyle, carboxy ou carbalcoxy avec 2 à 5 atomes de carbone,
- Q représente phényle qui est substitué par sulfo et/ou un groupe de formule $-SO_2-Y^1$ dans laquelle $Y^1$ ayant la signification donnée dans la revendication 2 et est de préférence le $\beta$-sulfatoéthyle,
- $R^6$ représente l'hydrogène, sulfo, méthyle, éthyle, méthoxy ou éthoxy,
- $R^7$ représente l'hydrogène, méthyle, éthyle, méthoxy, éthoxy, chlore, amino, amino substitué, alcanoylamino avec 2 à 5 atomes de carbone, benzoylamino ou phénylsulfonylamino,
- $R^8$ représente l'hydrogène, méthyle, éthyle ou alkyle avec 2 à 4 atomes de carbone substitué par hydroxy, sulfo, sulfato ou carboxy et
- $R^9$ représente l'hydrogène, méthyle, éthyle ou alkyle avec 2 à 4 atomes de carbone qui est substitué par hydroxy, sulfo, sulfato ou carboxy.

**5.** Procédé selon la revendication 3, caractérisé en ce que K est un radical de formule (3e), (3k) ou (3m) dans lesquelles
- R* représente acétyle, sulfobenzoyle ou benzoyle,
- R" représente l'hydrogène ou alkyle avec 1 à 4 atomes de carbone,
- m vaut 1 ou 2,
- M représente l'hydrogène ou un métal alcalin,
- $R^{10}$ représente l'hydrogène ou de préférence méthyle,
- $R^{11}$ représente l'hydrogène, cyano, sulfométhyle, carbamoyle ou carboxy,
- $B^3$ représente l'hydrogène, éthyle ou alkyle avec 2 à 4 atomes de carbone substitué par sulfo, phényle ou Sulfophényle et
- $B^4$ représente l'hydrogène, méthyle, éthyle ou alkyle avec 2 à 4 atomes de carbone substitué par sulfo, phényle ou sulfophényle.

**6.** Procédé selon la revendication 3, caractérisé en ce que K représente un radical de formule (3p) dans laquelle
- R* représente acétyle, sulfobenzoyle ou benzoyle ou de préférence l'hydrogène,
- R" représente alkyle avec 1 à 4 atomes de carbone ou de préférence l'hydrogène,
- m vaut 2, et
- M représente l'hydrogène ou un métal alcalin.

**7.** Procédé selon au moins l'une des revendication 1 à 6, caractérisé en ce que R est l'hydrogène.

**8.** Procédé selon au moins l'une des revendication 1 à 7, caractérisé en ce que X est le chlore.

**9.** Procédé selon au moins l'une des revendications 1 à 8 caractérisé en ce que Y est sulfato ou de préférence le chlore.

**10.** Procédé pour la teinture (y compris l'impression) de matière contenant des groupes hydroxy et/ou carboxamido, plus particulièrement des matières fibreuses, dans lequel on applique un colorant sur la matière ou on l'introduit dans celle-ci et on le fixe à l'aide de la chaleur et/ou à l'aide d'un agent à action alcaline, caractérisé en ce qu'on utilise en tant que colorant un composé de formule générale (1) définie et citée de la revendication 1, ou un composé de formule générale (1) préparé selon la revendication (1).

**11.** Procédé pour la préparation d'un composé répondant à la formule générale (7)

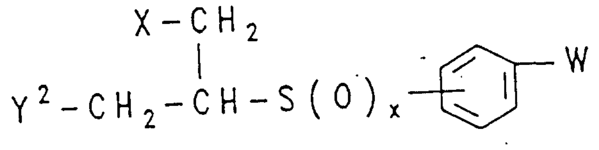

(7)

dans laquelle

- X représente le chlore ou le brome,
- x vaut 0 ou 2,
- W représente nitro ou amino et
- $Y^2$ représente le groupe hydroxy ou un substituant éliminable en milieu alcalin en forman le groupe vinyle,

caractérisé en ce qu'on fait réagir un halogénure de nitrobenzènesulfényle avec un composé de formule générale (8)

$Y^2 - CH_2 - CH = CH_2$ (8)

dans laquelle $Y^2$ a la signification donnée ci-dessus, pour obtenir le composé de formule (7) dans laquelle $Y^2$ et X ont les significations données ci-dessus, x vaut 0 et W représente le groupe nitro, et en ce que dans le cas de la préparation d'un composé de formule générale (7) dans laquelle $Y^2$ et X ont les significations données ci-dessus, x vaut 2 et W représente le groupe nitro, le composé répondant à la formule générale (7) dans laquelle $Y^2$ et X ont les significations données ci-dessus, x vaut 0 et W représente le groupe nitro, avec un agent oxydant, et en ce que dans le cas de la préparation d'un composé de formule générale (7), dans laquelle $Y^2$ et X ont les significations données ci-dessus, x vaut 2 et W est le groupe amino, en le composé de formule (7) dans laquelle $Y^2$ et X ont les significations données ci-dessus et x vaut 2 et W est le groupe nitro, on réduit ce groupe nitro.

**12.** Procédé selon la revendication 11, caractérisé en ce que le composé de formule (7) est un composé de formule générale (4)

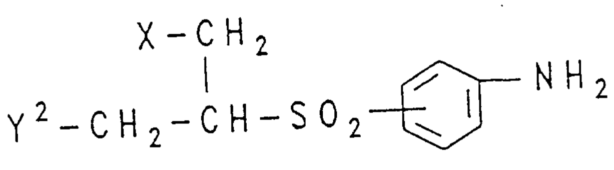

(4)

dans laquelle x et $Y^2$ ont la signification donnée dans la revendication 11.

**13.** Composé selon la revendication 11 ou 12, caractérisé en ce que X est le chlore.

**14.** Composé selon au moins l'une des revendications 11 à 13, caractérisé en ce que $Y^2$ représente chlore, brome, cyano, alcanoyloxy avec 2 à 5 atomes de carbone ou sulfato ou de préférence chlore, brome, cyano ou acétyloxy.